# EUROPEAN PATENT SPECIFICATION

(11) **EP 2 150 553 B1**
(45) Date of publication and mention of the grant of the patent: **05.10.2011**
(21) Application number: 08780617.0
(22) Date of filing: 09.05.2008
(51) Int. Cl.: C07D 487/04, A61K 31/4985, A61P 35/00, A61K 31/53

(54) **CONDENSED HETEROCYCLIC COMPOUNDS AS INHIBITORS OF PROTEIN KINASES**
KONDENSIERTE HETEROZYKLISCHE VERBINDUNGEN ALS HEMMER VON PROTEINKINASEN
COMPOSÉS HÉTÉROCYCLIQUES CONDENSÉS UTILISÉS EN TANT QU'INHIBITEURS DE PROTÉINES KINASES

(30) Priority: 09.05.2007 US 916838 P; 09.05.2007 US 916846 P
(43) Date of publication of application: 10.02.2010
(73) Proprietor: Abbott Laboratories, Abbott Park, IL 60064 (US)
(72) Inventor: MICHAELIDES, Michael, R., Libertyville, Illinois 60048 (US); JI, Zhiqin, Libertyville, Illinois 60048 (US)
(74) Representative: Modiano, Micaela Nadia
(86) International application number: PCT/US2008/063197
(87) International publication number: WO 2008/141145

(56) References cited:
- WO-A-2005/037836
- WO-A-2005/097800
- WO-A-2007/041712
- WO-A-2007/064993
- WO-A2-2007/061737
- WO-A2-2007/087395
- WO-A2-2007/106503

## Description

### FIELD OF THE INVENTION

This invention pertains to compounds that inhibit protein kinases such as Aurora-kinases, compositions containing the compounds and methods of treating diseases using the compounds.

### BACKGROUND OF THE INVENTION

Mitosis is a process by which a complete copy of a duplicated genome is segregated by the microtuble spindle apparatus into two daughter cells. Aurora-kinases, key mitotic regulators required for genome stability, have been found to be overexpressed in human tumors.

Document WO 2007/041712 discloses pyrazolopyrimidines as inhibitors of protein and/or checkpoint kinases, pharmaceutical compositions including one or more such compounds, methods of preparing said pyrazolopyrimidines and pharmaceutical compositions, and methods of treatment, prevention, inhibition, or amelioration of one or more diseases associated with the protein or checkpoint kinases using such compounds or pharmaceutical compositions.

Document WO 2005/097800 discloses 6,6 ring substituted heterobicyclic protein kinase which inhibit the IGF-1R enzyme and are useful for the treatment and/or prevention of hyperproliferative diseases such as cancer, inflammation, psoriasis, allergy/asthma, disease and conditions of the immune system, disease and conditions of the central nervous system.

Document WO 2005/037836 discloses imidazo[1,5-a]pyrazine tyrosine kinase inhibitors which inhibit the IGF-1R enzyme and are useful for the treatment and/or prevention of various diseases and conditions that respond to treatment by inhibition of tyrosine kinases.

There is therefore an existing need in the therapeutic arts for compounds which inhibit Aurora-kinases, compositions comprising the inhibitors and methods of treating diseases during which Aurora-kinases are unregulated or overexpressed.

### SUMMARY OF THE INVENTION

One embodiment of this invention, therefore, pertains to compounds that inhibit Aurora-kinases, the compounds having Formula I 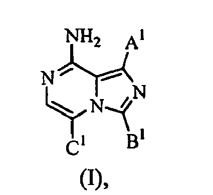 and therapeutically acceptable salts thereof, wherein
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵; wherein
R¹ is R², R³ or R⁴;
R² is phenyl which is unfused or fused with benzene, heteroarene or R^{2A}; R^{2A} is cycloalkane, cycloalkene, heterocycloalkane or heterocycloalkene;
R³ is heteroaryl which is unfused or fused with benzene, heteroarene or R^{3A}; R^{3A} is cycloalkane, cycloalkene, heterocycloalkane or heterocycloalkene;
R⁴ is cycloalkyl, cycloalkenyl, heterocycloalkyl or heterocycloalkenyl, each of which is unfused or fused with benzene, heteroarene or R^{4A}; R^{4A} is cycloalkane, cycloalkene, heterocycloalkane or heterocycloalkene;
R⁵ is alkyl, alkenyl, or alkynyl, each of which is unsubstituted or substituted with one, two, three, four or five of independently selected R⁶, OR⁶, SR⁶, S(O)R⁶, SO₂R⁶, NH₂, NHR⁶, N(R⁶)₂, C(O)R⁶ C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br or I;
R⁶ is R⁷, R⁸, R⁹, R^{9A};
R⁷ is phenyl which is unfused or fused with benzene, heteroarene or R^{7A}; R^{7A} is cycloalkane, cycloalkene, heterocycloalkane or heterocycloalkene;
R⁸ is heteroaryl which is unfused or fused with benzene, heteroarene or R^{8A}; R^{8A} is cycloalkane, cycloalkene, heterocycloalkane or heterocycloalkene;
R⁹ is cycloalkyl, cycloalkenyl, heterocycloalkyl or heterocycloalkenyl, each of which is unfused or fused with benzene, heteroarene or R^{9A}; R^{9A} is cycloalkane, cycloalkene, heterocycloalkane or heterocycloalkene;
R^{9A} is alkyl, alkenyl, or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, OR³⁰, OCH₂R³⁰, SR³⁰, S(O)R³⁰, SO₂R³⁰, C(O)R³⁰, CO(O)R³⁰, OC(O)R³⁰, OC(O)OR³⁰, NO₂, NH₂, NHR³⁰, N(R³⁰)₂, C(O)NH₂, C(O)NHR³⁰, C(O)N(R³⁰)₂, NHC(O)R³⁰, NHC(O)NHR³⁰, NHC(O)N(R³⁰)₂, NR³⁰C(O)NHR³⁰, NR³⁰C(O)N(R³⁰)₂, C(O)NHOH, C(O)NHOR³⁰, C(O)NHSO₂R³⁰, C(O)NR³⁰SO₂R³⁰, SO₂NH₂, SO₂NHR³⁰, SO₂N(R³⁰)₂, CF₃, CF₂CF₃, C(O)H, C(O)OH, C(N)NH₂, C(N)NHR³⁰ C(N)N(R³⁰)₂, CNOH, CNOCH₃, OH, (O), N₃, CF₃, CF₂CF₃, OCF₃, OCF₂CF₃, F, Cl, Br or I;
R³⁰ is R³¹, R³², R³³ or R³⁴;
R³¹ is phenyl which is unfused or fused with benzene, heteroarene or R^{31A}; R^{31A} is cycloalkane, cycloalkene, heterocycloalkane or heterocycloalkene;
R³² is heteroaryl which is unfused or fused with benzene, heteroarene or R^{32A}; R^{32A} is cycloalkane, cycloalkene, heterocycloalkane or heterocycloalkene;
R³³ is cycloalkyl, cycloalkenyl, heterocycloalkyl or heterocycloalkenyl, each of which is unfused or fused with benzene, heteroarene or R^{33A}; R^{33A} is cycloalkane, cycloalkene, heterocycloalkane or heterocycloalkene;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁵ or R³⁹;
R³⁶ is phenyl which is unfused or fused with benzene, heteroarene or R^{36A}; R^{36A} is cycloalkane, cycloalkene, heterocycloalkane or heterocycloalkene;
R³⁷ is heteroaryl which is unfused or fused with benzene, heteroarene or R^{37A}; R^{37A} is cycloalkane, cycloalkene, heterocycloalkane or heterocycloalkene;
R³⁸ is cycloalkyl, cycloalkenyl, heterocycloalkyl or heterocycloalkenyl, each of which is unfused or fused with benzene, heteroarene or R^{38A}; R^{38A} is cycloalkane, cycloalkene, heterocycloalkane or heterocycloalkene;
R³⁹ is alkyl, alkenyl or alkenyl, each of which is unsubstituted or substituted with
R⁴⁰;
R⁴⁰ is phenyl, heteroaryl, cycloalkyl, cycloalkenyl or heterocycloalkyl;
wherein the moieties represented by R³¹, R³², R³³ R³⁶, R³⁷ and R³⁸ are independently unsubstituted or substituted with one or two or three of independently selected F, Cl, Br, I, OH, (O)OH, NO₂, NH₂, CF₃, OH, R⁴⁵, OR⁴⁵, SR⁴⁵, S(O)R⁴⁵ SO₂R⁴⁵, C(O)NHR⁴⁵, C(O)N(R⁴⁵)₂, NHC(O)R⁴⁵, NR⁴⁵C(O)R⁴⁵, NHC(O)NHR⁴⁵, NHC(O)N(R⁴⁵)₂, NR⁴⁵C(O)NHR⁴⁵, NR⁴⁵C(O)N(R⁴⁵)₂, SO₂NHR⁴⁵, SO₂N(R⁴⁵)₂, NHSO₂R⁴⁵, NR¹SO₂R⁴⁵, OC(O)OR⁴⁵, NHC(O)OR⁴⁵ or NR⁴⁵C(O)OR⁴⁵;
R⁴⁵ is alkyl, alkenyl or alkynyl, each of which is unsubstituted or substituted with one or two or three of independently selected R⁵⁰, F, Cl, Br, I, OH, C(O)OH, NO₂ or NH₂; and
R⁵⁰ is phenyl, heteroaryl, cycloalkyl, cycloalkenyl or heterocycloalkyl.

Another embodiment of the present invention refers to a compound having Formula I 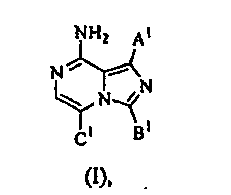 or a therapeutically acceptable salt thereof, wherein
A¹ is C(O)NH-phenyl;
B¹ and C¹ are independently H, or, R⁴ ;
wherein each foregoing cyclic moiety is independently unsubstituted or substituted with one or two or three or four or five of independently selected R³⁰, NHC(O)R³⁰, NHC(O)NHR³⁰, F, Cl, Br or I;
R³⁰ is R³¹, R³², or R³⁴;
R³² is heteroaryl
R³⁴ is alkyl, each of which is unsubstituted or substituted with F, Cl, Br or I;
R³⁵ is R³⁶, or R³⁷;
R³⁶ is phenyl;
R³⁷ is heteroaryl
wherein the moieties represented by R³¹ and R³⁶ are independently unsubstituted or substituted with one or two or three of independently selected F, Cl, Br, I ,
and R⁴⁵ is alkyl, which is unsubstituted or substituted with one or two or three of independently selected F, Cl, Br, I or OH.

Still another embodiment pertains to compositions comprising an excipient and a therapeutically affective amount of a compound having Formula I.

Still another embodiment pertains to a compound having Formula I, for use in treating bladder cancer, breast cancer, cervical cancer, colon cancer, endometrial cancer, esophageal cancer, lung cancer, ovarian cancer, pancreatic cancer, prostate cancer, rectal cancer, skin cancer, stomach cancer and thyroid cancer in a mammal, by administering thereto a therapeutically effective amount of said compound of Formula I with or without also administering radiotherapy thereto..

### DETAILED DESCRIPTION OF THE INVENTION

One embodiment of the invention refers to a compound having Formula I 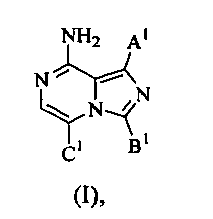 or a therapeutically acceptable salt thereof, wherein
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵; wherein
R¹ is R² R³ or R⁴;
R² is phenyl which is unfused or fused with benzene, heteroarene or R^{2A}; R^{2A} is cycloalkane, cycloalkene, heterocycloalkane or heterocycloalkene;
R³ is heteroaryl which is unfused or fused with benzene, heteroarene or R^{3A}; R^{3A} is cycloalkane, cycloalkene, heterocycloalkane or heterocycloalkene;
R⁴ is cycloalkyl, cycloalkenyl, heterocycloalkyl or heterocycloalkenyl, each of which is unfused or fused with benzene, heteroarene or R^{4A}; R^{4A} is cycloalkane, cycloalkene, heterocycloalkane or heterocycloalkene;
R⁵ is alkyl, alkenyl, or alkynyl, each of which is unsubstituted or substituted with one, two, three, four or five of independently selected R⁶, OR⁶, SR⁶, S(O)R⁶, SO₂R⁶, NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂ SO₂NHR⁶, SO₂N(R⁶)₂, NHC(O)NH₂, NHC(O)R⁶, NHC(O)N(R⁶)₂, NR⁶C(O)N(R)₂, OH, (O), C(O)OH, CN, CF₃, OCF₃, CF₂CF₃, F, Cl, Br or I;
R⁶ is R⁷, R⁸, R⁹, or R^{9A};
R⁷ is phenyl which is unfused or fused with benzene, heteroarene or R^{7A}; R^{7A} is cycloalkane, cycloalkene, heterocycloalkane or heterocycloalkene;
R⁸ is heteroaryl which is unfused or fused with benzene, heteroarene or R^{8A}; R^{8A} is cycloalkane, cycloalkene, heterocycloalkane or heterocycloalkene;
R⁹ is cycloalkyl, cycloalkenyl, heterocycloalkyl or heterocycloalkenyl, each of which is unfused or fused with benzene, heteroarene or R^{9A}; R^{9A} is cycloalkane, cycloalkene, heterocycloalkane or heterocycloalkene;
R^{9A} is alkyl, alkenyl, or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, OR³⁰, OCH₂R³⁰, SR³⁰, S(O)R³⁰, SO₂R³⁰, C(O)R³⁰, CO(O)R³⁰, OC(O)R³⁰, OC(O)OR³⁰, NO₂, NH₂, NHR³⁰, N(R³⁰)₂, C(O)NH₂, C(O)NHR³⁰, C(O)N(R³⁰)₂, NHC(O)R³⁰, NHC(O)NHR³⁰, NHC(O)N(R³⁰)₂, NR³⁰C(O)NHR³⁰, NR³⁰C(O)N(R³⁰)₂, C(O)NHOH, C(O)NHOR³⁰, C(O)NHSO₂R³⁰, C(O)NR³⁰SO₂R³⁰, SO₂NH₂, SO₂NHR³⁰, SO₂N(R³⁰)₂, CF₃, CF₂CF₃, C(O)H, C(O)OH, C(N)NH₂, C(N)NHR³⁰, C(N)N(R³)₂, CNOH, CNOCH₃, OH, (O), N₃, CF₃, CF₂CF₃, OCF₃, OCF₂CF₃, F, Cl, Br or I;
R³⁰ is R³¹, R³², R³³ or R³⁴;
R³¹ is phenyl which is unfused or fused with benzene, heteroarene or R^{31A}; R^{31A} is cycloalkane, cycloalkene, heterocycloalkane or heterocycloalkene;
R³² is heteroaryl which is unfused or fused with benzene, heteroarene or R^{32A}; R^{32A} is cycloalkane, cycloalkene, heterocycloalkane or heterocycloalkene;
R³³ is cycloalkyl, cycloalkenyl, heterocycloalkyl or heterocycloalkenyl, each of which is unfused or fused with benzene, heteroarene or R^{33A}; R^{33A} is cycloalkane, cycloalkene, heterocycloalkane or heterocycloalkene;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³¹)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁸ or R³⁹;
R³⁶ is phenyl which is unfused or fused with benzene, heteroarene or R^{36A}; R^{36A} is cycloalkane, cycloalkene, heterocycloalkane or heterocycloalkene;
R³⁷ is heteroaryl which is unfused or fused with benzene, heteroarene or R^{37A}; R^{37A} is cycloalkane, cycloalkene, heterocycloalkane or heterocycloalkene;
R³⁸ is cycloalkyl, cycloalkenyl, heterocycloalkyl or heterocycloalkenyl, each of which is unfused or fused with benzene, heteroarene or R^{38A}; R^{38A} is cycloalkane, cycloalkene, heterocycloalkane or heterocycloalkene;
R³⁹ is alkyl, alkenyl or alkenyl, each of which is unsubstituted or substituted with R⁴⁰;
R⁴⁰ is phenyl, heteroaryl, cycloalkyl, cycloalkenyl or heterocycloalkyl;
wherein the moieties represented by R³¹, R³², R³³ R³⁶, R³⁷ and R³⁸ are independently unsubstituted or substituted with one or two or three of independently selected F, Cl, Br, I, OH, (O)OH, NO₂, NH₂, CF₃, OH, R⁴⁵, OR⁴⁵, SR⁴⁵, S(O)R⁴⁵, SO₂R⁴⁵, C(O)NHR⁴⁵, C(O)N(R⁴⁵)₂, NHC(O)R⁴⁵, NR⁴⁵C(O)R⁴⁵, NHC(O)NHR⁴⁵, NHC(O)N(R⁴⁵)₂, NR⁴⁵C(O)NHR⁴⁵, NR⁴⁵C(O)N(R⁴⁵)₂, SO₂NHR⁴⁵, SO₂N(R⁴⁵)₂, NHSO₂R⁴⁵, NR¹SO₂R⁴⁵, OC(O)OR⁴⁵, NHC(O)OR⁴⁵ or NR⁴⁵C(O)OR⁴⁵;
R⁴⁵ is alkyl, alkenyl or alkynyl, each of which is unsubstituted or substituted with one or two or three of independently selected R⁵⁰, F, Cl, Br, I, OH, C(O)OH, NO₂ or NH₂; and
R⁵⁰ is phenyl, heteroaryl, cycloalkyl, cycloalkenyl or heterocycloalkyl.

Preferably, the invention refers to a compound having Formula I disclosed above, wherein
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵; wherein
R¹ is R², R³ or R⁴;
R² is phenyl which is unfused or fused with benzene, heteroarene or R^{2A}; R^{2A} is cycloalkane, cycloalkene, heterocycloalkane or heterocycloalkene;
R³ is heteroaryl which is unfused or fused with benzene, heteroarene or R^{3A}; R^{3A} is cycloalkane, cycloalkene, heterocycloalkane or heterocycloalkene;
R⁴ is cycloalkyl, cycloalkenyl, heterocycloalkyl or heterocycloalkenyl, each of which is unfused or fused with benzene, heteroarene or R^{4A}; R^{4A} is cycloalkane, cycloalkene, heterocycloalkane or heterocycloalkene;
R⁵ is alkyl, alkenyl, or alkynyl, each of which is unsubstituted or substituted with one, two, three, four or five of independently selected R⁶, OR⁶, SR⁶, S(O)R⁶, SO₂R⁶, NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br or I;
R⁶ is R⁷, R⁸, R⁹, or R^{9A};
R⁷ is phenyl which is unfused or fused with benzene, heteroarene or R^{7A}; R^{7A} is cycloalkane, cycloalkene, heterocycloalkane or heterocycloalkene;
R⁸ is heteroaryl which is unfused or fused with benzene, heteroarene or R^{8A}; R^{8A} is cycloalkane, cycloalkene, heterocycloalkane or heterocycloalkene;
R⁹ is cycloalkyl, cycloalkenyl, heterocycloalkyl or heterocycloalkenyl, each of which is unfused or fused with benzene, heteroarene or R^{9A}; R^{9A} is cycloalkane, cycloalkene, heterocycloalkane or heterocycloalkene;
R^{9A} is alkyl, alkenyl, or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, NHC(O)R³⁰, NHC(O)NHR³⁰, F, Cl, Br or I;
R³⁰ is R³¹, R³², R³³ or R³⁴;
R³¹ is phenyl which is unfused or fused with benzene, heteroarene or R^{31A}; R^{31A} is cycloalkane, cycloalkene, heterocycloalkane or heterocycloalkene;
R³² is heteroaryl which is unfused or fused with benzene, heteroarene or R^{32A}; R^{32A} is cycloalkane, cycloalkene, heterocycloalkane or heterocycloalkene;
R³³ is cycloalkyl, cycloalkenyl, heterocycloalkyl or heterocycloalkenyl, each of which is unfused or fused with benzene, heteroarene or R^{33A}; R^{33A} is cycloalkane, cycloalkene, heterocycloalkane or heterocycloalkene;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁸ or R³⁹;
R³⁶ is phenyl which is unfused or fused with benzene, heteroarene or R^{36A}; R^{36A} is cycloalkane, cycloalkene, heterocycloalkane or heterocycloalkene;
R³⁷ is heteroaryl which is unfused or fused with benzene, heteroarene or R^{37A}; R^{37A} is cycloalkane, cycloalkene, heterocycloalkane or heterocycloalkene;
R³⁸ is cycloalkyl, cycloalkenyl, heterocycloalkyl or heterocycloalkenyl, each of which is unfused or fused with benzene, heteroarene or R^{38A}; R^{38A} is cycloalkane, cycloalkene, heterocycloalkane or heterocycloalkene;
R³⁹ is alkyl, alkenyl or alkenyl, each of which is unsubstituted or substituted with R⁴⁰;
R⁴⁰ is phenyl, heteroaryl, cycloalkyl, cycloalkenyl or heterocycloalkyl;
wherein the moieties represented by R³¹, R³², R³³ R³⁶, R³⁷ and R³⁸ are independently unsubstituted or substituted with one or two or three of independently selected F, Cl, Br, I, CF₃, R⁴⁵; and

R⁴⁵ is alkyl, alkenyl or alkynyl, each of which is unsubstituted or substituted with one or two or three of independently selected F, Cl, Br, I, OH, C(O)OH, NO₂ or NH₂.

In a preferred embodiment, in the compound of Formula (1) disclosed above
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵; wherein
R¹ is R², R³ or R⁴;
R² is phenyl;
R³ is heteroaryl;
R⁴ is cycloalkyl;
R⁵ is alkyl, alkenyl, or alkynyl, each of which is unsubstituted or substituted with one, two, three, four or five of independently selected R⁶, OR⁶, SR⁶, S(O)R⁶, SO₂R⁶ NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br or I;
R⁶ is R⁷, R⁸, R⁹, or R^{9A};
R⁷ is phenyl;
R⁸ is heteroaryl;
R⁹ is cycloalkyl, cycloalkenyl, heterocycloalkyl or heterocycloalkenyl;
R^{9A} is alkyl, alkenyl, or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, NHC(O)R³⁰, NHC(O)NHR³⁰, F, Cl, Br or I;
R³⁰ is R³¹, R³², R³³ or R³⁴;
R³¹ is phenyl;
R³² is heteroaryl;
R³³ is cycloalkyl, cycloalkenyl, heterocycloalkyl or heterocycloalkenyl;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁸ or R³⁹;
R³⁶ is phenyl;
R³⁷ is heteroaryl;
R³⁸ is cycloalkyl, cycloalkenyl, heterocycloalkyl or heterocycloalkenyl;
R³⁹ is alkyl, alkenyl or alkenyl, each of which is unsubstituted or substituted with R⁴⁰;
R⁴⁰ is phenyl, heteroaryl, cycloalkyl, cycloalkenyl or heterocycloalkyl;
wherein the moieties represented by R³, R³², R³³ R³⁶, R³⁷ and R³⁸ are independently unsubstituted or substituted with one or two or three of independently selected F, Cl, Br, I, CF₃, R⁴⁵; and

R⁴⁵ is alkyl, alkenyl or alkynyl, each of which is unsubstituted or substituted with one or two or three of independently selected F, Cl, Br, I, OH, C(O)OH, NO₂ or NH₂.

In a preferred embodiment the compound of Formula (I) disclosed above is:
8-amino-N-(4-((((3-fluorophenyl)amino)carbonyl)amino)phenyl)-3-methylimidazo[1,5-a]pyrazine-1-carboxamide,
8-amino-N-(4-((anilinocarbonyl)amino)phenyl)-3-methylimidazo[1,5-a]pyrazine-1-carboxamide,
8-amino-N-(4-(benzoylamino)phenyl)-3-methylimidazo[1,5-a]pyrazine-1-carboxamide,
8-amino-3-methyl-N-(4-((((3-(trifluoromethyl)phenyl)amino)carbonyl)amino)phenyl)imidazo[1,5-a]pyrazine-1-carboxamide,
8-amino-N-(3-(((((3-fluorophenyl)amino)carbonyl)amino)methyl)phenyl)-3-methylimidazo[1,5-a]pyrazine-1-carboxamide,
8-amino-N-(3-(((anilinocarbonyl)amino)methyl)phenyl)-3-methylimidazo[1,5-a]pyrazine-1-carboxamide,
8-amino-N-(3-(((((2-fluorophenyl)amino)carbonyl)amino)methyl)phenyl)-3-methylimidazo[1,5-a]pyrazine-1-carboxamide,
8-amino-N-(3-(((((4-fluorophenyl)amino)carbonyl)amino)methyl)phenyl)-3-methylimidazo[1,5-a]pyrazine-1-carboxamide,
8-amino-3-methyl-N-(3-(((((4-(trifluoromethyl)phenyl)amino)carbonyl)amino)methyl)phenyl)imidazo[1,5-a]pyrazine-1-carboxamide,
8-amino-N-(4-((((3-(2-hydroxyethyl)phenyl)amino)carbonyl)amino)phenyl)-3-methylimidazo[1,5-a]pyrazine-1-carboxamide,
8-amino-N-(4-((((4-(2-hydroxyethyl)phenyl)amino)carbonyl)amino)phenyl)-3-methylimidazo[1,5-a]pyrazine-1-carboxamide;
8-amino-3-(3-(ethyl(2-hydroxyethyl)amino)-3-oxopropyl)-N-(3-(((((4-(trifluoromethyl)phenyl)amino)carbonyl)amino)methyl)phenyl)imidazo[1,5-a]pyrazine-1-carboxamide;
8-amino-N-(3-(((((4-chloro-2-fluorophenyl)amino)carbonyl)amino)methyl)phenyl)-3-(3-(ethyl(2-hydroxyethyl)amino)-3-oxopropyl)imidazo [1,5-a]pyrazine-1-carboxamide;
8-amino-3-(3-(ethyl(2-hydroxyethyl)amino)-3-oxopropyl)-N-(3-(((((3-fluorophenyl)amino)carbonyl)amino)methyl)phenyl)imidazo[1,5-a]pyrazine-1-carboxamide;
8-amino-3-(3-(ethyl(2-hydroxyethyl)amino)-3-oxopropyl)-N-(4-((((3-fluorophenyl)amino)carbonyl)amino)phenyl)imidazo[1,5-a]pyrazine-1-carboxamide,
or a therapeutically acceptable salt thereof.

Another embodiment of the invention refers to a composition comprising an excipient and a therapeutically effective amount of a compound having Formula I disclosed above.

Still another embodiment of the invention refers to a compound having Formula I as disclosed above for use in treating bladder cancer, breast cancer, cervical cancer, colon cancer, endometrial cancer, esophageal cancer, lung cancer, ovarian cancer, pancreatic cancer, prostate cancer, rectal cancer, skin cancer, stomach cancer or thyroid cancer in a mammal by administering thereto a therapeutically effective amount of said compound.

Another embodiment of the invention refers to a compound having Formula I 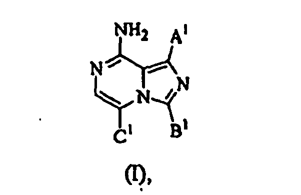 or a therapeutically acceptable salt thereof, wherein
A¹ is C(O)NH-phenyl;
B¹ and C¹ are independently H or R⁴;
R⁴ is cycloalkyl;
wherein each foregoing cyclic moiety is independently unsubstituted or substituted with one or two or three or four or five of independently selected R³⁰, NHC(O)R³⁰, NHC(O)NHR³⁰, F, Cl Br or I;
R³⁰ is R³¹, R³², or R³⁴;
R³¹ is phenyl;
R³² is heteroaryl;
R³⁴ is alkyl, each of which is unsubstituted or substituted with NHSO₂R³⁵, NHC(O)NHR³⁵, F, Cl, Br, or I;
R³⁵ is R³⁶, or R³⁷;
R³⁶ is phenyl;
R³⁷ is heteroaryl;
wherein the moieties represented by R³¹ and R³⁶ are independently unsubstituted or substituted with one or two or three of independently selected F, Cl, Br, I, CF₃, R⁴⁵; and
R⁴⁵ is alkyl, which is unsubstituted or substituted with one or two or three of independently selected F, Cl, Br, I, or OH.

Preferably, the compound of Formula (I) is
8-amino-3-cyclopropyl-N-(4-((((3-fluorophenyl)amino)carbonyl)amino)phenyl)imidazo[1,5-a]pyrazine-1-carboxamide;
8-amino-N-(4-((anilinocarbonyl)amino)phenyl)-3-cyclopropylimidazo[1,5-a]pyrazine-1-carboxamide;
8-amino-3-cyclopropyl-N-(4-((((3-methylphenyl)amino)carbonyl)amino)phenyl)imidazo[1,5-a]pyrazine-1-carboxamide;
8-amino-3-cyclopropyl-N-(4-((((2-fluoro-5-(trifluoromethyl)phenyl)amino)carbonyl)amino)phenyl)imidazo[1,5-a]pyrazine-1-carboxamide;
8-amino-3-cyclopropyl-N-(4-((((4-(trifluoromethyl)phenyl)amino)carbonyl)amino)phenyl)imidazo[1,5-a]pyrazine-1-carboxamide;
8-amino-3-cyclopropyl-N-(3-((((pyridin-3-ylamino)carbonyl)amino)methyl)phenyl)imidazo[1,5-a]pyrazine-1-carboxamide;
8-amino-3-cyclopropyl-N-(4-((((3-fluorophenyl)amino)carbonyl)amino)-3-methylphenyl)imidazo[1,5-a]pyrazine-1-carboxamide;
8-amino-3-cyclopropyl-N-(4-((((2-fluorophenyl)amino)carbonyl)amino)-3-methylphenyl)imidazo[1,5-a]pyrazine-1-carboxamide;
8-amino-3-cyclopropyl-N-(3-methyl-4-((((4-(trifluoromethyl)phenyl)amino)carbonyl)amino)phenyl)imidazo[1,5-a]pyrazine-1-carboxamide;
8-amino-3-cyclopropyl-N-(3-((((3-fluorophenyl)amino)carbonyl)amino)phenyl)imidazo[1,5-a]pyrazine-1-carboxamide;
8-amino-3-cyclopropyl-N-(3-(((((3-fluorophenyl)amino)carbonyl)amino)methyl)phenyl)imidazo[1,5-a]pyrazine-1-carboxamide;
8-amino-N-(3-(((((4-chloro-2-fluorophenyl)amino)carbonyl)amino)methyl)phenyl)-3-cyclopropylimidazo[1,5-a]pyrazine-1-carboxamide;
8-amino-N-(3-(((anilinocarbonyl)amino)methyl)phenyl)-3-cyclopropylimidazo[1,5-a]pyrazine-1-carboxamide;
8-amino-3-cyclopropyl-N-(3-(((((3,4-difluorophenyl)amino)carbonyl)amino)methyl)phenyl)imidazo[1,5-a]pyrazine-1-carboxamide;
8-amino-3-cyclopropyl-N-(3-(((((2-fluorophenyl)amino)carbonyl)amino)methyl)phenyl)imidazo[1,5-a]pyrazine-1-carboxamide;
8-amino-3-cyclopropyl-N-(3-(((((4-fluorophenyl)amino)carbonyl)amino)methyl)phenyl)imidazo[1,5-a]pyrazine-1-carboxamide;
8-amino-N-(3-(((((3-chlorophenyl)amino)carbonyl)amino)methyl)phenyl)-3-cyclopropylimidazo[1,5-a]pyrazine-1-carboxamide;
8-amino-N-(3-(((((4-chlorophenyl)amino)carbonyl)amino)methyl)phenyl)-3-cyclopropylimidazo[1,5-a]pyrazine-1-carboxamide;
8-amino-3-cyclopropyl-N-(4-(((pyridin-3-ylamino)carbonyl)amino)phenyl)imidazo[1,5-a]pyrazine-1-carboxamide;
8-amino-3-cyclopropyl-N-(3-(((phenylsulfonyl)amino)methyl)phenyl)imidazo[1,5-a]pyrazine-1-carboxamide;
8-amino-3-cyclopropyl-N-(3-(((((4-(trifluoromethyl)phenyl)amino)carbonyl)amino)methyl)phenyl)imidazo[1,5-a]pyrazine-1-carboxamide;
or a pharmaceutically acceptable salt thereof.

Another embodiment of the invention refers to a pharmaceutical composition comprising an excipient and a therapeutically effective amount of a compound having Formula I so as disclosed above.

Still another embodiment of the invention refers to a compound having Formula I so as disclosed above for use in treating bladder cancer, breast cancer, cervical cancer, colon cancer, endometrial cancer, esophageal cancer, lung cancer, ovarian cancer, pancreatic cancer, prostate cancer, rectal cancer, skin cancer, stomach cancer or thyroid cancer in a mammal by administering thereto a therapeutically effective amount of said compound.

Variable moieties of compounds herein are represented by identifiers (capital letters with numerical and/or alphabetical superscripts) and may be specifically embodied.

It is meant to be understood that proper valences are maintained for all one moieties and combinations thereof, that monovalent moieties having more than one atom are attached through their left ends.

It is also meant to be understood that a specific embodiment of a variable moiety may be the same or different as another specific embodiment having the same identifier.

The term "cyclic moiety," as used herein, means benzene, cycloalkane, cycloalkyl, cycloalkene, cycloalkenyl, heteroarene, heteroaryl, heterocycloalkane, heterocycloalkyl, heterocycloalkene, heterocycloalkenyl and phenyl.

The term "cycloalkane," as used herein, means C₃-cycloalkane, C₄-cycloalkane, C₅-cycloalkane and C₆-cycloalkane.

The term "cycloalkyl," as used herein, means C₃-cycloalkyl, C₄-cycloalkyl, C₅-cycloalkyl and C₆-cycloalkyl.

The term "cycloalkene," as used herein, means C₄-cycloalkene, C₅-cycloalkene and C₆-cycloalkene.

The term "cycloalkenyl," as used herein, means C₄-cycloalkenyl, C₅-cycloalkenyl and C₆-cycloalkenyl.

The term "heteroarene," as used herein, means furan, imidazole, isothiazole, isoxazole, 1,2,3-oxadiazole, 1,2,5-oxadiazole, oxazole, pyrazine, pyrazole, pyridazine, pyridine, pyrimidine, pyrrole, thiazole, thiophene, triazine and 1,2,3-triazole.

The term "heteroaryl," as used herein, means furanyl, imidazolyl, isothiazolyl, isoxazolyl, 1,2,3-oxadiazoyl, 1,2,5-oxadiazolyl, oxazolyl, pyrazinyl, pyrazolyl, pyridazinyl, pyridinyl, pyrimidinyl, pyrrolyl, tetrazolyl, thiazolyl, thiophenyl, triazinyl and 1,2,3-triazolyl.

The term "heterocycloalkane," as used herein, means cycloalkane having one or two or three CH₂ moieties replaced with independently selected O, S, S(O), SO₂ or NH and one or two CH moieties unreplaced or replaced with N and also means cycloalkane having one or two or three CH₂ moieties unreplaced or replaced with independently selected O, S, S(O), SO₂ or NH and one or two CH moieties replaced with N.

The term "heterocycloalkyl," as used herein, means cycloalkyl having one or two or three CH₂ moieties replaced with independently selected O, S, S(O), SO₂ or NH and one or two CH moieties unreplaced or replaced with N and also means cycloalkyl having one or two or three CH₂ moieties unreplaced or replaced with independently selected O, S, S(O), SO₂ or NH and one or two CH moieties replaced with N.

The term "heterocycloalkene," as used herein, means cycloalkene having one or two or three CH₂ moieties replaced with independently selected O, S, S(O), SO₂ or NH and one or two CH moieties unreplaced or replaced with N and also means cycloalkene having one or two or three CH₂ moieties unreplaced or replaced with independently selected O, S, S(O), SO₂ or NH and one or two CH moieties replaced with N.

The term "heterocycloalkenyl," as used herein, means cycloalkenyl having one or two or three CH₂ moieties replaced with independently selected O, S, S(O), SO₂ or NH and one or two CH moieties unreplaced or replaced with N and also means cycloalkenyl having one or two or three CH₂ moieties unreplaced or replaced with independently selected O, S, S(O), SO₂ or NH and one or two CH moieties replaced with N.

The term "alkenyl," as used herein, means C₂-alkenyl, C₃-alkenyl, C₄-alkenyl, C₅-alkenyl and C₆-alkenyl.

The term "alkyl," as used herein, means C₁-alkyl, C₂-alkyl, C₃-alkyl, C₄-alkyl, C₅-alkyl and C₆-alkyl.

The term "alkynyl," as used herein, means C₂-alkynyl, C₃-alkynyl, C₄-alkynyl, C₅-alkynyl and C₆-alkynyl.

Compounds of this invention may contain asymmetrically substituted carbon atoms in the R or S configuration, wherein the terms "R" and "S" are as defined in Pure Appl. Chem. (1976) 45, 13-10. Compounds having asymmetrically substituted carbon atoms with equal amounts of R and S configurations are racemic at those atoms. Atoms having excess of one configuration over the other are assigned the configuration in excess, preferably an excess of about 85%-90%, more preferably an excess of about 95%-99%, and still more preferably an excess greater than about 99%. Accordingly, this invention is meant to embrace racemic mixtures and relative and absolute diastereoisomers of the compounds thereof.

Compounds of this invention may also contain carbon-carbon double bonds or carbon-nitrogen double bonds in the Z or E configuration, in which the term "Z" represents the larger two substituents on the same side of a carbon-carbon or carbon-nitrogen double bond and the term "E" represents the larger two substituents on opposite sides of a carbon-carbon or carbon-nitrogen double bond. The compounds of this invention may also exist as a mixture of "Z" and "E" isomers.

Compounds of this invention may also exist as tautomers or equilibrium mixtures thereof wherein a proton of a compound shifts from one atom to another. Examples of tautomers include, but are not limited to, keto-enol, phenol-keto, oxime-nitroso, nitro-aci, imine-enamine and the like.

Compounds of this invention containing NH, C(O)OH, OH or SH moieties may have attached thereto prodrug-forming moieties. The prodrug-forming moieties are removed by metabolic processes and release the compounds having the freed NH, C(O)OH, OH or SH in vivo. Prodrugs are useful for adjusting such pharmacokinetic properties of the compounds as solubility and/or hydrophobicity, absorption in the gastrointestinal tract, bioavailability, tissue penetration, and rate of clearance.

Metabolites of compounds having Formula I produced by in vitro or in vivo metabolic processes, may also have utility for treating diseases associated with overexpression or unregulation of protein kinases.

Certain precursor compounds which may be metabolized in vitro or in vivo to form compounds having Formula I may also have utility for treating diseases associated with overexpression or unregulation of protein kinases.

Compounds having Formula I may exist as acid addition salts, basic addition salts or zwitterions. Salts of compounds having Formula I are prepared during their isolation or following their purification. Acid addition salts are those derived from the reaction of a compound having Formula I with acid. Accordingly, salts including the acetate, adipate, alginate, bicarbonate, citrate, aspartate, benzoate, benzenesulfonate (besylate), bisulfate, butyrate, camphorate, camphorsufonate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfonate, naphthylenesulfonate, nicotinate, oxalate, pamoate, pectinate, persulfate, phosphate, picrate, propionate, succinate, tartrate, thiocyanate, trichloroacetic, trifluoroacetic, para-toluenesulfonate and undecanoate salts of the compounds having Formula I are meant to be embraced by this invention. Basic addition salts of compounds are those derived from the reaction of the compounds having Formula I with the bicarbonate, carbonate, hydroxide or phosphate of cations such as lithium, sodium, potassium, calcium and magnesium.

Compounds having Formula I may be administered, for example, bucally, ophthalmically, orally, osmotically, parenterally (intramuscularly, intraperintoneally intrasternally, intravenously, subcutaneously), rectally, topically, transdermally, vaginally and intraarterially as well as by intraarticular injection, infusion, and placement in the body, such as, for example, the vasculature.

Therapeutically effective amounts of a compound having Formula I depend on recipient of treatment, disease treated and severity thereof, composition comprising it, time of administration, route of administration, duration of treatment, potency, rate of clearance and whether or not another drug is co-administered. The amount of a compound having Formula I used to make a composition to be administered daily to a patient in a single dose or in divided doses is from about 0.03 to about 200 mg/kg body weight. Single dose compositions contain these amounts or a combination of submultiples thereof.

Compounds having Formula I may be administered with or without an excipient. Excipients include, but are not limited to, encapsulators and additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents, mixtures thereof and the like.

Excipients for preparation of compositions comprising a compound having Formula I to be administered orally include, but are not limited to, agar, alginic acid, aluminum hydroxide, benzyl alcohol, benzyl benzoate, 1,3-butylene glycol, carbomers, castor oil, cellulose, cellulose acetate, cocoa butter, com starch, com oil, cottonseed oil, cross-povidone, diglycerides, ethanol, ethyl cellulose, ethyl laureate, ethyl oleate, fatty acid esters, gelatin, germ oil, glucose, glycerol, groundnut oil, hydroxypropylmethyl celluose, isopropanol, isotonic saline, lactose, magnesium hydroxide, magnesium stearate, malt, mannitol, monoglycerides, olive oil, peanut oil, potassium phosphate salts, potato starch, povidone, propylene glycol, Ringer's solution, safflower oil, sesame oil, sodium carboxymethyl cellulose, sodium phosphate salts, sodium lauryl sulfate, sodium sorbitol, soybean oil, stearic acids, stearyl fumarate, sucrose, surfactants, talc, tragacanth, tetrahydrofurfuryl alcohol, triglycerides, water, mixtures thereof and the like. Excipients for preparation of compositions comprising a compound having Formula I to be administered ophthalmically or orally include, but are not limited to, 1,3-butylene glycol, castor oil, com oil, cottonseed oil, ethanol, fatty acid esters of sorbitan, germ oil, groundnut oil, glycerol, isopropanol, olive oil, polyethylene glycols, propylene glycol, sesame oil, water, mixtures thereof and the like. Excipients for preparation of compositions comprising a compound having Formula I to be administered osmotically include, but are not limited to, chlorofluorohydrocarbons, ethanol, water, mixtures thereof and the like. Excipients for preparation of compositions comprising a compound having Formula I to be administered parenterally include, but are not limited to, 1,3-butanediol, castor oil, com oil, cottonseed oil, dextrose, germ oil, groundnut oil, liposomes, oleic acid, olive oil, peanut oil, Ringer's solution, safflower oil, sesame oil, soybean oil, U.S.P. or isotonic sodium chloride solution, water, mixtures thereof and the like. Excipients for preparation of compositions comprising a compound having Formula I to be administered rectally or vaginally include, but are not limited to, cocoa butter, polyethylene glycol, wax, mixtures thereof and the like.

Compounds having Formula I are also expected to be useful as chemotherapeutic agents in combination with actinomycins, alkylating agents, anthracyclines, antifolates, antiestrogen agents, anti-metabolites, anti-androgens, antimicrotubule agents, aromatase inhibitors, bleomycins, Ca²⁺ adenosine triphosphate (ATP)ase inhibitors, cytosine analogs, deltoids/retinoids, dihydrofolate reductase inhibitors, deoxyribonucleic acid (DNA) topoisomerase inhibitors, dopaminergic neurotoxins, glucocorticoids, histone deacetylase inhibitors, hormonal therapies, immunotherapeutic agents, inosine monophosphate (IMP) dehydrogenase inhibitors, isoprenylation inhibitors, luteinizing hormone-releasing hormone agonists, mammalian target of rapamycin (mtor) inhibitors, multi-drug resistance (MDR) inhibitors, mitomycins, photodyamic therapies, proteasome inhibitors, platinum containing compounds, radiation, receptor tyrosine kinase inhibitors, ribonuclotide reductase inhibitors, thrombospondin mimetics, uracil analogs, vinca alkaloids, and vitamin D3 analogs such as, but not limited to, γ-radiation or an additional chemotherapeutic agent or additional chemotherapeutic agents such as N-Ac-Sar-Gly-Val-D-allolle-Thr-Nva-Ile-Arg-Pro-NHCH₂CH₃ or a salt thereof, actinomycin D, AG13736, 17-allylamino-17-demethoxygeldanamycin, 9-aminocamptothecin, N-(4-(3-amino-1H-indazol-4-yl)phenyl)-N'-(2-fluoro-5-methylphenyl)urea or a salt thereof, N-(4-(4-aminothieno(2,3-d)pyrimidin-5-yl)phenyl)-N'-(2-fluoro-5-(trifluoromethyl)phenyl)urea or a salt thereof, anastozole, AP-23573, asparaginase, azacitidine, bevacizumab, bicalutamide, bleomycin a2, bleomycin b2, bortezamib, busulfan, campathecins, carboplatin, carmustine (BCNU), CB1093, cetuximab, CHOP (C: Cytoxan® (cyclophosphamide); H: Adriamycin® (hydroxydoxorubicin); O: Vincristine (Oncovin®); P: prednisone), chlorambucil, CHIR258, cisplatin, CNF-101, CNF-1001, CNF-2024, CP547632, crisnatol, cytarabine, cyclophosphamide, cytosine arabinoside, daunorubicin, dacarbazine, dactinomycin, dasatinib, daunorubicin, deferoxamine, demethoxyhypocrellin A, depsipeptide, dexamethasone, 17-dimethylaminoethylamino-17-demethoxygeldanamycin, docetaxel, doxifluridine, doxorubicin, EB1089, epothilone D, epirubicin, 5-ethynyl-1-β-D-ribofuranosylimidazole-4-carboxamide (EICAR), erlotinib, etoposide, everolimus, 5-fluorouracil (5-FU), floxuridine, fludarabine, flutamide, gefitinib, geldanamycin, gemcitabine, goserelin, N-(2-(4-hydroxyanilino)-3-pyridinyl)-4-methoxybenzenesulfonamide or a salt thereof, hydroxyurea, idarubicin, ifosfamide, imatinab, interferon-α, interferon-γ, IPI-504, irinotecan, KH 1060, lapatanib, LAQ824, leuprolide acetate, letrozole, lomustine (CCNU), lovastatin, megestrol, melphalan, mercaptopurine, methotrexate, 1-methyl-4-phyenylpyridinium, MG132, mitomycin, mitoxantrone, MLN-518, MS-275, mycophenolic acid, mitomycin C, nitrosoureas, oxaliplatin, paclitaxel, PD98059, peplomycin, photosensitizer Pc4, phtalocyanine, pirarubicin, plicamycin, prednisone, procarbizine, PTK787, PU24FCl, PU3, radicicol, raloxifene, rapamycin, ratitrexed, retinoids such as pheuretinide, ribavirin, rituximab (Rituxin®), sorafenib, staurosporine, steroids such as dexamethasone and prednisone, suberoylanilide hydroxamic acid, sunitinib, tamoxifen, taxol, temozolamide, temsirolimus, teniposide, thapsigargin, thioguanine, thrombospondin-1, tiazofurin, topotecan, trapoxin, trastuzumab, treosulfan, trichostatin A, trimetrexate, trofosfamide, tumor necrosis factor, valproic acid, VER49009, verapamil, vertoporfin, vinblastine, vincristine, vindesine, vinorelbine vitamin D3, VX-680, zactima, ZK-EPO, zorubicin or combinations thereof.

To determine activity of representative compounds of the invention, Active Aurora B enzyme (recombinant residues 1-344) and INCENP (recombinant GST fusion protein from Upstate) were incubated in wells of a 384 well plate with biotinylted histone H3 peptide residues 1-21 (Upstate), I mM ATP, and various concentrations of inhibitors in a Hepes buffer, pH 7.4 containing MgCl₂, sodium othrovanadate, and Triton X-100. After 1 hour, the reaction was stopped with EDTA and anti-phospho-histone H3 Europium Cryptate (Cis-Bio) and SA-APC (Phycolink, Prozyme) were added to detect the phosphopeptide. The amount of phosphorylation was determined by the time-resolved fluorescence ratio of signals at 665 nm and 615 nm. The IC₅₀'s were calculated by an exponential fit of the inhibition values with the inhibitor concentrations using Assay Explorer software and are shown in TABLE 1.

**TABLE 1**

| | | | |
|---|---|---|---|
| 0.003 | 0.005 | 0.008 | 0.008 |
| 0.008 | 0.009 | 0.010 | 0.010 |
| 0.011 | 0.014 | 0.015 | 0.017 |
| 0.020 | 0.025 | 0.027 | 0.029 |
| 0.029 | 0.035 | 0.035 | 0.036 |
| 0.038 | 0.045 | 0.071 | 0.087 |
| 0.087 | 0.103 | 0.146 | 0.168 |
| 0.192 | 0.440 | 0.670 | 0.715 |
| 1.016 | 1.596 | 1.765 | 1.817 |

These data demonstrate the utility of compounds having Formula I as inhibitors of Aurora-kinase B.

It is expected that, because compounds having Formula I inhibit the activity of Aurora-kinase B, they could also have utility as inhibitors of protein kinases having close structural homology thereto, such as, for example, Aurora-kinase A and Aurora-kinase C.

The structural homology between Protein Kinases A, B and C is reported in Nature Reviews/Cancer, Vol. 4 december, 2004.

Accordingly, compounds having Formula I are expected to have utility in treatment of diseases during which protein kinases such as any or all Aurora-kinase family members are expressed.

Diseases involving overexpression or unregulation of Aurora-kinase family members include, but are not limited to, acoustic neuroma, acute leukemia, acute lymphocytic leukemia, acute myelocytic leukemia (monocytic, mycloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myleogeneous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, heavy chain disease, hemangioblastoma, hepatoma, hepatocellular cancer, hormone insensitive prostate cancer, leiomyosarcoma, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenström's macroglobulinemia, testicular tumors, uterine cancer and Wilms' tumor.

It is also expected that compounds having Formula I would inhibit the growth of cells derived from a cancer or neoplasm such as breast cancer (including estrogen-receptor positive breast cancer), colorectal cancer, endometrial cancer, lung cancer (including small cell lung cancer), lymphoma (including follicular or Diffuse Large B-cell), lymphoma (including non-Hodgkin's lymphoma), neuroblastoma, ovarian cancer, prostate cancer (including hormone-insensitive prostate cancer) and testicular cancer (including germ cell testicular cancer).

It is also expected that compounds having Formula I would inhibit the growth of cells derived from a pediatric cancer or neoplasm such as embryonal rhabdomyosarcoma, pediatric acute lymphoblastic leukemia, pediatric acute myelogenous leukemia, pediatric alveolar rhabdomyosarcoma, pediatric anaplastic ependymoma, pediatric anaplastic large cell lymphoma, pediatric anaplastic medulloblastoma, pediatric atypical teratoid/rhabdoid tumor of the central nervous syatem, pediatric biphenotypic acute leukemia, pediatric Burkitts lymphoma, pediatric cancers of Ewing's family of tumors such as primitive neuroectodermal rumors, pediatric diffuse anaplastic Wilm's tumor, pediatric favorable histology Wilm's tumor, pediatric glioblastoma, pediatric medulloblastoma, pediatric neuroblastoma, pediatric neuroblastoma-derived myelocytomatosis, pediatric pre-B-cell cancers (such as leukemia), pediatric psteosarcoma, pediatric rhabdoid kidney tumor, pediatric rhabdomyosarcoma, and pediatric T-cell cancers such as lymphoma and skin cancer.

Compounds having Formula I are expected to be useful when used with alkylating agents, angiogenesis inhibitors, antibodies, antimetabolites, antimitotics, antiproliferatives, aurora kinase inhibitors, Bcl-2 family protein (for example, Bcl-xL, Bcl-2, Bcl-w, Bfl-1) inhibitors, Bcr-Abl kinase inhibitors, biologic response modifiers, cyclin-dependent kinase inhibitors, cell cycle inhibitors, cyclooxygenase-2 inhibitors, leukemia viral oncogene homolog (ErbB2) receptor inhibitors, growth factor inhibitors, heat shock protein HSP-90 inhibitors, histone deacetylase (HDAC) inhibitors inhibitors, hormonal therapies, immunologicals, intercalating antibiotics, kinase inhibitors, mammalian target of rapomycin inhibitors, mitogen-activated extracellular signal-regulated kinase inhibitors, non-steroidal anti-inflammatory drugs (NSAID's), platinum chemotherapeutics, polo-like kinase inhibitors, proteasome inhibitors, purine analogs, pyrimidine analogs, receptor tyrosine kinase inhibitors, retinoids/deltoids plant alkaloids, topoisomerase inhibitors and the like.

Alkylating agents include altretamine, AMD-473, AP-5280, apaziquone, bendamustine, brostallicin, busulfan, carboquone, carmustine (BCNU), chlorambucil, Cloretazine™ (VNP 40101M), cyclophosphamide, decarbazine, estramustine, fotemustine, glufosfamide, ifosfamide, KW-2170, lomustine (CCNU), mafosfamide, melphalan, mitobronitol, mitolactol, nimustine, nitrogen mustard N-oxide, ranimustine, temozolomide, thiotepa, treosulfan, trofosfamide and the like.

Angiogenesis inhibitors include endothelial-specific receptor tyrosine kinase (Tie-2) inhibitors, epidermal growth factor receptor (EGFR) inhibitors, insulin growth factor-2 receptor (IGFR-2) inhibitors, matrix metalloproteinase-2 (MMP-2) inhibitors, matrix metalloproteinase-9 (MMP-9) inhibitors, platelet-derived growth factor receptor (PDGFR) inhibitors, thrombospondin analogs vascular endothelial growth factor receptor tyrosine kinase (VEGFR) inhibitors and the like.

Aurora kinase inhibitors include AZD-1152, MLN-8054, VX-680 and the like.

Bcl protein family member inhibitors include AT-101 ((-)gossypol), GENASENSE^{®} (G3139 or oblimersen (Bcl-2-targeting antisense oglionucleotide)), IPI-194, IPI-565, N-(4-(4-((4'-chloro(1,1'-biphenyl)-2-yl)methyl)piperazin-1-yl)benzoyl)-4-(((1R)-3-(dimethylamino)-1-((phenylsulfanyl)methyl)propyl)amino)-3-nitrobenzenesulfonamide) (ABT-737), N-(4-(4-((2-(4-chlorophenyl)-5,5-dimethyl-1-cyclohex-1-en-1-yl)methyl)piperazin-1-yl)benzoyl)-4-(((1R)-3-(morpholin-4-yl)-1-((phenylsulfanyl)methyl)propyl)amino)-3-((trifluoromethyl)sulfonyl)benzenesulfonamide (ABT-263), GX-070 (obatoclax) and the like.

Bcr-Abl kinase inhibitors include DASATINIB^{®} (BMS-354825), GLEEVEC^{®} (imatinib) and the like.

CDK inhibitors include AZD-5438, BMI-1040, BMS-032, BMS-387, CVT-2584, flavopyridol, GPC-286199, MCS-5A, PD0332991, PHA-690509, seliciclib (CYC-202, R-roscovitine), ZK-304709 and the like.

COX-2 inhibitors include ABT-963, ARCOXIA^{®} (etoricoxib), BEXTRA^{®} (valdecoxib), BMS347070, CELEBREX™ (celecoxib), COX-189 (lumiracoxib), CT-3, DERAMAXX^{®} (deracoxib), JTE-522, 4-methyl-2-(3,4-dimethylphenyl)-1-(4-sulfamoylphenyl-1H-pyrrole), MK-663 (etoricoxib), NS-398, parecoxib, RS-57067, SC-58125, SD-8381, SVT-2016, S-2474, T-614, VIOXX^{®} (rofecoxib) and the like.

EGFR inhibitors include ABX-EGF, anti-EGFr immunoliposomes, EGF-vaccine, EMD-7200, ERBITUX^{®} (cetuximab), HR3, IgA antibodies, IRESSA^{®} (gefitinib), TARCEVA^{®} (erlotinib or OSI-774), TP-38, EGFR fusion protein, TYKERB^{®} (lapatinib) and the like.

ErbB2 receptor inhibitors include CP-724-714, CI-1033 (canertinib), Herceptin^{®} (trastuzumab), TYKERB^{®} (lapatinib), OMNITARG^{®} (2C4, petuzumab), TAK-165, GW-572016 (ionafarnib), GW-282974, EKB-569, PI-166, dHER2 (HER2 vaccine), APC-8024 (HER-2 vaccine), anti-HER/2neu bispecific antibody, B7.her2lgG3, AS HER2 trifunctional bispecfic antibodies, mAB AR-209, mAB 2B-1 and the like.

Histone deacetylase inhibitors include depsipeptide, LAQ-824, MS-275, trapoxin, suberoylanilide hydroxamic acid (SAHA), TSA, valproic acid and the like.

HSP-90 inhibitors include 17-AAG-nab, 17-AAG, CNF-101, CNF-1010, CNF-2024, 17-DMAG, geldanamycin, IPI-504, KOS-953, MYCOGRAB^{®}, NCS-683664, PU24FCl, PU-3, radicicol, SNX-2112, STA-9090 VER49009 and the like.

MEK inhibitors include ARRY-142886, ARRY-438162 PD-325901, PD-98059 and the like.

mTOR inhibitors include AP-23573, CCI-779, everolimus, RAD-001, rapamycin, temsirolimus and the like.

Non-steroidal anti-inflammatory drugs include AMIGESIC^{®} (salsalate), DOLOBID^{®} (diflunisal), MOTRIN^{®} (ibuprofen), ORUDIS^{®} (ketoprofen), RELAFEN^{®} (nabumetone), FELDENE^{®} (piroxicam) ibuprofin cream, ALEVE^{®} and NAPROSYN^{®} (naproxen), VOLTAREN^{®} (diclofenac), INDOCIN^{®} (indomethacin), CLINORIL^{®} (sulindac), TOLECTIN^{®} (tolmetin), LODINE^{®} (etodolac), TORADOL^{®} (ketorolac), DAYPRO^{®} (oxaprozin) and the like.

PDGFR inhibitors include C-451, CP-673, CP-868596 and the like.

Platinum chemotherapeutics include cisplatin, ELOXATIN^{®} (oxaliplatin) eptaplatin, lobaplatin, nedaplatin, PARAPLATIN^{®} (carboplatin), satraplatin and the like.

Polo-like kinase inhibitors include BI-2536 and the like.

Thrombospondin analogs include ABT-510, ABT-567, ABT-898, TSP-1 and the like.

VEGFR inhibitors include AVASTIN^{®} (bevacizumab), ABT-869, AEE-788, ANGIOZYME™, axitinib (AG-13736), AZD-2171, CP-547,632, IM-862, Macugen (pegaptamib), NEXAVAR^{®} (sorafenib, BAY43-9006), pazopanib (GW-786034), (PTK-787, ZK-222584), SUTENT^{®} (sunitinib, SU-11248), VEGF trap, vatalanib, ZACTIMA™ (vandetanib, ZD-6474) and the like.

Antimetabolites include ALIMTA^{®} (premetrexed disodium, LY231514, MTA), 5-azacitidine, XELODA^{®} (capecitabine), carmofur, LEUSTAT^{®} (cladribine), clofarabine, cytarabine, cytarabine ocfosfate, cytosine arabinoside, decitabine, deferoxamine, doxifluridine, eflornithine, EICAR, enocitabine, ethnylcytidine, fludarabine, hydroxyurea, 5-fluorouracil (5-FU) alone or in combination with leucovorin, GEMZAR^{®} (gemcitabine), hydroxyurea, ALKERAN^{®}(melphalan), mercaptopurine, 6-mercaptopurine riboside, methotrexate, mycophenolic acid, nelarabine, nolatrexed, ocfosate, pelitrexol, pentostatin, raltitrexed, Ribavirin, triapine, trimetrexate, S-1, tiazofurin, tegafur, TS-1, vidarabine, UFT and the like.

Antibiotics include intercalating antibiotics aclarubicin, actinomycin D, amrubicin, annamycin, adriamycin, BLENOXANE^{®} (bleomycin), daunorubicin, CAELYX^{®} or MYOCET^{®} (doxorubicin), elsamitrucin, epirbucin, glarbuicin, ZAVEDOS^{®} (idarubicin), mitomycin C, nemorubicin, neocarzinostatin, peplomycin, pirarubicin, rebeccamycin, stimalamer, streptozocin, VALSTAR^{®} (valrubicin), zinostatin and the like.

Topoisomerase inhibitors include aclarubicin, 9-aminocamptothecin, amonafide, amsacrine, becatecarin, belotecan, BN-80915, CAMPTOSAR^{®} (irinotecan hydrochloride), camptothecin, CARDIOXANE^{®} (dexrazoxine), diflomotecan, edotecarin, ELLENCE^{®} or PHARMORUBICIN^{®} (epirubicin), etoposide, exatecan, 10-hydroxycamptothecin, gimatecan, lurtotecan, mitoxantrone, orathecin, pirarbucin, pixantrone, rubitecan, sobuzoxane, SN-38, tafluposide, topotecan and the like.

Antibodies include AVASTIN^{®} (bevacizumab), CD40-specific antibodies, chTNT-1/B, denosumab, ERBITUX^{®} (cetuximab), HUMAX-CD4^{®} (zanolimumab), IGF1R-specific antibodies, lintuzumab, PANOREX^{®} (edrecolomab), RENCAREX^{®} (WX G250), RITUXAN^{®} (rituximab), ticilimumab, trastuzimab and and the like.

Hormonal therapies include ARIMIDEX^{®} (anastrozole), AROMASIN^{®} (exemestane), arzoxifene, CASODEX^{®} (bicalutamide), CETROTIDE^{®} (cetrorelix), degarelix, deslorelin, DESOPAN^{®} (trilostane), dexamethasone, DROGENIL^{®}, (flutamide), VISTA^{®} (raloxifene), fadrozole, FARESTON^{®} (toremifene), FASLODEX^{®} (fulvestrant), FEMARA^{®}, (letrozole), formestane, glucocorticoids, HECTOROL^{®} or RENAGEL^{®} (doxercalciferol), lasofoxifene, leuprolide acetate, MEGACE^{®} (megesterol), MIFEPREX^{®} (mifepristone), NILANDRON™ (nilutamide), NOLVADEX^{®} (tamoxifen citrate), PLENAXIS™ (abarelix), predisone, PROPECIA^{®} (finasteride), rilostane, SUPREFACT^{®} (buserelin), TRELSTAR^{®} (luteinizing hormone releasing hormone (LHRH)), vantas, VETORYL^{®}, (trilostane or modrastane), ZOLADEX^{®} (fosrelin, goserelin) and the like.

Deltoids and retinoids include seocalcitol (EB1089, CB1093), lexacalcitrol (KH1060), fenretinide, PANRETIN^{®} (aliretinoin), ATRAGEN^{®} (liposomal tretinoin), TARGRETIN^{®}(bexarotene), LGD-1550 and the like.

Plant alkaloids include, but are not limited to, vincristine, vinblastine, vindesine, vinorelbine and the like.

Proteasome inhibitors include VELCADE^{®} (bortezomib), MG132, NP1-0052, PR-171 and the like.

Examples of immunologicals include interferons and other immune-enhancing agents. Interferons include interferon alpha, interferon alpha-2a, interferon alpha-2b, interferon beta, interferon gamma-la, ACTIMMUNE^{®} (interferon gamma-1b), or interferon gamma-n1, combinations thereof and the like. Other agents include ALFAFERONE^{®} BAM-002, BEROMUN^{®} (tasonermin), BEXXAR^{®} (tositumomab), CamPath^{®} (alemtuzumab), CTLA4 (cytotoxic lymphocyte antigen 4), decarbazine, denileukin, epratuzumab, GRANOCYTE^{®} (lenograstim), lentinan, leukocyte alpha interferon, imiquimod, MDX-010, melanoma vaccine, mitumomab, molgramostim, MYLOTARG™ (gemtuzumab ozogamicin), NEUPOGEN^{®} (filgrastim), OncoVAC-CL, OvaRex^{®} (oregovomab), pemtumomab (Y-muHMFG1), PROVENGE^{®}, sargaramostim, sizofilan, teceleukin, TheraCys^{®}, ubenimex, VIRULIZIN^{®}, Z-100, WF-10, PROLEUKIN^{®} (aldesleukin), ZADAXIN^{®} (thymalfasin), ZENAPAX^{®} (daclizumab), ZEVALIN^{®} (90Y-Ibritumomab tiuxetan) and the like.

Biological response modifiers are agents that modify defense mechanisms of living organisms or biological responses, such as survival, growth, or differentiation of tissue cells to direct them to have anti-tumor activity and include include krestin, lentinan, sizofiran, picibanil PF-3512676 (CpG-8954), ubenimex and the like.

Pyrimidine analogs include cytarabine (ara C or Arabinoside C), cytosine arabinoside, doxifluridine, FLUDARA^{®} (fludarabine), 5-FU (5-fluorouracil), floxuridine, GEMZAR^{®} (gemcitabine), TOMUDEX^{®} (ratitrexed), TROXATYL™ (triacetyluridine troxacitabine) and the like.

Purine analogs include LANVIS^{®} (thioguanine) and PURI-NETHOL^{®} (mercaptopurine).

Antimitotic agents include batabulin, epothilone D (KOS-862), N-(2-((4-hydroxyphenyl)amino)pyridin-3-yl)-4-methoxybenzenesulfonamide, ixabepilone (BMS 247550), paclitaxel, TAXOTERE^{®} (docetaxel), PNU100940 (109881), patupilone, XRP-9881, vinflunine, ZK-EPO and the like.

Compounds of the present invention are also intended to be used as a radiosensitizer that enhances the efficacy of radiotherapy. Examples of radiotherapy include, but are not limited to, external beam radiotherapy, teletherapy, brachtherapy and sealed and unsealed source radiotherapy.

Additionally, compounds having Formula I may be combined with other chemptherapeutic agents such as ABRAXANE™ (ABI-007), ABT-100 (farnesyl transferase inhibitor), ADVEXIN^{®}, ALTOCOR^{®} or MEVACOR^{®} (lovastatin), AMPLIGEN^{®} (poly I:poly C12U, a synthetic RNA), APTOSYN™ (exisulind), AREDIA^{®} (pamidronic acid), arglabin, L-asparaginase, atamestane (1-methyl-3,17-dione-androsta-1,4-diene), AVAGE^{®} (tazarotne), AVE-8062, BEC2 (mitumomab), cachectin or cachexin (tumor necrosis factor), canvaxin (vaccine), CeaVac™ (cancer vaccine), CELEUK^{®} (celmoleukin), CEPLENE^{®} (histamine dihydrochloride), CERVARIX^{®} (human papillomavirus vaccine), CHOP^{®} (C: CYTOXAN^{®} (cyclophosphamide); H: ADRIAMYCIN^{®} (hydroxydoxorubicin); O: Vincristine (ONCOVIN^{®}); P: prednisone), CyPat™, combrestatin A4P, DAB(389)EGF or TransMID-107R™ (diphtheria toxins), dacarbazine, dactinomycin, 5,6-dimethylxanthenone-4-acetic acid (DMXAA), eniluracil, EVIZON™ (squalamine lactate), DIMERICINE^{®} (T4N5 liposome lotion), discodermolide, DX-8951f (exatecan mesylate), enzastaurin, EP0906, GARDASIL^{®} (quadrivalent human papillomavirus (Types 6, 11, 16, 18) recombinant vaccine), gastrimmune, genasense, GMK (ganglioside conjugate vaccine), GVAX^{®} (prostate cancer vaccine), halofuginone, histerelin, hydroxycarbamide, ibandronic acid, IGN-101, IL-13-PE38, IL-13-PE38QQR (cintredekin besudotox), IL-13-pseudomonas exotoxin, interferon-α, interferon-y, JUNOVAN™ or MEPACT™ (mifamurtide), lonafarnib, 5,10-methylenetetrahydrofolate, miltefosine (hexadecylphosphocholine), NEOVASTAT^{®} (AE-941), NEUTREXIN^{®} (trimetrexate glucuronate), NIPENT^{®} (pentostatin), ONCONASE^{®} (a ribonuclease enzyme), ONCOPHAGE^{®} (melanoma vaccine treatment), OncoVAX (IL-2 Vaccine), ORATHECIN™ (rubitecan), OSIDEM^{®} (antibody-based cell drug), OvaRex^{®} MAb ( murine monoclonal antibody), paditaxel, PANDIMEX™ (aglycone saponins from ginseng comprising 20(S)protopanaxadiol (aPPD) and 20(S)protopanaxatriol (aPPT)), panitumumab, PANVAC^{®}-VF (investigational cancer vaccine), pegaspargase, PEG Interferon A, phenoxodiol, procarbazine, rebimastat, REMOVAB^{®} (catumaxomab), REVLIMID^{®} (lenalidomide), RSR13 (efaproxiral), SOMATULINE^{®} LA (lanreotide), SORIATANE^{®} (acitretin), staurosporine (Streptomyces staurospores), talabostat (PT100), TARGRETIN^{®} (bexarotene), Taxoprexin^{®} (DHA-paclitaxel), TELCYTA™ (TLK286), temilifene, TEMODAR^{®} (temozolomide), tesmilifene, thalidomide, THERATOPE^{®} (STn-KLH), thymitaq (2-amino-3,4-dihydro-6-methyl-4-oxo-5-(4-pyridylthio)quinazoline dihydrochloride), TNFerade™ (adenovector: DNA carrier containing the gene for tumor necrosis factor-α), TRACLEER^{®} or ZAVESCA^{®} (bosentan), tretinoin (Retin-A), tetrandrine, TRISENOX^{®} (arsenic trioxide), VIRULIZIN^{®}, ukrain (derivative of alkaloids from the greater celandine plant), vitaxin (anti-alphavbeta3 antibody), XCYTRIN^{®} (motexafin gadolinium), XINLAY™ (atrasentan), XYOTAX™ (paclitaxel poliglumex), YONDELIS™ (trabectedin), ZD-6126, ZINECARD^{®} (dexrazoxane), zometa (zolendronic acid), zorubicin and the like.

It is also expected that compounds having Formula 1 would inhibit growth of cells derived from a pediatric cancer or neoplasm including embryonal rhabdomyosarcoma, pediatric acute lymphoblastic leukemia, pediatric acute myelogenous leukemia, pediatric alveolar rhabdomyosarcoma, pediatric anaplastic ependymoma, pediatric anaplastic large cell lymphoma, pediatric anaplastic medulloblastoma, pediatric atypical teratoid/rhabdoid tumor of the central nervous syatem, pediatric biphenotypic acute leukemia, pediatric Burkitts lymphoma, pediatric cancers of Ewing's family of tumors such as primitive neuroectodermal rumors, pediatric diffuse anaplastic Wilm's tumor, pediatric favorable histology Wilm's tumor, pediatric glioblastoma, pediatric medulloblastoma, pediatric neuroblastoma, pediatric neuroblastoma-derived myelocytomatosis, pediatric pre-B-cell cancers (such as leukemia), pediatric psteosarcoma, pediatric rhabdoid kidney tumor, pediatric rhabdomyosarcoma, and pediatric T-cell cancers such as lymphoma and skin cancer and the like (commonly-owned United States Application Serial No. 10/988,338), Cancer Res., 2000, 60, 6101-10); and autoimmune disorders include, acquired immunodeficiency disease syndrome, autoimmune lymphoproliferative syndrome, hemolytic anemia, inflammatory diseases, thrombocytopenia and the like (Current Allergy and Asthma Reports 2003, 3:378-384; Br. J. Haematol. 2000 Sep; 110(3): 584-90; Blood 2000 Feb 15;95(4):1283-92; and New England Journal of Medicine 2004 Sep; 351(14): 1409-1418).

For example, involvement of Aurora-kinases in bladder cancer, breast cancer, cervical cancer, colon cancer, endometrial cancer, esophageal cancer, lung cancer, ovarian cancer, pancreatic cancer, prostate cancer, rectal cancer, skin cancer, stomach cancer and thyroid cancer is reported in Nature Reviews/Cancer, Vol. 4 december, 2004.

Compounds having Formula I may be made by synthetic chemical processes, examples of which are shown hereinbelow. It is meant to be understood that the order of the steps in the processes may be varied, that reagents, solvents and reaction conditions may be substituted for those specifically mentioned, and that vulnerable moieties may be protected and deprotected, as necessary.

Protecting groups for C(O)OH moieties include, but are not limited to, acetoxymethyl, allyl, benzoylmethyl, benzyl, benzyloxymethyl, tert-butyl, tert-butyldiphenylsilyl, diphenylmethyl, cyclobutyl, cyclohexyl, cyclopentyl, cyclopropyl, diphenylmethylsilyl, ethyl, para-methoxybenzyl, methoxymethyl, methoxyethoxymethyl, methyl, methylthiomethyl, naphthyl, para-nitrobenzyl, phenyl, n-propyl, 2,2,2-trichloroethyl, triethylsilyl, 2-(trimethylsilyl)ethyl, 2-(trimethylsilyl)ethoxymethyl, triphenylmethyl and the like.

Protecting groups for C(O) and C(O)H moieties include, but are not limited to, 1,3-dioxylketal, diethylketal, dimethylketal, 1,3-dithianylketal, O-methyloxime, O-phenyloxime and the like.

Protecting groups for NH moieties include, but are not limited to, acetyl, alanyl, benzoyl, benzyl (phenylmethyl), benzylidene, benzyloxycarbonyl (Cbz), tert-butoxycarbonyl (Boc), 3,4-dimethoxybenzyloxycarbonyl, diphenylmethyl, diphenylphosphoryl, formyl, methanesulfonyl, para-methoxybenzyloxycarbonyl, phenylacetyl, phthaloyl, succinyl, trichloroethoxycarbonyl, triethylsilyl, trifluoroacetyl, trimethylsilyl, triphenylmethyl, triphenylsilyl, para-toluenesulfonyl and the like.

Protecting groups for OH and SH moieties include, but are not limited to, acetyl, allyl, allyloxycarbonyl, benzyloxycarbonyl (Cbz), benzoyl, benzyl, tert-butyl, tert-butyldimethylsilyl, tert-butyldiphenylsilyl, 3,4-dimethoxybenzyl, 3,4-dimethoxybenzyloxycarbonyl, 1,1-dimethyl-2-propenyl, diphenylmethyl, formyl, methanesulfonyl, methoxyacetyl, 4-methoxybenzyloxycarbonyl, para-methoxybenzyl, methoxycarbonyl, methyl, para-toluenesulfonyl, 2,2,2-trichloroethoxycarbonyl, 2,2,2-trichloroethyl, triethylsilyl, trifluoroacetyl, 2-(trimethylsilyl)ethoxycarbonyl, 2-trimethylsilylethyl, triphenylmethyl, 2-(triphenylphosphonio)ethoxycarbonyl and the like.

The following abbreviations have the meanings indicated. ADDP means 1,1'-(azodicarbonyl)dipiperidine; AD-mix-β means a mixture of (DHQD)₂PHAL, K₃Fe(CN)₆, K₂CO₃ and K₂SO₄); AIBN means 2,2'-azobis(2-methylpropionitrile); 9-BBN means 9-borabicyclo(3.3.1)nonane; Cp means cyclopentadiene; (DHQD)₂PHAL means hydroquinidine 1,4-phthalazinediyl diethyl ether; DBU means 1,8-diazabicyclo(5.4.0)undec-7-ene; DCC means 1,3-dicyclohexylcarbodiimide, DIBAL means diisobutylaluminum hydride; DIEA means diisopropylethylamine; DMAP means N,N-dimethylaminopyridine; DME means 1,2-dimethoxyethane; DMF means N,N-dimethylformamide; dmpe means 1,2-bis(dimethylphosphino)ethane; DMSO means dimethylsulfoxide; dppa means diphenylphosphoryl azide; dppb means 1,4-bis(diphenylphosphino)butane; dppe means 1,2-bis(diphenylphosphino)ethane; dppf means 1,1'-bis(diphenylphosphino)ferrocene; dppm means 1,1-bis(diphenylphosphino)methane; EDAC or EDCI or EDC means 1-(3-dimethylaminopropyl)-3-ethylcarbodiimide; Fmoc means fluorenylmethoxycarbonyl; HATU means O-(7-azabenzotriazol-1-yl)-N,N'N'N'-tetramethyluronium hexafluorophosphate; HMPA means hexamethylphosphoramide; HOAT means 1-hydroxy-7-azabenzotriazole; HOBT means 1-hydroxybenzotriazole hydrate, IPA means isopropyl alcohol; LDA means lithium diisopropylamide; LHMDS means lithium bis(hexamethyldisilylamide); MP-BH₃ means macroporus triethylammonium methylpolystyrene cyanoborohydride; LAH means lithium aluminum hydride; NCS means N-chlorosuccinimide; PyBOP means benzotriazol-1-yloxytripyrrolidinophosphonium hexafluorophosphate; TBTU means O-benzotriazol-1-yl-N,N,N',N'-tetramethyluronium tetrafluoroborate; TDA-1 means tris(2-(2-methoxyethoxy)ethyl)amine; TEA means triethylamine; TFA means trifluoroacetic acid; THF means tetrahydrofuran; NCS means N-chlorosuccinimide; NMM means N-methylmorpholine; NMP means N-methylpyrrolidine; PPh₃ means triphenylphosphine.

The following scheme and examples are presented to provide what is believed to be the most useful and readily understood description of procedures and conceptual aspects of this invention.

Compounds having Formula (1) can be prepared a number of ways, such as ones described in W02005/097800.

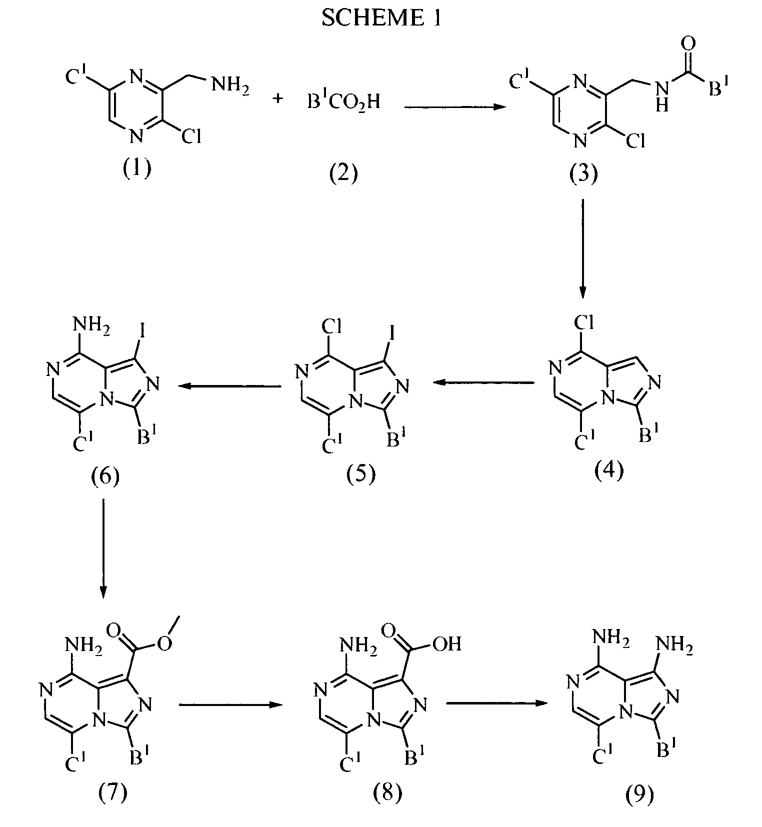

As shown in SCHEME 1, compounds having Formula (1) can be converted to compounds having Formula (3) by reacting the former, compounds having Formula (2), a coupling agent, and a first base, with or without a coupling auxiliary. Examples of coupling agents include DCC, EDCI, HATU, TBTU and the like. Examples of bases include DIEA, TEA, NMM and the like. Examples of coupling auxiliaries include DMAP, HOAT, HOBT and the like. The reaction is typically conducted between about 25°C to 45°C, over about 1 to about 24 hours, in solvents such as THF, DMF, dichloromethane, ethyl acetate, mixtures thereof and the like.

Compounds having Formula (3) can be converted to compounds having Formula (4) by reacting the former, POCl₃ and DMF. The reaction is typically conducted, over about 0.5 to about two hours, in refluxing acetonitrile.

Compounds having Formula (4) can be converted to compounds having Formula (5) by reacting the former and NIS. The reaction is typically conducted, over about one to about five hours, between about 40°C and 80°C in solvents such as DMF and the like.

Compounds having Formula (5) can be converted to compounds having Formula (6) by reacting the former and ammonia. The reaction is typically conducted in a sealed container between about 40°C and 150°C in the ammonia or in solvents such as methanol, ethanol, isopropanol, mixtures thereof and the like.

Compounds having Formula (6) can be converted to compounds having Formula (7) by reacting the former, carbon monoxide, methanol, a palladium catalyst, and a second base. Examples of palladium catalysts include palladium acetate, (1,1'-bis(diphenylphosphino)ferrocene)dichloropalladium(II), and the like. Examples of second bases include TEA, pyridine and the like. The reaction is typically conducted (in a sealed container), over about one to about three hours, between about 80°C and 120°C, in methanol.

Compounds having Formula (7) can be converted to compounds having Formula (8) by reacting the former and a third base. Examples of third bases include lithium hydroxide, sodium hydroxide, potassium hydroxide and the like. The reaction is typically conducted over about 1 hour to about 48 hours, between about 0°C and 35°C, in solvents such as water, methanol, ethanol, isopropanol, mixtures thereof and the like.

Introduction of moieties represented by A¹ can be accomplished by reacting compounds having formula (8), a primary or a secondary amine, a coupling agent, a first base, with or without a coupling auxiliary, using the reaction conditions described hereinabove for conversion of compounds having Formula (1) to compounds having Formula (3).

Compounds having Formula (8) can be converted to compounds having Formula (9) by reacting the former and DPPA followed by hydrolysis of the isocyanate intermediate with water. The reactions are typically conducted over about 1 hour to about 24 hours, between about 50°C and 110°C, in solvents such as benzene, toluene, THF, water, mixtures thereof and the like.

Introduction of moieties represented by A¹ can be accomplished by reacting the compounds having formula (9) and the appropriate isocyanate, carbonyl chloride, sulfonyl chloride, carbamoyl chloride. The reactions are typically conducted over about 1 hour to about 48 hours, between about 0°C and 110°C, in solvents such as THF, ethyl acetate, dichloromethane, DMF, DMSO, chloroform, mixtures thereof and the like.

### EXAMPLE 1A

A mixture of (3-chloropyrazin-2-yl)methanamine (4.9 g), acetic acid (2.25 g), EDC (7.2 g), HOBT (5.75 g) and NMM (6.9 g) in dichloromethane (40 mL) was stirred at room temperature for 48 hours, treated with water and extracted with ethyl acetate. The extract was washed with brine, dried (MgSO₄), filtered and concentrated. The concentrate was purified by flash chromatography on silica gel with 0-5% methanol/ dichloromethane.

### EXAMPLE 1B

A mixture of EXAMPLE 1A (3.98 g) and acetonitrile (100 mL) was treated with DMF (100 µL) and POCl₃ (9.8 mL). The mixture was heated at 55°C for 30 minutes cooled to room temperature and concentrated. The concentrate was dissolved in dichloromethane, neutralized with pre-cooled ammonia in isopropanol and concentrated. The concentrate was partitioned between dichloromethane and water, and the extract was washed with brine and dried (MgSO₄), filtered and concentrated. The concentrate was dissolved in dichloromethane and purified by flash chromatography on silica gel with 0-5% methanol/dichloromethane.

### EXAMPLE I C

A mixture of EXAMPLE I B (2.83 g) and NIS (4.94 g) in DMF (20 mL) was heated at 60°C for 3 hours, cooled to room temperature, diluted with water and extracted with ethyl acetate. The extract was washed with brine, dried (MgSO₄), filtered and concentrated; and the concentrate was triturated with hexanes and filtered.

### EXAMPLE 1D

In a stainless steel reactor, EXAMPLE 1C (3.3 g) in 2M NH₃ in isopropanol (45 mL) and THF (4mL) was cooled with a dry ice-acetone bath and treated with anhydrous NH₃ (15mL). The mixture was heated at 110°C for 48 hours, cooled to room temperature and vented. The mixture was concentrated, and the concentrate was triturated with water and filtered. The filtrate was extracted with ethyl acetate and the extract was washed with brine, dried (MgSO₄), filtered and concentrated. The concentrate was purified by flash chromatography on silica gel with 0-5% methanol/dichloromethane.

### EXAMPLE 1E

A mixture of EXAMPLE 1D (1.3 g), PdCl₂(dppf) (150 mg) and triethylamine (0.480 g) in methanol (10 mL) was purged with CO, sealed and heated at 100°C for 2 hours under 60 psi. The reaction mixture was concentrated and the concentrate was purified by flash chromatography on silica gel with 0-5% methanol/dichloromethane.

### EXAMPLE 1F

A mixture of EXAMPLE 1E (0.87 g) and 2N LiOH (10 mL) in methanol (10 mL) was stirred at room temperature for 3 hours, neutralized with 2N HCl to pH 6-7, and filtered.

### EXAMPLE 1G

1-fluoro-3-isocyanatobenzene (0.56 mL) was added to a solution of (4-aminophenyl)carbamic acid tert-butyl ester (1.04 g) in dichloromethane (20 mL) at 0°C. The mixture was stirred at ambient temperature for 4 hours and filtered. The filtrant was collected was suspended in dichloromethane (20 mL), cooled in an ice bath, treated with TFA (5 mL), stirred for 15 minutes at ambient temperature for 3 hours and concentrated. The concentrate was concentrated twice from methanol and toluene and dried to provide the title compound as the trifluoroacetate salt.

### EXAMPLE 1H

A mixture of TEA (61 mg), EXAMPLE 1F (0.038 g), EXAMPLE 1G trifluoroacatate (0.072 g) and HATU (0.084 g) in DMF (2 mL) was stirred at ambient temperature for 20 hours and extracted with ethyl acetate. The extract was washed with brine, dried (MgSO₄), filtered and concentrated. The concentrate was purified by flash chromatography on silica gel with 0 to 5% methanol/dichloromethane. ¹H NMR (500 MHz, DMSO-d₆) δ 2.62 (s, 3H), 6.55-6.90 (m, 1H), 6.98-7.64 (m, 8H), 7.75 (d, J=8.85 Hz, 2H), 8.71 (s, 1H), 8.88 (s, 1H), 9.26 (s, 1H), 10.22 (s, 1H).

### EXAMPLE 2

This example was prepared by substituting 1-(4-aminophenyl)-3-phenylurea for EXAMPLE 1G in EXAMPLE 1H. ¹H NMR (400 MHz, DMSO-d₆) δ 2.62 (s, 3H), 6.96 (t, J=7.36 Hz, 1H), 7.18 (d, J=4.91 Hz, 1H), 7.28 (t, J=7.83 Hz, 2H), 7.38-7.58 (m, 5H), 7.74 (d, J=8.90 Hz, 2H), 8.62 (s, 2H), 10.18 (s, 1H).

### EXAMPLE 3

This example was prepared by substituting N-(4-aminophenyl)benzamide for EXAMPLE 1G in EXAMPLE 1H. ¹H NMR (300 MHz, DMSO-d₆) δ 2.63 (s, 3H), 7.19 (d, J=4.76 Hz, 1H),7.46-7.64 (m, 4H), 7.71-7.86 (m, 4H), 7.91-8.01 (m, 2H), 10.24 (s, 1H), 10.28 (s, 1H).

### EXAMPLE 4

This example was prepared as described in EXAMPLES 1G-1H by substituting 1-isocyanato-3-(trifluoromethyl)benzene for 1-fluoro-3-isocyanatobenzene in EXAMPLE 1G. ¹H NMR (300 MHz, DMSO-d₆) δ 2.62 (s, 3H), 7.18 (d, J=4.76 Hz, 1H), 7.30 (d, J=7.14 Hz, 1H), 7.39-7.64 (m, 5H), 7.76 (d, J=8.73 Hz, 2H), 8.02 (s, 1H), 8.76 (s, 1H), 9.02 (s, 1H), 10.22 (s, 1H).

### EXAMPLE 5

This example was prepared as described in EXAMPLE 1G-H by substituting tert-butyl 3-(aminomethyl)phenylcarbamate for (4-aminophenyl)carbamic acid tert-butyl ester in EXAMPLE 1G. ¹H NMR (300 MHz, DMSO-d₆) δ 2.62 (s, 3H), 4.31 (d, J=5.95 Hz, 2H), 6.58-6.81 (m, 2H), 7.05 (d, J=7.93 Hz, 2H), 7.15-7.35 (m, 3H), 7.39-7.57 (m, 2H), 7.68 (d, J=9.12 Hz, 1H), 7.83 (s, 1H), 8.82 (s, 1H), 10.27 (s, 1H).

### EXAMPLE 6

This example was prepared as described in EXAMPLE 1G-H by substituting tert-butyl 3-(aminomethyl)phenylcarbamate and isocyanatobenzene for (4-aminophenyl)carbamic acid tert-butyl ester and 1-fluoro-3-isocyanatobenzene, respectively in EXAMPLE 1G. ¹H NMR (300 MHz, DMSO-d₆) δ 2.61 (s, 3H) ,4.30 (d, J=5.76 Hz, 2H), 6.61 (t, J=5.76 Hz, 1H), 6.89 (t, J=7.29 Hz, 1H), 7.06 (d, J=8.14 Hz, 1H), 7.15-7.46 (m, 6H), 7.67 (d, J=8.14 Hz, 1H), 7.67 (d, J=8.14 Hz, 1H), 7.84 (s, 1H), 8.55 (s, 1H), 10.26 (s, 1H).

### EXAMPLE 7

This example was prepared as described in EXAMPLE 1G-H by substituting tert-butyl 3-(aminomethyl)phenylcarbamate and 1-fluoro-2-isocyanatobenzene for (4-aminophenyl)carbamic acid tert-butyl ester and 1-fluoro-3-isocyanatobenzene, respectively in EXAMPLE 1G. ¹H NMR (300 MHz, DMSO-d₆) δ 2.62 (s, 3H) 4.33 (d, J=5.76 Hz, 2H) 6.87-6.98 (m, 1H) 7.03-7.24 (m, 5H) 7.32 (t, J=7.80 Hz, 1H) 7.55 (d, J=4.75 Hz, 1H) 7.62-7.74 (m, 1H) 7.84 (s, 1H) 8.00-8.22 (m, 1H) 8.39 (d, J=2.37 Hz, 1H) 10.31 (s, 1H).

### EXAMPLE 8

This example was prepared as described in EXAMPLE 1G-H by substituting tert-butyl 3-(aminomethyl)phenylcarbamate and 1-fluoro-4-isocyanatobenzene for (4-aminophenyl)carbamic acid tert-butyl ester and 1-fluoro-3-isocyanatobenzene, respectively, in EXAMPLE 1G. ¹H NMR (300 MHz, DMSO-d₆) δ 2.61 (s, 3H) 4.30 (d, J=5.76 Hz, 2H) 6.61 (t, J=5.76 Hz, 1H) 6.97-7.13 (m, 3H) 7.19 (d, J=4.75 Hz, 1H) 7.30 (t, J=7.80 Hz, 1H) 7.36-7.46 (m, 3H) 7.52 (d, J=4.75 Hz, 1H) 7.67 (d, J=8.14 Hz, 1H) 7.83 (s, I H) 8.59 (s, 1H).

### EXAMPLE 9

This example was prepared as described in EXAMPLE 1G-H by substituting tert-butyl 3-(aminomethyl)phenylcarbamate and 1-isocyanato-4-trifluoromethylbenzene for (4-aminophenyl)carbamic acid tert-butyl ester and 1-fluoro-3-isocyanatobenzene, respectively, in EXAMPLE 1G. ¹H NMR (300 MHz, DMSO-d₆) δ 2.61 (s, 3H) 4.33 (d, J=5.95 Hz, 2H) 6.80 (t, J=5.75 Hz, 1H) 7.07 (d, J=7.54 Hz, 1H) 7.19 (d, J=4.76 Hz, 1H) 7.31 (t, J=7.73 Hz, 1H) 7.45-7.74 (m, 6H) 7.85 (s, 1H) 9.02 (s, 1H) 10.26 (s, 1H).

### EXAMPLE 10A

A solution of 2-(3-aminophenyl)ethanol (0.6 g) and 1-isocyanato-4-nitrobenzene (0.82 g) in dichloromethane (20 mL) was stirred at ambient temperature for 1 hourour. The resulting suspension was filtered and the solid collected was dried.

### EXAMPLE 10B

A mixture of EXAMPLE 10A (1 g) and 5% Pd/carbon (100 mg) was stirred under hydrogen for 10 hours and filtered. The filtrate was diluted with ethyl acetate and washed with water. A white precipitate that formed in the aqueous layer which was filtered and dried. Additional product was obtained after drying (MgSO₄), filtering and concentrating.

### EXAMPLE 10C

This example was prepared by substituting EXAMPLE 10B for EXAMPLE 1G in EXAMPLE 1H. ¹H NMR (300 MHz, DMSO-d₆) δ 2.62 (s, 3H), 2.69 (t, J=7.12 Hz, 2H), 3.49-3.68 (m, 2H), 4.63 (t, J=5.26 Hz, 1H), 6.82 (d, J=7.46 Hz, 1H), 7.07-7.22 (m, 2H) , 7.24-7.33 (m, 2H), 7.42 (d, J=9.15 Hz, 2H), 7.52 (d, J=5.09 Hz, 1H), 7.73 (d, J=8.81 Hz, 2H), 8.59 (s, 1H), 8.62 (s, 1H), 10.20 (s, 1H).

### EXAMPLE 11

This example was prepared as described in EXAMPLE 10 by substituting 2-(4-aminophenyl)ethanol for 2-(3-aminophenyl)ethanol. ¹H NMR (400 MHz, DMSO-d₆) δ 2.62 (s, 3H), 2.66 (t, J=6.90 Hz, 2H) , 3.49-3.78 (m, 2H) , 4.58 (t, J=4.91 Hz, 1H), 6.89-7.62 (m, 9H) 7.73 (d, J=8.59 Hz, 2H) 8.52 (s, 1H) 8.57 (s, 1H) 9.29 (s, 1H) 10.17 (s, 1H).

### EXAMPLE 12A

A mixture of 3-iodo-1H-pyrrole-2-carbaldehyde (2.6 g) and NH2OH.HCl (0.9 g) in pyridine (915 mL) was stirred overnight at ambient temperature, treated with acetic anhydride (1.24 mL) and heated to 90°C for 6 hours. The reaction mixture was concentrated, the concentrate was partitioned between ethyl acetate and water and the organic extract was washed with brine, dried (MgSO₄), filtered and concentrated. The concentrate was purified by flash chromatography on silica gel with 0-20% ethyl acetate/hexanes.

### EXAMPLE 12B

A solution of EXAMPLE 12A (0.97 g) in DMF (60 mL) was treated with NaH (214 mg, 60% oil dispersion), stirred at ambient temperature for 5 min, treated with O-(diphenylphosphoryl)hydroxylamine (2.13 g) and stirred an additional 2 hours. The reaction was quenched with pH 7.2 phosphate buffer and extracted with ethyl acetate. The organic extract was washed with brine, dried (MgSO₄), filtered and concentrated. The concentrate was purified by flash chromatography on silica gel with ethyl acetate/ hexanes.

### EXAMPLE 12C

A mixture of EXAMPLE 12B (0.7 g), triethylorthoformate (10 mL) and (NH₄)₂SO₄ (40 mg) was refluxed for 3 hours, cooled to ambient temperature treated with 7N ammonia in methanol (30 mL) and stirred at ambient temperature overnight. The reaction mixture was concentrated, and the concentrate was triturated with water and filtered.

### EXAMPLE 12D

This example was prepared as described in EXAMPLES 1E-F by substituting EXAMPLE 12C for EXAMPLE 1D.

### EXAMPLE 12E

This example was prepared by substituting EXAMPLE 12D for EXAMPLE 1F in EXAMPLE 1H. ¹H NMR (300 MHz, DMSO-d₆) δ 6.68-6.84 (m, 1H), 7.06-7.16 (m, 1H), 7.22-7.37 (m, 1H), 7.40-7.66 (m, 6H), 7.75 (d, J = 3.05 Hz, 1H), 7.93 (s, 1H), 8.16 (s, 1H), 8.72 (s, 1H), 8.87 (s, 1H), 9.97 (s, 1H), 10.08 (s, 1H).

### EXAMPLE 13

This example was prepared as described in EXAMPLES 1G-1H by substituting 1-fluoro-4-isocyanatobenzene for 1-fluoro-3-isocyanatobenzene in EXAMPLE 1G and EXAMPLE 12D for 1F in EXAMPLE 1H. ¹H NMR (300 MHz, DMSO-d₆) δ 7.12 (t, J=8.99 Hz, 2H) 7.39-7.52 (m, 4H) 7.53-7.66 (m, 3H) 7.75 (d, J=3.05 Hz, 1H) 7.93 (s, 1H) 8.17 (s, 1H) 8.65 (s, 1H) 8.67 (s, 1H) 9.97 (s, 1H) 10.08 (s, 1H)

### EXAMPLE 14

This example was prepared as described in EXAMPLES 1G-1H by substituting 1-fluoro-2-isocyanatobenzene for 1-fluoro-3-isocyanatobenzene in EXAMPLE 1G and EXAMPLE 12D for 1F in EXAMPLE 1H. ¹H NMR (300 MHz, DMSO-d₆) δ 6.93-7.06 (m, 1H) 7.08-7.30 (m, 2H) 7.45 (d, J=9.16 Hz, 2H) 7.53-7.68 (m, 3H) 7.75 (d, J=3.05 Hz, 1H) 7.94 (s, 1H) 8.09-8.26 (m, 2H) 8.52 (d, J=2.37 Hz, 1H) 9.07 (s, 1H) 9.98 (s, 1H) 10.09 (s, 1H).

### EXAMPLE 15

This example was prepared as described in EXAMPLES 1G-1H by substituting isocyanatobenzene for 1-fluoro-3-isocyanatobenzene in EXAMPLE 1G and EXAMPLE 12D for 1F in EXAMPLE 1H. ¹H NMR (300 MHz, DMSO-d₆) δ 6.97 (t, J=7.29 Hz, 1H), 7.28 (t, J=7.97 Hz, 2H), 7.39-7.50 (m, 4H), 7.53-7.64 (m, 3H) , 7.75 (d, J=3.05 Hz, 1H), 7.93 (s, 1H), 8.17 (s, 1H), 8.65 (d, J = 6.10 Hz, 2H), 9.99 (s, 1H), 10.08 (s, 1H).

### EXAMPLE 16

This example was prepared as described in EXAMPLES 1G-1H by substituting 1-isocyanato-3,5-dimethylbenzene for 1-fluoro-3-isocyanatobenzene in EXAMPLE 1G and EXAMPLE 12D for 1F in EXAMPLE 1H. ¹H NMR (300 MHz, DMSO-d₆) δ 2.23 (s, 6H) 6.61 (s, I H) 7.07 (s, 2H) 7.44 (d, J=8.81 Hz, 2H) 7.52-7.66 (m, 3H) 7.75 (d, J=3.05 Hz, 1H) 7.93 (s, 1H) 8.17 (s, 1H) 8.48 (s, 1H) 8.62 (s, 1H) 9.99 (s, 1H) 10.07 (s, 1H).

### EXAMPLE 17

This example was prepared as described in EXAMPLES 1G-1H by substituting 1-isocyanato-3-methylbenzene for 1-fluoro-3-isocyanatobenzene in EXAMPLE 1G and EXAMPLE 12D for 1F in EXAMPLE 1H. ¹H NMR (300 MHz, DMSO-d₆) δ 2.28 (s, 3H) 6.79 (d, J=7.12 Hz, 1H) 7.08-7.27 (m, 2H) 7.30 (s, 1H) 7.45 (d, J=8.82 Hz, 2H) 7.52-7.68 (m, 3H) 7.75 (d, J=3.05 Hz, 1H) 7.93 (s, 1H) 8.17 (s, 1H) 8.56 (s, 1H) 8.64 (s, 1H) 9.84-10.05 (m, 1H) 10.08 (s, 1H).

### EXAMPLE 18

This example was prepared as described in EXAMPLES 1G-1H by substituting 1-isocyanato-4-methylbenzene for 1-fluoro-3-isocyanatobenzene in EXAMPLE 1G and EXAMPLE 12D for 1F in EXAMPLE 1H. ¹H NMR (300 MHz, DMSO-d₆) δ 2.24 (s, 3H) 7.08 (d, J=8.14 Hz, 2H) 7.34 (d, J=8.48 Hz, 2H) 7.44 (d, J=9.16 Hz, 2H) 7.52-7.64 (m, 3H) 7.74 (d, J=3.05 Hz, 1H) 7.93 (s, 1H) 8.16 (s, I H) 8.52 (s, 1H) 8.60 (s, 1H) 9.99 (s, 1H) 10.07 (s, I H)

### EXAMPLE 19

This example was prepared as described in EXAMPLES 1G-1H by substituting 1-isocyanato-2-methylbenzene for 1-fluoro-3-isocyanatobenzene in EXAMPLE 1G and EXAMPLE 12D for 1F in EXAMPLE 1H. ¹H NMR (300 MHz, DMSO-d₆) δ 2.25 (s, 3H) 6.86-6.98 (m, 1H) 7.07-7.22 (m, 2H) 7.42-7.52 (m, 2H) 7.54-7.65 (m, 3H) 7.79 (d, J=3.39 Hz, 1H) 7.85 (d, J=7.12 Hz, 1H) 7.93 (s, 1H) 7.97 (s, 1H) 8.33 (s, 1H) 9.07 (s, 1H) 10.12 (s, 1H) 10.17 (s, 1H)

### EXAMPLE 20

This example was prepared as described in EXAMPLES 1G-1H by substituting 1-isocyanato-3-methoxybenzene for 1-fluoro-3-isocyanatobenzene in EXAMPLE 1G and EXAMPLE 12D for 1F in EXAMPLE 1H. ¹H NMR (300 MHz, DMSO-d₆) δ 3.74 (s, 3H) 6.55 (dd, J=7.97, 2.20 Hz, 1H) 6.83-7.01 (m, 1H) 7.09-7.27 (m, 2H) 7.35-7.50 (m, 2H) 7.52-7.67 (m, 3H) 7.75 (d, J=3.05 Hz, 1H) 7.93 (s, 1H) 8.17 (s, 1H) 8.65 (d, J=2.03 Hz, 2H) 9.99 (s, 1H) 10.08 (s, 1H)

### EXAMPLE 21

This example was prepared as described in EXAMPLES 1G-1H by substituting 1-isocyanato-4-methoxybenzene for 1-fluoro-3-isocyanatobenzene in EXAMPLE 1G and EXAMPLE 12D for 1F in EXAMPLE 1H. ¹H NMR (300 MHz, DMSO-d₆) δ 3.72 (s, 3H) 6.76-6.95 (m, 2H) 7.28-7.49 (m, 4H) 7.52-7.66 (m, 3H) 7.75 (d, J=3.05 Hz, 1H) 7.93 (s, 1H) 8.17 (s, 1H) 8.44 (s, 1H) 8.57 (s, 1H) 9.99 (s, 1H) 10.07 (s, 1H)

### EXAMPLE 22

This example was prepared as described in EXAMPLES 1G-1H by substituting 1-isocyanato-3-trifluoromethylbenzene for 1-fluoro-3-isocyanatobenzene in EXAMPLE 1G and EXAMPLE 12D for 1F in EXAMPLE 1H. ¹H NMR (500 MHz, DMSO-d₆) δ 7.31 (d, J=7.63 Hz, 1H) 7.43-7.68 (m, 7H) 7.75 (d, J=2.75 Hz, I H) 7.94 (s, 1H) 8.04 (s, 1H) 8.19 (s, 1H) 8.82 (s, 1H) 9.06 (s, 1H) 10.00 (s, 1H) 10.11 (s, 1H).

### EXAMPLE 23

This example was prepared as described in EXAMPLES 1G-1H by substituting isocyanatocyclopropane for 1-fluoro-3-isocyanatobenzene in EXAMPLE 1G and EXAMPLE 12D for 1F in EXAMPLE 1H. ¹H NMR (300 MHz, DMSO-d₆) δ 0.33-0.45 (m, 2H) 0.56-0.68 (m, 2H) 2.52-2.61 (m, 1H) 6.35 (d, J=2.71 Hz, I H) 7.39 (d, J=8.82 Hz, 2H) 7.49-7.60 (m, 3H) 7.74 (d, J=3.05 Hz, 1H) 7.93 (s, 1H) 8.17 (d, J=3.39 Hz, 1H) 8.27 (s, 1H) 10.03 (s, 2H).

### EXAMPLE 24

This example was prepared as described in EXAMPLES 1G-1H by substituting isocyanatocyclopentane for 1-fluoro-3-isocyanatobenzene in EXAMPLE 1G and EXAMPLE 12D for 1F in EXAMPLE 1H. ¹H NMR (300 MHz, DMSO-d₆) δ 1.21-1.45 (m, 2H) 1.45-1.70 (m, 4H) 1.73-1.97 (m, 2H) 3.71-4.11 (m, 1H) 6.11 (d, J=7.12 Hz, 1H) 7.36 (d, J=8.82 Hz, 2H) 7.44-7.62 (m, 3H) 7.73 (d, J=3.05 Hz, 1H) 7.93 (s, 1H) 8.15 (s, 1H) 8.23 (s, I H) 10.02 (s, 2H).

### EXAMPLE 25

This example was prepared as described in EXAMPLES 1G-1H by substituting 3-isocyanatothiophene for 1-fluoro-3-isocyanatobenzene in EXAMPLE 1G and EXAMPLE 12D for 1F in EXAMPLE 1H. ¹H NMR (300 MHz, DMSO-d₆) δ 7.05 (dd, J=5.09, 1.36 Hz, 1H) 7.21-7.33 (m, 1H) 7.37-7.48 (m, 3H) 7.51-7.66 (m, 3H) 7.75 (d, J=3.05 Hz, 1H) 7.93 (s, 1H) 8.17 (s, 1H) 8.62 (s, 1H) 8.90 (s, 1H) 10.00 (s, 1H) 10.07 (s, 1H).

### EXAMPLE 26

This example was prepared as described in EXAMPLES 1G-1H by substituting 2-isocyanatopyridine for 1-fluoro-3-isocyanatobenzene in EXAMPLE 1G and EXAMPLE 12D for 1F in EXAMPLE 1H. ¹H NMR (300 MHz, DMSO-d₆) δ 7.31 (dd, J=8.14, 4.75 Hz, 1H) 7.41-7.52 (m, 2H) 7.54-7.67 (m, 3H) 7.75 (d, J=3.05 Hz, 1H) 7.87-8.00 (m, 2H) 8.19 (dd, J=4.75, 1.36 Hz, 2H) 8.61 (d, J=2.71 Hz, 1H) 8.81 (d, J=5.76 Hz, 2H) 9.98 (s, 1H) 10.09 (s, 1H).

### EXAMPLE 27

This example was prepared as described in EXAMPLE I H by substituting N-(4-aminophenyl)benzamide and 12D, for 1G and 1F respectively. ¹H NMR (300 MHz, DMSO-d₆) δ 7.46-7.64 (m, 4H) 7.64-7.84 (m, 5H) 7.89-8.01 (m, 3H) 8.13-8.19 (m, 1H) 9.97 (s, 1H) 10.14 (s, 1H) 10.26 (s, 1H).

### EXAMPLE 28

This example was prepared as described in EXAMPLES 1G-1H by substituting 1-fluoro-2-isocyanato-4-methylbenzene for 1-fluoro-3-isocyanatobenzene in EXAMPLE 1G and EXAMPLE 12D for 1F in EXAMPLE 1Hz, NMR (300 MHz, DMSO-d₆) δ 2.28 (s, 3H) 6.64-6.85 (m, 1H) 7.10 (dd, J=11.53, 8.48 Hz, 1H) 7.46 (d, J=9.16 Hz, 2H) 7.54-7.70 (m, 3H) 7.76 (d, J=2.71 Hz, 1H) 7.90-8.07 (m, 2H) 8.24 (s, 1H) 8.47 (d, J=2.03 Hz, 1H) 9.10 (s, 1H) 10.06 (s, 1H) 10.11 (s, 1H).

### EXAMPLE 29

This example was prepared as described in EXAMPLE 1H by substituting 1-(4-aminophenyl)-3-(4-(2-hydroxyethyl)phenyl)urea and 12D, for I G and 1F respectively. ¹H NMR (300 MHz, DMSO-d₆) δ 2.66 (t, J=7.12 Hz, 2H), 3.57 (t, J=7.12 Hz, 2H), 7.12 (d, J=8.48 Hz, 2H), 7.35 (d, J=8.14 Hz, 2H), 7.45 (d, J=9.16 Hz, 2H), 7.55-7.64 (m, 3H), 7.80 (d, J=3.05 Hz, 1H), 7.99 (s, 1H), 8.39 (s, 1H), 8.61 (s, 1H), 8.69 (s, 1H), 10.14 (s, 1H), 10.24 (s, 1H).

### EXAMPLE 30

This example was prepared as described in EXAMPLE I H by substituting EXAMPLE10B and 12D, for 1G and 1F respectively. ¹H NMR (300 MHz, DMSO-d₆) δ 2.69 (s, 2H) 3.59 (s, 2H) 4.63 (s, 1H) 6.83 (s, 1H) 7.06-8.01 (m, 10H) 8.17 (s, 1H) 8.57 (s, 1H) 8.63 (s, 1H) 9.97 (s, 1H) 10.08 (s, 1H)

### EXAMPLE 31

This example was prepared as described in EXAMPLE 1H by substituting 1-(4-aminophenyl)-3-(3-(hydroxymethyl)phenyl)urea and 12D for EXAMPLES I G and 1 F, respectively. ¹H NMR (300 MHz, DMSO-d₆) δ 4.47 (s, 2H), 6.91 (d, J=7.46 Hz, 1H), 7.13-7.27 (m, 1H), 7.28-7.36 (m, 1H), 7.39-7.51 (m, 3H), 7.55-7.66 (m, 3H), 7.78 (d, J=3.05 Hz, 1H), 7.97 (s, 1H), 8.32 (s, 1H), 8.68 (d, J=2.03 Hz, 2H), 10.12 (s, 1H) 10.16 (s, 1H).

### EXAMPLE 32A

A solution of 1-(3-hydroxyphenyl)-3-(4-nitrophenyl)urea (1.37 g), Cs2CO3 (3.25 g), 3-bromopropan-1-ol (1.4 mL) in ethanol (30 mL) was refluxed for 30 hours, cooled to ambient temperature and partitioned between ethyl acetate and water. The organic extract was washed with 1N NaOH, brine, dried (MgSO₄), filtered and concentrated. A mixture of the concentrate and iron (2 g), NH₄Cl (0.29 g) in ethanol (10 mL)/water (10 mL) was refluxed for 24 hours, treated with 5 drops of 3N HCl and stirred at reflux for 3 hours. The mixture was cooled and filtered through diatomaceous earth (Celite®). The filtrate was extracted with ethyl acetate, and the extract was concentrated. The concentrate was triturated with boiling diethyl ether and filtered.

### EXAMPLE 32B

This example was prepared as described in EXAMPLE 1H by substituting 32A and 12D, for 1G and 1F respectively. ¹H NMR (300 MHz, DMSO-d₆) δ 1.77-1.94 (m, 2H) 3.50-3.62 (m, 2H) 4.01 (t, J=6.44 Hz, 2H) 4.53 (t, J=5.09 Hz, 1H) 6.54 (dd, J=8.14, 1.70 Hz, 1H) 6.89 (d, J=9.16 Hz, 1H) 7.16 (t, J=8.14 Hz, 1H) 7.22 (t, J=2.03 Hz, 1H) 7.44 (d, J=8.82 Hz, 2H) 7.56 (d, J=3.39 Hz, 1H) 7.61 (d, J=8.82 Hz, 2H) 7.74 (d, J=3.05 Hz, 1H) 7.93 (s, 1H) 8.16 (s, 1H) 8.63 (d, J=4.41 Hz, 2H) 9.99 (s, 1H) 10.07 (s, 1H).

### EXAMPLE 33

This example was prepared as described in EXAMPLE 1H by substituting 1-(3-aminophenyl)-3-phenylurea and 12D, for 1G and 1F respectively. H NMR (300 MHz, DMSO-d₆) δ 6.97 (t, J=7.29 Hz, 1H) 7.19-7.36 (m, J=14.24, 6.78 Hz, 5H) 7.46 (d, J=7.80 Hz, 2H) 7.62 (d, J=3.05 Hz, 1H) 7.76 (d, J=3.05 Hz, 1H) 7.95 (s, 2H) 8.23 (s, 1H) 8.62 (s, 1H) 8.77 (s, 1H) 9.95 (s, 1H) 10.16 (s, 1H).

### EXAMPLE 34

This example was prepared as described in EXAMPLE 1H by substituting 1-(3-aminophenyl)-3-m-tolylurea and 12D, for 1G and 1F respectively. ¹H NMR (300 MHz, DMSO-d₆) δ 2.28 (s, 3H) 6.79 (d, J=7.46 Hz, 1H) 7.07-7.37 (m, 6H) 7.61 (d, J=3.05 Hz, 1H) 7.76 (d, J=3.05 Hz, 1H) 7.87-8.01 (m, 2H) 8.20 (s, I H) 8.53 (s, 1H) 8.74 (s, 1H) 9.94 (s, 1H) 10.13 (s, 1H).

### EXAMPLE 35

This example was prepared as described in EXAMPLE 1H by substituting 2-(2-aminothiazol-5-yl)-N-(3-fluorophenyl)acetamide and 12D, for I G and 1F respectively. ¹H NMR (500 MHz, DMSO-d₆) δ 3.90 (s, 2H) 6.80-6.98 (m, 1H) 7.23-7.47 (m, 3H) 7.61 (d, J=11.60 Hz, 1H) 7.78 (s, 2H) 7.99 (s, 1H) 8.33 (s, 1H) 9.66 (s, 1H) 10.46 (s, 1H) 12.43 (s, 1H).

### EXAMPLE 36A

A solution of 3-(morpholinomethyl)aniline (0.46 g), TEA (0.37 mL) and 4-nitrophenyl carbonochloridate (530 mg) in THF (18 mL) was stirred at ambient temperature for 2 hours, treated with tert-butyl 4-aminophenylcarbamate (500mg) and an additional 0.37mL of TEA. the resulting mixture was stirred at ambient temperature for 48 hours, poured into water and exatrcted 3x with ethyl acetate. The extract was washed with brine, dried (MgSO₄), filtered and concentrated. The concentrate was purified by flash chromatography on silica gel with 2% methanol/dichloromethane to provide 1-(3-(morpholinomethyl)phenyl)-3-(4-nitrophenyl)urea which was dissolved in dichloromethane (30 mL), cooled in an ice bath, and treated with TFA (1.8 mL). The reaction mixture was stirred for 30 minutes at 0°C and for 12 hours at ambient temperature, and concentrated three times from methanol/toluene.

### EXAMPLE 36B

This example was prepared as described in EXAMPLE I H by substituting 36A and 12D, for 1G and 1F respectively. ¹H NMR (300 MHz, DMSO-d₆) δ 2.31-2.40 (m, 4H) 3.43 (s, 2H) 3.52-3.62 (m, 4H) 6.90 (d, J=7.46 Hz, I H) 7.22 (t, J=7.63 Hz, 1H) 7.34 (d, J=9.15 Hz, 1H) 7.41-7.50 (m, 3H) 7.53-7.64 (m, 3H) 7.75 (d, J=3.05 Hz, 1H) 7.93 (s, 1H) 8.17 (s, 1H) 8.62 (s, 1H) 8.66 (s, 1H) 10.00 (s, 1H) 10.08 (s, 1H).

### EXAMPLE 37A

The title compound was prepared by first substituting tert-butyl 4-aminobenzylcarbamate and 12D for EXAMPLES 1 G and 1F respectively, in EXAMPLE 1H, removing the Nboc protecting group as described in EXAMPLE 1G.

### EXAMPLE 37B

A mixture of EXAMPLE 37A (0.1 mmol) and TEA (0.2 mmol) in dichloromethane at -20°C (3 mL) was treated with 1-isocyanato-3-methylbenzene (0.1 mmol), stirred at ambient temperature for 1 hour, and filtered. ¹H NMR (300 MHz, DMSO-d₆) δ 2.24 (s, 3H) 4.23-4.39 (m, 2H) 6.56 (t, J=5.59 Hz, 1H) 6.71 (d, J=6.78 Hz, 1H) 7.01-7.35 (m, 5H) 7.53-7.82 (m, 4H) 7.94 (s, 1H) 8.19 (s, 1H) 8.45 (s, 1H) 9.93 (s, 1H) 10.13 (s, 1H).

### EXAMPLE 38

This example was prepared as described in EXAMPLE 37B by substituting 1-fluoro-3-isocyanatobenzene for 1-isocyanato-3-methylbenzene. ¹H NMR (300 MHz, DMSO-d₆) δ 4.29 (d, J=5.43 Hz, 2H) 6.43-6.83 (m, 2H) 7.05 (d, J=7.80 Hz, I H) 7.15-7.36 (m, 3H) 7.41-7.81 (m, 5H) 7.94 (s, I H) 8.19 (s, 1H) 8.80 (s, I H) 9.93 (s, 1H) 10.13 (s, 1H).

### EXAMPLE 39

This example was prepared as described in EXAMPLE 37 by substituting tert-butyl 3-aminobenzylcarbamate for tert-butyl 4-aminobenzylcarbamate in 37A and 1-fluoro-3-isocyanatobenzene for 1-isocyanato-3-methylbenzene in EXAMPLE 37B. 4¹H NMR (400 MHz, DMSO-d₆) δ 4.32 (d, J=5.22 Hz, 2H) 6.62-6.81 (m, 2H) 7.01-7.13 (m, 2H) 7.18-7.37 (m, 2H) 7.48 (d, J=11.97 Hz, 1H) 7.55-7.71 (m, 3H) 7.75 (s, 1H) 7.95 (s, 1H) 8.22 (s, 1H) 8.85 (s, 1H) 9.92 (s, 1H) 10.17 (s, 1H).

### EXAMPLE 40

This example was prepared as described in EXAMPLE 37 by substituting tert-butyl 3-aminobenzylcarbamate for tert-butyl 4-aminobenzylcarbamate in 37A and isocyanatobenzene for 1-isocyanato-3-methylbenzene in EXAMPLE 37B. ¹H NMR (500 MHz, DMSO-d₆) δ 4.32 (d, J=5.80 Hz, 2H) 6.63 (t, J=5.65 Hz, 1H) 6.89 (t, J=7.17 Hz, 1H) 7.08 (d, J=7.32 Hz, 1H) 7.22 (t, J=7.63 Hz, 2H) 7.26-7.47 (m, 3H) 7.53-7.78 (m, 4H) 7.94 (s, 1H) 8.19 (s, 1H) 8.56 (s, 1H) 9.92 (s, 1H) 10.16 (s, 1H).

### EXAMPLE 54

### 8-amino-3-cyclopropyl-N-(4-((((3-fluorophenyl)amino)carbonyl)amino)phenyl)imidazo[1,5-a]pyrazine-1-carboxamide

### EXAMPLE 54A

### 8-amino-3-cyclopropylimidazo[1,5-a]pyrazine-1-carboxylic acid

The title compound was prepared as described in EXAMPLES 1A-1F, by substituting cyclopropanecarboxylic acid for acetic acid in 1A.

### EXAMPLE 54B

### 8-amino-3-cyclopropyl-N-(4-((((3-fluorophenyl)amino)carbonyl)amino)phenyl)imidazo[1,5-a]pyrazine-1-carboxamide

The title compound was prepared as described in EXAMPLES 1 H, except substituting EXAMPLE 54A for EXAMPLE 1F. ¹H NMR (300 MHz, DMSO-d₆) δ ppm 0.87 - 1.28 (m, 4 H) 2.19 - 2.45 (m, 1 H) 6.60 - 6.91 (m, I H) 7.04 - 7.59 (m, 7 H) 7.58 - 7.90 (m, 3 H) 8.74 (s, I H) 8.90 (s, I H) 9.21 (s, 1 H) 9.96 (s, 1 H). MS(ESI(+)) m/e 446(M+H)^{+.}

### EXAMPLE 55

### 8-amino-N-(4-((anilinocarbonyl)amino)phenyl)-3-cyclopropylimidazo[1,5-a]pyrazine-1-carboxamide

The title compound was prepared as described in EXAMPLES 1G-1H by substituting isocyanatobenzene for 1-fluoro-3-isocyanatobenzene in EXAMPLE 1G and EXAMPLE 54A for EXAMPLE IF in EXAMPLE 1H.¹H NMR (300 MHz, DMSO-d₆) δ ppm 0.87 - 1.32 (m, 4 H) 2.15 - 2.42 (m, 1 H) 6.96 (t, *J*=7.29 Hz, 1 H) 7.11 - 7.59 (m, 8 H) 7.59 - 7.86 (m, 3 H) 8.64 (d, *J*=2.03 Hz, 2 H) 9.17 (s, 1 H) 9.94 (s, 1 H). MS(ESI(+)) m/e 428(M+H)^{+.}

### EXAMPLE 56

### 8-amino-3-cyclopropyl-N-(4-((((3-methylphenyl)amino)carbonyl)amino)phenyl)imidazo[1,5-a]pyrazine-1-carboxamide

The title compound was prepared as described in EXAMPLES 1G-1H by substituting 1-isocyanato-3-methylbenzene for 1-fluoro-3-isocyanatobenzene in EXAMPLE 1G and EXAMPLE 54A for EXAMPLE IF in EXAMPLE 1H. ¹H NMR (300 MHz, DMSO-d₆) δ ppm 0.85 - 1.41 (m, 4 H) 2.28 (s, 3 H) 2.29 - 2.45 (m, 1 H) 6.78 (d,J=7.14 Hz, 1 H) 7.00 - 7.54 (m, 7 H) 7.59 - 7.85 (m, 3 H) 8.57 (s, 1 H) 8.64 (s, 1 H) 9.15 (s, I H) 9.94 (s, 1 H). MS(ESI(+)) m/e 442(M+H)^{+.}

### EXAMPLE 57

### 8-amino-3-cyclopropyl-N-(4-((((2-fluoro-5-(trifluoromethyl)phenyl)amino)carbonyl)amino)phenyl)imidazo[1,5-a]pyrazine-1-carboxamide

The title compound was prepared as described in EXAMPLES 1G-1H by substituting 1-fluoro-2-isocyanato-4-(trifluoromethyl)benzene for 1-fluoro-3-isocyanatobenzene in EXAMPLE 1G and EXAMPLE 54A for EXAMPLE IF in EXAMPLE 1H. ¹H NMR (300 MHz, DMSO-d₆) δ ppm 0.84 - 1.29 (m, 4 H) 2.16 - 2.45 (m, 1 H) 7.20 (d, *J*=3.73 Hz, I H) 7.30 - 7.64 (m, 5 H) 7.66 - 8.03 (m, 3 H) 8.63 (dd, *J*=7.29, 2.20 Hz, 1 H) 8.87 (d, *J*=2.71 Hz, 1 H) 9.16 (s, 2 H) 9.98 (s, 1 H). MS(ESI(+)) m/e 514(M+H)^{+.}

### EXAMPLE 58

### 8-amino-3-cyclopropyl-N-(4-((((4-(trifluoromethyl)phenyl)amino)carbonyl)amino)phenyl)imidazo[1,5-a]pyrazine-1-carboxamide

The title compound was prepared as described in EXAMPLES 1G-1H by substituting 1-isocyanato-4-(trifluoromethyl)benzene for 1-fluoro-3-isocyanatobenzene in EXAMPLE I G and EXAMPLE 54A for EXAMPLE IF in EXAMPLE 1H.¹H NMR (300 MHz, DMSO-d₆) δ ppm 1.01 - 1.33 (m, 4 H) 2.46 - 2.62 (m, 1 H) 7.22 (d, *J*=5.52 Hz, 1 H) 7.50 (d, *J*=8.90 Hz, 2 H) 7.56 - 7.81 (m, 6 H) 7.99 (d, *J*=5.83 Hz, 1 H) 8.99 (s, I H) 9.24 (s, 2 H) 10.33 (s, 1 H) 10.92 (s, 1 H). MS(ESI(+)) m/e 496(M+H)^{+.}

### EXAMPLE 59

### 8-amino-3-cyclopropyl-N-(3-((((pyridin-3-ylamino)carbonyl)amino)methyl)phenyl)imidazo[1,5-a]pyrazine-1-carboxamide

The title compound was prepared as described in EXAMPLES 1G-1H by substituting 3-isocyanatopyridine for 1-fluoro-3-isocyanatobenzene in EXAMPLE 1G and EXAMPLE 54A for EXAMPLE IF in EXAMPLE 1H. ¹H NMR (400 MHz, DMSO-d₆) δ ppm 1.16 (s, 4 H) 2.22 - 2.43 (m, 1 H) 6.93 - 8.01 (m, 9 H) 8.20 (s, 1 H) 8.64 (s, 1 H) 9.01 (d, *J*=19.03 Hz, 2 H) 10.14 (s, 2 H). MS(ESI(+)) m/e 429(M+H)⁺.

### EXAMPLE 60

### 8-amino-3-cyclopropyl-N-(4-((((3-fluorophenyl)amino)carbonyl)amino)-3-methylphenyl)imidazo[1,5-a]pyrazine-1-carboxamide

The title compound was prepared as described in EXAMPLES 1G-1H by substituting tert-butyl 4-amino-3-methylphenylcarbamate for (4-aminophenyl)carbamic acid tert-butyl ester in EXAMPLE 1G and EXAMPLE 54A for EXAMPLE 1F in EXAMPLE 1H. ¹H NMR (300 MHz, DMSO-d₆) δ ppm 1.01 - 1.15 (m, 4 H) 2.26 (s, 3 H) 2.29 - 2.41 (m, 1 H) 6.63 - 6.85 (m, 1 H) 7.10 (d, *J*=7.54 Hz, 1 H) 7.20 (d, *J*=4.76 Hz, 1 H) 7.24 - 7.36 (m, 1 H) 7.46 - 7.62 (m, 2 H) 7.62 - 7.76 (m, 3 H) 7.98 (s, 1 H) 9.17 (s, 1 H) 9.90 (s, 1 H). MS(ESI(+)) m/e 460(M+H)^{+.}

### EXAMPLE 61

### 8-amino-3-cyclopropyl-N-(4-((((2-fluorophenyl)amino)carbonyl)amino)-3-methylphenyl)imidazo[1,5-a]pyrazine-1-carboxamide

The title compound was prepared as described in EXAMPLES 1G-1H by substituting 1-fluoro-2-isocyanatobenzene and tert-butyl 4-amino-3-methylphenylcarbamate for 1-fluoro-3-isocyanatobenzene and (4-aminophenyl)carbamic acid tert-butyl ester, respectively in EXAMPLE 1G and EXAMPLE 54A for EXAMPLE 1 F in EXAMPLE 1H. ¹H NMR (300 MHz, DMSO-d₆) δ pm 0.98 - 1.30 (m, 4 H) 2.10 - 2.37 (m, 4 H) 6.88 - 7.34 (m, 4 H) 7.52 - 7.70 (m, 2 H) 7.75 - 7.90 (m, 1 H) 7.93 - 8.06 (m, 1 H) 8.11 - 8.27 (m, 1 H) 8.28 - 8.49 (m, 1 H) 8.71 - 9.26 (m, 2 H) 10.24 (s, I H) 10.77 (s, 1 H). MS(ESI(+)) m/e 460(M+H)^{+.}

### EXAMPLE 62

### 8-amino-3-cyclopropyl-N-(3-methyl-4-((((4-(trifluoromethyl)phenyl)amino)carbonyl)amino)phenyl)imidazo[1,5-a]pyrazine-1-carboxamide

The title compound was prepared as described in EXAMPLES 1G-1H by substituting 1-isocyanato-4-trifluoromethylbenzene and tert-butyl 4-amino-3-methylphenylcarbamate for 1-fluoro-3-isocyanatobenzene and (4-aminophenyl)carbamic acid tert-butyl ester, respectively in EXAMPLE 1G and EXAMPLE 54A for EXAMPLE IF in EXAMPLE 1H. ¹H NMR (300 MHz, DMSO-d₆) δ ppm 0.96 - 1.30 (m, 4 H) 2.04 - 2.37 (m, 4 H) 7.02 - 7.34 (m, 1 H) 7.51 - 7.83 (m, 7 H) 7.89 - 8.04 (m, I H) 8.13 (s, 1 H) 9.11 (s, 1 H) 9.44 (s, 1 H) 10.27 (s, 1 H) 10.84 (s, 1 H). MS(ESI(+)) m/e 510(M+H)⁺.

### EXAMPLE 63

### 8-amino-3-cyclopropyl-N-(3-((((3-fluorophenyl)amino)carbonyl)amino)phenyl)imidazo[1,5-a]pyrazine-1-carboxamide

The title compound was prepared as described in EXAM PLES 1G-1H by substituting (3-aminophenyl)carbamic acid tert-butyl ester for (4-aminophenyl)carbamic acid tert-butyl ester in EXAMPLE 1G and EXAMPLE 54A for EXAMPLE IF in EXAMPLE 1H. ¹H NMR (300 MHz, DMSO-d₆) δ ppm 0.80 - 1.26 (m, 4 H) 2.14 - 2.45 (m, 1 H) 6.50 - 6.87 (m, 1 H) 7.05 - 7.60 (m, 8 H) 7.74 (d, *J*=4.75 Hz, 1 H) 8.00 (s, 1 H) 8.83 (d, *J*=3.05 Hz, 2 H) 9.15 (s, 1 H) 9.96 (s, 1 H). MS(ESI(+)) m/e 446(M+H)⁺.

### EXAMPLE 64

### 8-amino-3-cyclopropyl-N-(3-(((((3-fluorophenyl)amino)carbonyl)amino)methyl)phenyl)imidazo[1,5-a]pyrazine-1-carboxamide

The title compound was prepared as described in EXAMPLE 1G-H by substituting tert-butyl 3-(aminomethyl)phenylcarbamate for (4-aminophenyl)carbamic acid tert-butyl ester in EXAMPLE I G and EXAMPLE 54A for EXAMPLE IF in EXAMPLE 1H. ¹H NMR (300 MHz, DMSO-d₆) δ ppm 0.86 - 1.28 (m, 4 H) 2.16 - 2.44 (m, 1 H) 4.31 (d, *J*=5.76 Hz, 2 H) 6.47 - 6.90 (m, 2 H) 6.94 - 7.55 (m, 7 H) 7.57 - 7.97 (m, 3 H) 8.82 (s, 1 H) 9.16 (s, 1 H) 10.01 (s, 1 H). MS(ESI(+)) m/e 460(M+H)^{+.}

### EXAMPLE 65

### 8-amino-N-(3-(((((4-chloro-2-fluorophenyl)amino)carbonyl)amino)methyl)phenyl)-3-cyclopropylimidazo[1,5-a]pyrazine-1-carboxamide

The title compound was prepared as described in EXAMPLE 1G-H by substituting tert-butyl 3-(aminomethyl)phenylcarbamate and 4-chloro-2-fluoro-1-isocyanatobenzene for (4-aminophenyl)carbamic acid tert-butyl ester and 1-fluoro-3-isocyanatobenzene, respectively in EXAMPLE 1G and EXAMPLE 54A for EXAMPLE I F in EXAMPLE 1H. ¹H NMR (300 MHz, DMSO-d₆) δ ppm 0.58 - 1.50 (m, 4 H) 2.17 - 2.43 (m, I H) 4.33 (d, *J*=5.76 Hz, 2 H) 6.89 - 7.53 (m, 7 H) 7.59 - 7.90 (m, 3 H) 8.18 (t, *J*=8.98 Hz, 1 H) 8.52 (d, *J*=2.03 Hz, 1 H) 9.12 (s, 1 H) 10.02 (s, 1 H). MS(ESI(+)) m/e 494(M+H)^{+.}

### EXAMPLE 66

### 8-amino-N-(3-(((anilinocarbonyl)amino)methyl)phenyl)-3-cyclopropylimidazo[1,5-a]pyrazine-1-carboxamide

The title compound was prepared as described in EXAMPLE 1G-H by substituting tert-butyl 3-(aminomethyl)phenylcarbamate and isocyanatobenzene for (4-aminophenyl)carbamic acid tert-butyl ester and 1-fluoro-3-isocyanatobenzene, respectively in EXAMPLE 1G and EXAMPLE 54A for EXAMPLE 1F in EXAMPLE 1H. ¹H NMR (300 MHz, DMSO-d₆) δ ppm 0.84 - 1.33 (m, 4 H) 2.14 - 2.44 (m, 1 H) 4.31 (d, *J*=5.76 Hz, 2 H) 6.62 (t, *J*=5.93 Hz, 1 H) 6.89 (t, *J*=7.46 Hz, 1 H) 7.07 (d, *J*=7.80 Hz, 1 H) 7.15 - 7.53 (m, 7 H) 7.54 - 8.00 (m, 3 H) 8.54 (s, 1 H) 9.06 (s, 1 H) 10.00 (s, 1 H). MS(ESI(+)) m/e 442(M+H)^{+.}

### EXAMPLE 67

### 8-amino-3-cyclopropyl-N-(3-(((((3,4-difluorophenyl)amino)carbonyl)amino)methyl)phenyl)imidazo[1,5-a]pyrazine-1-carboxamide

The title compound was prepared as described in EXAMPLE 1G-H by substituting tert-butyl 3-(aminomethyl)phenylcarbamate and 3,4-difluoro-1-isocyanatobenzene for (4-aminophenyl)carbamic acid tert-butyl ester and I -fluoro-3-isocyanatobenzene, respectively in EXAMPLE 1G and EXAMPLE 54A for EXAMPLE IF in EXAMPLE 1H. ¹H NMR (300 MHz, DMSO-d₆) δ ppm 0.82 - 1.41 (m, 4 H) 2.21 - 2.44 (m, 1 H) 4.31 (d, *J*=5.76 Hz, 2 H) 6.73 (t, *J*=5.93 Hz, 1 H) 6.90 - 7.13 (m, 1 H) 7.14 - 7.46 (m, 5 H) 7.50 - 7.95 (m, 4 H) 8.81 (s, 1 H) 9.20 (s, 1 H) 10.00 (s, 1 H). MS(ESI(+)) m/e 478(M+H)^{+.}

### EXAMPLE 68

### 8-amino-3-cyclopropyl-N-(3-(((((2-fluorophcnyl)amino)carbonyl)amino)methyl)phenyl)imidazo[1,5-a]pyrazine-1-carboxamide

### EXAMPLE 68A

### 8-amino-N-(3-(aminomethyl)phenyl)-3-cyclopropylimidazo[1,5-a]pyrazine-1-carboxamide

The title compound was prepared as a bis-trifluoroaceate salt by first substituting tert-butyl 3-aminobenzylcarbamate and EXAMPLE 54A for EXAMPLES 1G and EXAMPLE IF respectively, in EXAMPLE 1H, followed by removing the NBoc protecting group by treatment with TFA as described in EXAMPLE I G. MS(ESI(+)) m/e 323(M+H)^{+.}

### EXAMPLE 68B

### 8-amino-3-cyclopropyl-N-(3-(((((2-fluorophenyl)amino)carbonyl)amino)methyl)phenyl)imidazo[1,5-a]pyrazine-1-carboxamide

The title compound was prepared as described in EXAMPLE 37B by substituting 1-fluoro-2-isocyanatobenzene and EXAMPLE 68A for 1-isocyanato-3-methylbenzene and EXAMPLE 37A, respectively. ¹H NMR (300 MHz, DMSO-d₆) δ ppm 1.13 - 1.41 (m, 4 H) 2.36 - 2.63 (m, 1 H) 4.34 (d, *J*=5.52 Hz, 2 H) 6.94 (s, 1 H) 7.01 - 7.29 (m, 5 H) 7.37 (t, *J*=7.67 Hz, 1 H) 7.57 - 7.85 (m, 2 H) 7.99 (d, *J*=5.52 Hz, 1 H) 8.06 - 8.28 (m, 1 H) 8.39 (d, *J*=1.84 Hz, 1 H) 9.26 (s, 1 H) 10.39 (s, I H) 10.83 (s, 1 H)
MS(ESI(+)) m/e 460(M+H)^{+.}

### EXAMPLE 69

### 8-amino-3-cyclopropyl-N-(3-(((((4-fluorophenyl)amino)carbonyl)amino)methyl)phenyl)imidazo[1,5-a]pyrazine-1-carboxamide

The title compound was prepared as described in EXAMPLE 37B by substituting 1-fluoro-4-isocyanatobenzene and EXAMPLE 68A for 1-isocyanato-3-methylbenzene and EXAMPLE 37A, respectively. ¹H NMR (300 MHz, DMSO-d₆) δ ppm 1.07 - 1.36 (m, 4 H) 2.38 - 2.60 (m, 1 H) 4.32 (d, *J*=5.83 Hz, 2 H) 6.73 (t, *J*=5.68 Hz, 1 H) 6.94 - 7.56 (m, 7 H) 7.58 - 7.89 (m, 2 H) 7.99 (d, *J*=5.83 Hz, 1 H) 8.68 (s, 1 H) 9.33 (s, 1 H) 10.37 (s, 1 H) 10.82 (s, 1 H). MS(ESI(+)) m/e 460(M+H)^{+.}

### EXAMPLE 70

### 8-amino-N-(3-(((((3-chlorophenyl)amino)carbonyl)amino)methyl)phenyl)-3-cyclopropylimidazo[1,5-a]pyrazine-1-carboxamide

The title compound was prepared as described in EXAMPLE 37B by substituting 1-chloro-3-isocyanatobenzene and EXAMPLE 68A for 1-isocyanato-3-methylbenzene and EXAMPLE 37A, respectively. ¹H NMR (300 MHz, DMSO-d₆) δ ppm 1.13 - 1.33 (m, 4 H) 2.31 - 2.49 (m, 1 H) 4.32 (d, *J*=5.83 Hz, 2 H) 6.82 (t, *J*=6.14 Hz, 1 H) 6.89 - 7.01 (m, 1 H) 7.06 - 7.27 (m, 4 H) 7.36 (t, *J*=7.83 Hz, 1 H) 7.52 - 7.86 (m, 3 H) 7.97 (d, *J*=5.52 Hz, 1 H) 8.86 (s, 1 H) 9.09 (s, 1 H) 10.35 (s, 1 H) 10.69 (s, 1 H). MS(ESI(+)) m/e 476(M+H)^{+.}

### EXAMPLE 71

### 8-amino-N-(3-(((((4-chlorophenyl)amino)carbonyl)amino)methyl)phenyl)-3-cyclopropylimidazo[1,5-a]pyrazine-1-carboxamide

The title compound was prepared as described in EXAMPLE 37B by substituting 1-chloro-4-isocyanatobenzene and EXAMPLE 68A for 1-isocyanato-3-methylbenzene and EXAMPLE 37A, respectively. ¹H NMR (300 MHz, DMSO-d₆) δ ppm 0.99 - 1.41 (m, 4 H) 2.24 - 2.48 (m, 1 H) 4.32 (d, *J*=5.76 Hz, 2 H) 6.74 (t, *J*=5.76 Hz, 1 H) 7.03 - 7.56 (m, 7 H) 7.59 - 7.85 (m, 2 H) 8.00 (d, *J*=5.76 Hz, I H) 8.78 (s, 1 H) 9.24 (s, 1 H) 10.40 (s, 1 H) 10.86 (s, I H) MS(ESI(+)) m/e 476(M+H)^{+.}

### EXAMPLE 72

### 8-amino-3-cyclopropyl-N-(4-(((pyridin-3-ylamino)carbonyl)amino)phenyl)imidazo[1,5-a]pyrazine-1-carboxamide

The title compound was prepared as described in EXAMPLE 37B by substituting 3-isocyanatopyridine and EXAMPLE 68A for 1-isocyanato-3-methylbenzene and EXAMPLE 37A, respectively. ¹H NMR (300 MHz, DMSO-d6) δ ppm 0.79 - 1.39 (m, 4 H) 2.17 - 2.43 (m, 1 H) 4.33 (d, *J*=5.83 Hz, 2 H) 6.80 (t, *J*=5.83 Hz, 1 H) 6.95 - 7.56 (m, 5 H) 7.57 - 7.84 (m, 3 H) 7.82 - 8.00 (m, 1 H) 8.12 (d, *J*=3.68 Hz, 1 H) 8.55 (d, *J*=2.15 Hz, 1 H) 8.77 (s, 1 H) 9.05 (s, 1 H) 10.00 (s, 1 H)
MS(ESI(+)) m/e 443(M+H)^{+.}

### EXAMPLE 73

### 8-amino-3-cyclopropyl-N-(3-(((phenylsulfonyl)amino)methyl)phenyl)imidazo[1,5-a]pyrazine-1-carboxamide

A solution of EXAMPLE 68A (0.102 g) in DMF 3mL was treated with triethylamine (0.112 ml, 0.800 mmol) and benzenesulfonyl chloride (0.026 ml, 0.200 mmol), stirred at room temperature overnight then diluted with water and extracted with ethyl acetate. The extract was washed (brine), dried ( MgSO₄), concentrated to dryness and purified by preparative HPLC. ¹H NMR (300 MHz, DMSO-d₆) δ ppm 1.01 - 1.29 (m, 4 H) 2.34 - 2.58 (m, 1 H) 3.98 (d, *J*=5.95 Hz, 2 H) 7.04 (d, *J*=7.54 Hz, 1 H) 7.19 - 7.36 (m, 2 H) 7.48 - 7.69 (m, 4 H) 7.72 (s, 1 H) 7.77 - 7.91 (m, 2 H) 7.98 (d, *J*=5.55 Hz, 1 H) 8.18 (t, *J*=6.35 Hz, 1 H) 9.08 (s, 1 H) 10.32 (s, I H) 10.69 (s, 1 H). MS(ESI(+)) m/e 463(M+H)^{+.}

### EXAMPLE 74

### 8-amino-3-(3-(ethyl(2-hydroxyethyl)amino)-3-oxopropyl)-N-(3-(((((4-(trifluoromethyl)phenyl)amino)carbonyl)amino)methyl)phenyl)imidazo[1,5-a]pyrazine-1-carboxamide

### EXAMPLE 74A

### Ethyl 3-(8-chloro-1-iodoimidazo[1,5-a ]pyrazin-3-yl)propanoate

The title compound was prepared as described in EXAMPLES 1A-1C, except substituting 4-ethoxy-4-oxobutanoic acid for acetic acid in EXAMPLE 1A. MS(ESI(+)) m/e 380(M+H)^{+.}

### EXAMPLE 74B

### 3-(8-chloro-1-iodoimidazo[1,5-a]pyrazin-3-yl)propanoic acid

A solution of EXAMPLE 74A (1.96 g) in tetrahydrofuran (5mL) and ethanol (1mL) was treated with 2N LiOH (5.16 ml), stirred at room temperature for 3 hours and then neutralized to pH 3-4 with 3N HCl. The mixture was extracted with ethyl acetate. The extract was washed (brine), dried (MgSO₄) and concentrated to dryness to give the title compound. MS(ESI(+)) m/e 352(M+H)^{+.}

### EXAMPLE 74C

### 3-(8-amino-1-iodoimidazo[1,5-a]pyrazin-3-yl)-N-ethyl-N-(2-hydroxyethyl)propanamide

A mixture of EXAMPLE 74B (1.3 g) , 2-(ethylamino)ethanol (0.363 g), 1-ethyl-3-(3-(dimethylamino)propyl)-carbodiimide hydrochloride ( 0.78 g), 1-hydroxybenzotriazole hydrate (0.623 g), and N-methylmorpholine (0.748 g) in 5 mL DMF was stirred at room temperature for 10 hours. The reaction mixture was extracted with ethyl acetate 3 times and the combined extracts were washed (brine), dried (MgSO₄) and concentrated. The residue was purified by flash chromotography with 0-4% methanol/CH₂Cl₂ to afford 3-(8-(1H-benzo(d)(1,2,3)triazol-1-yloxy)-1-iodoimidazo[1,5-a]pyrazin-3-yl)-N-ethyl-N-(2-hydroxyethyl)propanamide. MS(ESI(+)) m/e 522(M+H)⁺ This product was placed in a high pressure tube with 7N ammonia (15 ml) in methanol, heated at 60C overnight then concentrated to dryness. The residue was purified by flash column with 0-10% methanol/CH₂Cl₂ to give the title compound. MS(ESI(+)) m/e 404(M+H)^{+.}

### EXAMPLE 74D

### 8-amino-3-(3-(ethyl(2-hydroxyethyl)amino)-3-oxopropyl)imidazo[1,5-a]pyrazine-1-carboxylic acid

The title compound was prepared as described in EXAMPLES 1E-1F, substituting EXAMPLE 74C for EXAMPLE I D in EXAMPLE 1E.

### EXAMPLE 74E

### 8-amino-3-(3-(ethyl(2-hydroxyethyl)amino)-3-oxopropyl)-N-(3-(((((4-(trifluoromethyl)phenyl)amino)carbonyl)amino)methyl)phenyl)imidazo[1,5-a]pyrazine-1-carboxamide

The title compound was prepared by substituting tert-butyl 3-(aminomethyl)phenylcarbamate and 4-trifluoromethyl-isocyanatobenzene for (4-aminophenyl)carbamic acid tert-butyl ester and 1-fluoro-3-isocyanatobenzene, respectively in EXAMPLE 1G, then reacting the product with EXAMPLE 74D as described in EXAMPLE 1H. ¹H NMR (300 MHz, DMSO-d₆) δ ppm 0.70 - 1.45 (m, 3 H) 2.86 - 3.15 (m, 2 H) 3.09 - 3.92 (m, 8 H) 4.34 (d, *J*=6.14 Hz, 2 H) 6.86 (t, *J*=5.68 Hz, 1 H) 7.12 - 7.27 (m, 2 H) 7.37 (t, *J*=7.83 Hz, 1 H) 7.52 - 7.71 (m, 5 H) 7.77 (s, 1 H) 7.89 (t, *J*=5.83 Hz, 1 H) 9.06 (s, 2 H) 10.45 (d, *J*=7.37 Hz, 1 H) 10.74 (s, I H). MS(ESI(+)) m/e 613(M+H)^{+.}

### EXAMPLE 75

### 8-amino-N-(3-(((((4-chloro-2-fluorophenyl)amino)carbonyl)amino)methyl)phenyl)-3-(3-(ethyl(2-hydroxyethyl)amino)-3-oxopropyl)imidazo[1,5-a]pyrazine-1-carboxamide

The title compound was prepared by substituting tert-butyl 3-(aminomethyl)phenylcarbamate and 4-chloro-2-fluoro-1-isocyanatobenzene for (4-aminophenyl)carbamic acid tert-butyl ester and 1-fluoro-3-isocyanatobenzene, respectively in EXAMPLE I G, then reacting the product with EXAMPLE 74D as described in EXAMPLE 1H. ¹H NMR (300 MHz, DMSO-d₆) δ ppm 0.73 - 1.40 (m, 3 H) 2.84 - 3.89 (m, 10 H) 4.26 - 4.45 (m, 2 H) 4.86 (s, I H) 7.00 - 7.54 (m, 6 H) 7.55 - 7.97 (m, 3 H) 8.17 (t, *J*=8.82 Hz, 1 H) 8.52 (d, *J*=2.37 Hz, 1 H) 9.04 (s, 1 H) 10.47 (d, *J*=5.43 Hz, 1 H) 10.70 (s, 1 H). MS(ESI(+)) m/e 597(M+H)^{+.}

### EXAMPLE 76

### 8-amino-3-(3-(ethyl(2-hydroxyethyl)amino)-3-oxopropyl)-N-(3-(((((3-fluorophenyl)amino)carbonyl)amino)methyl)phenyl)imidazo[1,5-a]pyrazine-1-carboxamide

The title compound was prepared by substituting tert-butyl 3-(aminomethyl)phenylcarbamate for (4-aminophenyl)carbamic acid tert-butyl ester in EXAMPLE 1G, then reacting the product with EXAMPLE 74D as described in EXAMPLE 1H. ¹H NMR (300 MHz, DMSO-d₆) δ ppm 0.84 - 1.30 (m, 3 H) 2.82-4.00 (m, 10 H) 4.27 - 4.42 (m, 2 H) 4.87 (s, 1 H) 6.53 - 6.87 (m, 2 H) 6.96 - 7.56 (m, 6 H) 7.59 - 8.01 (m, 3 H) 8.87 (s, 1 H) 9.12 (s, 1 H) 10.47 (d, *J*=5.43 Hz, I H) 10.76 (s, 1 H). MS(ESI(+)) m/e 563(M+H)^{+.}

### EXAMPLE 77

### 8-amino-3-(3-(ethyl(2-hydroxyethyl)amino)-3-oxopropyl)-N-(4-((((3-fluorophenyl)amino)carbonyl)amino)phenyl)imidazo[1,5-a]pyrazine-1-carboxamide

The title compound was prepared as described in EXAMPLE 1H by substituting EXAMPLE 74D for EXAMPLE 1F. ¹H NMR (300 MHz, DMSO-d₆) δ ppm 0.73 - 1.33 (m, 3 H) 2.82 - 4.08 (m, 10 H) 4.97 (s, 1 H) 6.59 - 6.88 (m, 1 H) 7.02 - 7.40 (m, 3 H) 7.42 - 7.60 (m, 3 H) 7.60 - 7.84 (m, 2 H) 7.83 - 8.09 (m, I H) 8.84 (s, 1 H) 8.97 (s, 2 H) 10.44 (d, *J*=2.71 Hz, 1 H) 10.84 (s, 1 H). MS(ESI(+)) m/e 549(M+H)^{+.}

### EXAMPLE 78

### 8-amino-3-cyclopropyl-N-(3-(((((4-(trifluoromethyl)phenyl)amino)carbonyl)amino)methyl)phenyl)imidazo[1,5-a]pyrazine-1-carboxamide

The title compound was prepared as described in EXAMPLEs 1G-1H by substituting tert-butyl 3-(aminomethyl)phenylcarbamate and 1-isocyanato-4-trifluoromethyl-benzene for (4-aminophenyl)carbamic acid tert-butyl ester and 1-fluoro-3-isocyanatobenzene, respectively in EXAMPLE I G and EXAMPLE 54A for EXAMPLE IF in EXAMPLE 1H. ¹H NMR (300 MHz, DMSO-d₆) δ ppm 0.89 - 1.28 (m, 4 H) 2.17 - 2.42 (m, 1 H) 4.33 (d, *J*=5.55 Hz, 2 H) 6.81 (t, *J*=5.75 Hz, 1 H) 7.08 (d, *J*=7.93 Hz, 1 H) 7.20 (d, *J*=5.16 Hz, 1 H) 7.32 (t, *J*=7.93 Hz, 1 H) 7.45 - 7.93 (m, 7 H) 9.02 (s, 1 H) 10.01 (s, 1 H). MS(ESI(+)) m/e 510(M+H)^{+.}

## Claims

1. A compound having Formula I 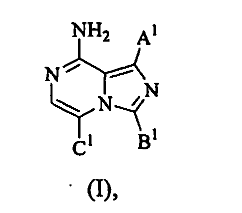 or a therapeutically acceptable salt thereof, wherein
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵; wherein
R¹ is R², R³ or R⁴;
R² is phenyl which is unfused or fused with benzene, heteroarene or R^{2A}; R^{2A} is cycloalkane, cycloalkene, heterocycloalkane or heterocycloalkene;
R³ is heteroaryl which is unfused or fused with benzene, heteroarene or R^{3A}; R^{3A} is cycloalkane, cycloalkene, heterocycloalkane or heterocycloalkene;
R⁴ is cycloalkyl, cycloalkenyl, heterocycloalkyl or heterocycloalkenyl, each of which is unfused or fused with benzene, heteroarene or R^{4A}; R^{4A} is cycloalkane, cycloalkene, heterocycloalkane or heterocycloalkene;
R⁵ is alkyl, alkenyl, or alkynyl, each of which is unsubstituted or substituted with one, two, three, four or five of independently selected R⁶, OR⁶, SR⁶, S(O)R⁶, SO₂R⁶,* NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br or I;
R⁶ is R⁷, R⁸, R⁹, or R^{9A};
R⁷ is phenyl which is unfused or fused with benzene, heteroarene or R^{7A}; R^{7A} is cycloalkane, cycloalkene, heterocycloalkane or heterocycloalkene;
R⁸ is heteroaryl which is unfused or fused with benzene, heteroarene or R^{8A}; R^{8A} is cycloalkane, cycloalkene, heterocycloalkane or heterocycloalkene;
R⁹ is cycloalkyl, cycloalkenyl, heterocycloalkyl or heterocycloalkenyl, each of which is unfused or fused with benzene, heteroarene or R^{9A}; R^{9A} is cycloalkane, cycloalkene, heterocycloalkane or heterocycloalkene;
R^{9A} is alkyl, alkenyl, or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, OR³⁰, OCH₂R³⁰, SR³⁰, S(O)R³⁰, SO₂R³⁰, C(O)R³⁰, CO(O)R³⁰, OC(O)R³⁰, OC(O)OR³⁰, NO₂, NH₂, NHR³⁰, N(R³⁰)₂, C(O)NH₂, C(O)NHR³⁰, C(O)N(R³⁰)₂, NHC(O)R³⁰, NHC(O)NHR³⁰, NHC(O)N(R³⁰)₂, NR³⁰C(O)NHR³⁰, NR³⁰C(O)N(R³⁰)₂, C(O)NHOH, C(O)NHOR³⁰, C(O)NHSO₂R³⁰, C(O)NR³⁰SO₂R³⁰, SO₂NH₂, SO₂NHR³⁰, SO₂N(R³⁰)₂, CF₃, CF₂CF₃, C(O)H, C(O)OH, C(N)NH₂, C(N)NHR³⁰, C(N)N(R³⁰)₂, CNOH, CNOCH₃, OH, (O), N₃, CF₃, CF₂CF₃, OCF₃, OCF₂CF₃, F, Cl, Br or I;
R³⁰ is R³¹, R³², R³³ or R³⁴;
R³¹ is phenyl which is unfused or fused with benzene, heteroarene or R^{31A}; R^{31A} is cycloalkane, cycloalkene, heterocycloalkane or heterocycloalkene;
R³² is heteroaryl which is unfused or fused with benzene, heteroarene or R^{32A};
R^{32A} is cycloalkane, cycloalkene, heterocycloalkane or heterocycloalkene;
R³³ is cycloalkyl, cycloalkenyl, heterocycloalkyl or heterocycloalkenyl, each of which is unfused or fused with benzene, heteroarene or R^{33A}; R^{33A} is cycloalkane, cycloalkene, heterocycloalkane or heterocycloalkene;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁸ or R³⁹;
R³⁶ is phenyl which is unfused or fused with benzene, heteroarene or R^{36A}; R^{36A} is cycloalkane, cycloalkene, heterocycloalkane or heterocycloalkene;
R³⁷ is heteroaryl which is unfused or fused with benzene, heteroarene or R^{37A}; R^{37A} is cycloalkane, cycloalkene, heterocycloalkane or heterocycloalkene;
R³⁸ is cycloalkyl, cycloalkenyl, heterocycloalkyl or heterocycloalkenyl, each of which is unfused or fused with benzene, heteroarene or R^{38A}; R^{38A} is cycloalkane, cycloalkene, heterocycloalkane or heterocycloalkene;
R³⁹ is alkyl, alkenyl or alkenyl, each of which is unsubstituted or substituted with R⁴⁰;
R⁴⁰ is phenyl, heteroaryl, cycloalkyl, cycloalkenyl or heterocycloalkyl; wherein the moieties represented by R³¹, R³², R³³ R³⁶, R³⁷ and R³⁸ are independently unsubstituted or substituted with one or two or three of independently selected F, Cl, Br, I, OH, (O)OH, NO₂, NH₂, CF₃, OH, R⁴⁵, OR⁴⁵, SR⁴⁵, S(O)R⁴⁵, SO₂R⁴⁵, C(O)NHR⁴⁵, C(O)N(R⁴⁵)₂, NHC(O)R⁴⁵, NR⁴⁵C(O)R⁴⁵, NHC(O)NHR⁴⁵, NHC(O)N(R⁴⁵)₂, NR⁴⁵C(O)NHR⁴⁵, NR⁴⁵C(O)N(R⁴⁵)₂, SO₂NHR⁴⁵, SO₂N(R⁴⁵)₂, NHSO₂R⁴⁵, NR¹SO₂R⁴⁵, OC(O)OR⁴⁵, NHC(O)OR⁴⁵ or NR⁴⁵C(O)OR⁴⁵_{;}
R⁴⁵ is alkyl, alkenyl or alkynyl, each of which is unsubstituted or substituted with one or two or three of independently selected R⁵⁰, F, Cl, Br, I, OH, C(O)OH, NO₂ or NH₂; and
R⁵⁰ is phenyl, heteroaryl, cycloalkyl, cycloalkenyl or heterocycloalkyl.

2. The compound of claim 1 wherein
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵; wherein
R¹ is R², R³ or R⁴;
R² is phenyl which is unfused or fused with benzene, heteroarene or R^{2A}; R^{2A} is cycloalkane, cycloalkene, heterocycloalkane or heterocycloalkene;
R³ is heteroaryl which is unfused or fused with benzene, heteroarene or R^{3A}; R^{3A} is cycloalkane, cycloalkene, heterocycloalkane or heterocycloalkene;
R⁴ is cycloalkyl, cycloalkenyl, heterocycloalkyl or heterocycloalkenyl, each of which is unfused or fused with benzene, heteroarene or R^{4A}; R^{4A} is cycloalkane, cycloalkene, heterocycloalkane or heterocycloalkene;
R⁵ is alkyl, alkenyl, or alkynyl, each of which is unsubstituted or substituted with one, two, three, four or five of independently selected R⁶, OR⁶, SR⁶, S(O)R⁶, SO₂R⁶, NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br or I;
R⁶ is R⁷, R⁸, R⁹, or R^{9A};
R⁷ is phenyl which is unfused or fused with benzene, heteroarene or R^{7A} ; R^{7A} is cycloalkane, cycloalkene, heterocycloalkane or heterocycloalkene;
R⁸ is heteroaryl which is unfused or fused with benzene, heteroarene or R^{8A}; R^{8A} is cycloalkane, cycloalkene, heterocycloalkane or heterocycloalkene;
R⁹ is cycloalkyl, cycloalkenyl, heterocycloalkyl or heterocycloalkenyl, each of which is unfused or fused with benzene, heteroarene or R^{9A}; R^{9A} is cycloalkane, cycloalkene, heterocycloalkane or heterocycloalkene;
R^{9A} is alkyl, alkenyl, or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, NHC(O)R³⁰, NHC(O)NHR³⁰, F, Cl, Br or I;
R³⁰ is R³¹, R³² R³³ or R³⁴;
R³¹ is phenyl which is unfused or fused with benzene, heteroarene or R^{31A}; R^{31A} is cycloalkane, cycloalkene, heterocycloalkane or heterocycloalkene;
R³² is heteroaryl which is unfused or fused with benzene, heteroarene or R^{32A};
R^{32A} is cycloalkane, cycloalkene, heterocycloalkane or heterocycloalkene;
R³³ is cycloalkyl, cycloalkenyl, heterocycloalkyl or heterocycloalkenyl, each of which is unfused or fused with benzene, heteroarene or R^{33A}; R^{33A} is cycloalkane, cycloalkene, heterocycloalkane or heterocycloalkene;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁸ or R³⁹;
R³⁶ is phenyl which is unfused or fused with benzene, heteroarene or R^{36A}; R^{36A} is cycloalkane, cycloalkene, heterocycloalkane or heterocycloalkene;
R³⁷ is heteroaryl which is unfused or fused with benzene, heteroarene or R^{37A};
R^{37A} is cycloalkane, cycloalkene, heterocycloalkane or heterocycloalkene;
R³⁸ is cycloalkyl, cycloalkenyl, heterocycloalkyl or heterocycloalkenyl, each of which is unfused or fused with benzene, heteroarene or R^{38A}; R^{38A} is cycloalkane, cycloalkene, heterocycloalkane or heterocycloalkene;
R³⁹ is alkyl, alkenyl or alkenyl, each of which is unsubstituted or substituted with R⁴⁰;
R⁴⁰ is phenyl, heteroaryl, cycloalkyl, cycloalkenyl or heterocycloalkyl;
wherein the moieties represented by R³¹, R³², R³³ R³⁶, R³⁷ and R³⁸ are independently unsubstituted or substituted with one or two or three of independently selected F, Cl, Br, I, CF₃, R⁴⁵; and
R⁴⁵ is alkyl, alkenyl or alkynyl, each of which is unsubstituted or substituted with one or two or three of independently selected F, Cl, Br, I, OH, C(O)OH, NO₂ or NH₂.

3. The compound of claim 2 wherein
A¹ is C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ or R⁵;
B¹ and C¹ are independently H, C(O)NHR¹, C(O)N(R¹)2, NHC(O)R¹, NR¹C(O)R¹, NHC(O)NHR¹, NHC(O)N(R¹)₂, NR¹C(O)NHR¹ NRC(O)N(R¹)₂, SO₂NHR¹, SO2N(R¹)₂, NHSO₂R¹, NR¹SO₂R¹, OC(O)OR¹, NHC(O)OR¹, NR¹C(O)OR¹ or R⁵; wherein
R¹ is R², R³ or R⁴;
R² is phenyl;
R³ is heteroaryl;
R⁴ is cycloalkyl;
R⁵ is alkyl, alkenyl, or alkynyl; each of which is unsubstituted or substituted with one, two, three, four or five of independently selected R⁶, OR⁶, SR³, S(O)R⁶,SO₂R⁶, NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br or I;
R⁶ is R⁷, R⁸, R⁹, or R^{9A};
R⁷ is phenyl;
R⁸ is heteroaryl;
R⁹ is cycloalkyl, cycloalkenyl, heterocycloalkyl or heterocycloalkenyl;
R^{9A} is alkyl, alkenyl or alkynyl, each of which is unsubstituted or substituted with one, two, three, four or five of independently selected NH₂, C(O)NH₂, SO₂NH₂, NHC(O)NH₂, OH, (O), C(O)OH, CN, CF₃, OCF₃, CF₂CF₃, F, Cl, Br or I;
wherein each foregoing cyclic moiety is independently unsubstituted or substituted with one or two or three or four or five of independently selected R³⁰, NHC(O)R³⁰, NHC(O)NHR³⁰, F, Cl, Br or I;
R³⁰ is R³¹ , R³² , R³³ or R³⁴;
R³¹ is phenyl;
R³² is heteroaryl;
R³³ is cycloalkyl, cycloalkenyl, heterocycloalkyl or heterocycloalkenyl;
R³⁴ is alkyl, alkenyl, or alkenyl, each of which is unsubstituted or substituted with one, two, three, four or five of independently selected R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br or I;
R³⁵ is R³⁶, R³⁷, R³⁸ or R³⁹;
R³⁶ is phenyl;
R³⁷ is heteroaryl;
R³⁸ is cycloalkyl, cycloalkenyl, heterocycloalkyl or heterocycloalkenyl;
R³⁹ is alkyl, alkenyl or alkenyl, each of which is unsubstituted or substituted with R⁴⁰;
R⁴⁰ is phenyl, heteroaryl, cycloalkyl, cycloalkenyl or heterocycloalkyl;
wherein the moieties represented by R³¹, R³², R³³ R³⁶ , R³⁷ and R³⁸ are independently unsubstituted or substituted with one or two or three of independently selected F, Cl, Br, I, CF₃, R⁴⁵; and
R⁴⁵ is alkyl, alkenyl or alkynyl, each of which is unsubstituted or substituted with one or two or three of independently selected F, Cl, Br, I, OH, C(O)OH, NO₂ or NH₂.

4. A compound of claim 1, which is
8-amino-N-(4-((((3-fluorophenyl)amino)carbonyl)amino)phenyl)-3-methylimidazo[1,5-a]pyrazine-1-carboxamide,
8-amino-N-(4-((anilinocarbonyl)amino)phenyl)-3-methylimidazo[1,5-a]pyrazine-1-carboxamide,
8-amino-N-(4-(benzoylamino)phenyl)-3-methylimidazo[1,5-a]pyrazine-1-carboxamide, 8-amino-3-methyl-N-(4-((((3-(trifluoromethyl)phenyl)amino)carbonyl)amino)phenyl)imidazo[1,5-a]pyrazine-1-carboxamide,
8-amino-N-(3-(((((3-fluorophenyl)amino)carbonyl)amino)methyl)phenyl)-3-methylimidazo[1,5-a]pyrazine-1-carboxamide,
8-amino-N-(3-(((anilinocarbonyl)amino)methyl)phenyl)-3-methylimidazo[1,5-a]pyrazine-1-carboxamide,
8-amino-N-(3-(((((2-fluorophenyl)amino)carbonyl)amino)methyl)phenyl)-3-methylimidazo[1,5-a]pyrazine-1-carboxamide,
8-amino-N-(3-(((((4-fluorophenyl)amino)carbonyl)amino)methyl)phenyl)-3-methylimidazo[1,5-a]pyrazine-1-carboxamide,
8-amino-3-methyl-N-(3-(((((4-(trifluoromethyl)phenyl)amino)carbonyl)amino)methyl)phenyl)imidazo[1,5-a]pyrazine-1-carboxamide,
8-amino-N-(4-((((3-(2-hydroxyethyl)phenyl)amino)carbonyl)amino)phenyl)-3-methylimidazo[1,5-a]pyrazine-1-carboxamide,
8-amino-N-(4-((((4-(2-hydroxyethyl)phenyl)amino)carbonyl)amino)phenyl)-3-methylimidazo[1,5-a]pyrazine-1-carboxamide;
8-amino-3-(3-(ethyl(2-hydroxyethyl)amino)-3-oxopropyl)-N-(3-(((((4-(trifluoromethyl)phenyl)amino)carbonyl)amino)methyl)phenyl)imidazo[1,5-a]pyrazine-1-carboxamide;
8-amino-N-(3-(((((4-chloro-2-fluorophenyl)amino)carbonyl)amino)methyl)phenyl)-3-(3-(ethyl(2-hydroxyethyl)amino)-3-oxopropyl)imidazo[1,5-a]pyrazine-1-carboxamide;
8-amino-3-(3-(ethyl(2-hydroxyethyl)amino)-3-oxopropyl)-N-(3-(((((3-fluorophenyl)amino)carbonyl)amino)methyl)phenyl)imidazo[1,5-a]pyrazine-1-carboxamide; and
8-amino-3-(3-(ethyl(2-hydroxyethyl)amino)-3-oxopropyl)-N-(4-((((3-fluorophenyl)amino)carbonyl)amino)phenyl)imidazo[1,5-a]pyrazine-1-carboxamide;
or a therapeutically acceptable salt thereof.

5. A composition comprising an excipient and a therapeutically effective amount of a compound of claim 1 having Formula I.

6. A compound of claim 1 having Formula I for use in treating bladder cancer, breast cancer, cervical cancer, colon cancer, endometrial cancer, esophageal cancer, lung cancer, ovarian cancer, pancreatic cancer, prostate cancer, rectal cancer, skin cancer, stomach cancer or thyroid cancer in a mammal by administering thereto a therapeutically effective amount of said compound.

7. A compound having formula I 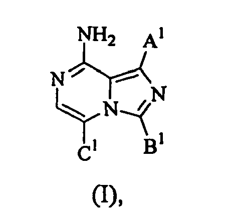 or a therapeutically acceptable salt thereof, wherein
A¹ is C(O)NH-phenyl;
B¹ and C¹ are independently H or R⁴;
R⁴ is cycloalkyl;
wherein each foregoing cyclic moiety is independently unsubstituted or substituted with one or two or three or four or five of independently selected R³⁰, NHC(O)R³⁰, NHC(O)NHR³⁰, F, Cl, Br or I;
R³⁰ is R³¹ , R³² or R³⁴;
R³¹ is phenyl;
R³² is heteroaryl;
R³⁴ is alkyl, each of which is unsubstituted or substituted with NHSO₂R³⁵, NHC(O)NHR³⁵, F, Cl, Br, or I;
R³⁵ is R³⁶, or R³⁷;
R³⁶ is phenyl;
R³⁷ is heteroaryl;
wherein the moieties represented by R³¹ and R³⁶ are independently unsubstituted or substituted with one or two or three of independently selected F, Cl, Br, I, CF₃, R⁴⁵;
and
R⁴⁵ is alkyl, which is unsubstituted or substituted with one or two or three of independently selected F, Cl, Br, I, or OH.

8. A compound of claim 7, which is
8-amino-3-cyclopropyl-N-(4-((((3-fluorophenyl)amino)carbonyl)amino)phenyl)imidazo[1,5-a]pyrazine-1-carboxamide;
8-amino-N-(4-((anilinocarbonyl)amino)phenyl)-3-cyclopropylimidazo[1,5-a]pyrazine-1-carboxamide;
8-amino-3-cyclopropyl-N-(4-((((3-methylphenyl)amino)carbonyl)amino)phenyl)imidazo[1,5-a]pyrazine-1-carboxamide;
8-amino-3-cyclopropyl-N-(4-((((2-fluoro-5-(trifluoromethyl)phenyl)amino)carbonyl)amino)phenyl)imidazo[1, 5-a]pyrazine-1-carboxamide;
8-amino-3-cyclopropyl-N-(4-((((4-(trifluoromethyl)phenyl)amino)carbonyl)amino)phenyl)imidazo[1,5-a]pyrazine-1-carboxamide;
8-amino-3-cyclopropyl-N-(3-((((pyridin-3-ylamino)carbonyl)amino)methyl)phenyl)imidazo[1,5-a]pyrazine-1-carboxamide;
8-amino-3-cyclopropyl-N-(4-((((3-fluorophenyl)amino)carbonyl)amino)-3-methylphenyl)imidazo[1,5-a]pyrazine-1-carboxamide;
8-amino-3-cyclopropyl-N-(4-((((2-fluorophenyl)amino)carbonyl)amino)-3-methylphenyl)imidazo[1,5-a]pyrazine-1-carboxamide;
8-amino-3-cyclopropyl-N-(3-methyl-4-((((4-(trifluoromethyl)phenyl)amino)carbonyl)amino)phenyl)imidazo[1,5-a]pyrazine-1-carboxamide;
8-amino-3-cyclopropyl-N-(3-((((3-fluorophenyl)amino)carbonyl)amino)phenyl)imidazo[1,5-a]pyrazine-1-carboxamide;
8-amino-3-cyclopropyl-N-(3-(((((3-fluorophenyl)amino)carbonyl)amino)methyl)phenyl)imidazo[1,5-a]pyrazine-1-carboxamide;
8-amino-N-(3-(((((4-chloro-2-fluorophenyl)amino)carbonyl)amino)methyl)phenyl)-3-cyclopropylimidazo[1,5-a]pyrazine-1-carboxamide;
8-amino-N-(3-(((anilinocarbonyl)amino)methyl)phenyl)-3-cyclopropylimidazo[1,5-a]pyrazine-1-carboxamide;
8-amino-3-cyclopropyl-N-(3-(((((3,4-difluorophenyl)amino)carbonyl)amino)methyl)phenyl)imidazo[1,5-a]pyrazine-1-carboxamide;
8-amino-3-cyclopropyl-N-(3-(((((2-fluorophenyl)amino)carbonyl)amino)methyl)phenyl)imidazo[1,5-a]pyrazine-1-carboxamide;
8-amino-3-cyclopropyl-N-(3-(((((4-fluorophenyl)amino)carbonyl)amino)methyl)phenyl)imidazo[1,5-a]pyrazine-1-carboxamide;
8-amino-N-(3-(((((3-chlorophenyl)amino)carbonyl)amino)methyl)phenyl)-3-cyclopropylimidazo[1,5-a]pyrazine-1-carboxamide;
8-amino-N-(3-(((((4-chlorophenyl)amino)carbonyl)amino)methyl)phenyl)-3-cyclopropylimidazo[1,5-a]pyrazine-1-carboxamide;
8-amino-3-cyclopropyl-N-(4-(((pyridin-3-ylamino)carbonyl)amino)phenyl)imidazo[1,5-a]pyrazine-1-carboxamide;
8-amino-3-cyclopropyl-N-(3-(((phenylsulfonyl)amino)methyl)phenyl)imidazo[1,5-a]pyrazine-1-carboxamide;
8-amino-3-cyclopropyl-N-(3-(((((4-(trifluoromethyl)phenyl)amino)carbonyl)amino)methyl)phenyl)imidazo[1,5-a]pyrazine-1-carboxamide;
or a pharmaceutically acceptable salt thereof.

9. A pharmaceutical composition comprising an excipient and a therapeutically effective amount of a compound of claim 7 having Formula 1.

10. A compound of claim 7 having Formula I for use in treating bladder cancer, breast cancer, cervical cancer, colon cancer, endometrial cancer, esophageal cancer, lung cancer, ovarian cancer, pancreatic cancer, prostate cancer, rectal cancer, skin cancer, stomach cancer or thyroid cancer in a mammal by administering thereto a therapeutically effective amount of said compound.

## Patentansprüche

1. Eine Verbindung mit der Formel I 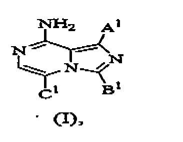 oder ein therapeutisch verträgliches Salz davon, worin
A¹ C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR1, SO₂N(R¹)₂, NHSO₂R¹, NR¹SO₂R¹, OC(O)OR¹, NHC(O)OR¹, NR¹C(O)OR¹ oder R⁵ ist:
B¹ und C¹ sind unabhängig H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ oder R⁵; worin
R¹ R², R³ oder R⁴ ist;
R² ist Phenyl, das nicht ankondensiert ist oder an Benzen, Heteroaren oder R^{2A} ankondensiert ist; R^{2A} ist Cycloalkan, Cycloalken, Heterocycloalkan oder Heterocycloalken;
R³ ist Heteroaryl, das nicht ankondensiert ist oder an Benzen, Heteroaren oder R^{3A} ankondensiert ist; R^{3A} ist Cycloalkan, Cycloalken, Heterocycloalkan oder Heterocycloalken;
R⁴ ist Cycloalkyl, Cycloalkenyl, Heterocycloalkyl oder Heterocycloalkenyl, wobei jedes davon nicht ankondensiert ist oder an Benzen, Heteroaren oder R^{4A} ankondensiert ist; R^{4A} ist Cycloalkan, Cycloalken, Heterocycloalkan oder Heterocycloalken;
R⁵ ist Alkyl, Alkenyl oder Alkinyl, wobei jedes davon unsubstituiert oder substituiert ist mit einem, zwei, drei, vier oder fünf unabhängig gewählten R⁶, OR⁶, SR⁶, S(O)R⁶, SO₂R⁶, NH₂, NHR⁶, N(R⁶)a, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O) , C(O)OH, CN, CF₃, OCF₃, CF₂CF₃, F, Cl, Br oder I,
R⁶ ist R¹, R⁸, R⁹ oder R^{9A};
R⁷ ist Phenyl, das nicht ankondensiert ist oder an Benzen, Heteroaren oder R^{7A} ankondensiert ist; R^{7A} ist Cycloalkan, Cycloalken, Heterocycloalkan oder Heterocycloalken;
R⁶ ist Heteroaryl, das nicht ankondensiert ist oder an Benzen, Heteroaren oder R^{8A} ankondensiert ist; R^{6A} ist Cycloalkan, Cycloalken, Heterocycloalkan oder Heterocycloalken;
R⁹ ist Cycloalkyl, Cycloalkenyl, Heterocycloalkyl oder Heterocycloalkenyl, wobei jedes davon nicht ankondensiert ist oder an Benzen, Heteroaren oder R^{9A} ankondensiert ist; R^{9A} ist Cycloalkan, Cycloalken, Heterocycloalkan oder Heterocycloalken;
R^{9A} ist Alkyl, Alkenyl oder Alkinyl, wobei jedes davon unsubstituiert oder substituiert ist mit einem, zwei, drei, vier oder fünf unabhängig gewählten NH₂, C(O)NH₂, SO₂NH₂, NHC(O)NH₃, OH, (O), C(O)OH, CN, CF₃, OCF₃, CF₂CF₃, F, Cl, Br oder I;
worin jeder vorhergehende zyklische Anteil unabhängig unsubstituiert oder substituiert ist mit einem oder zwei oder drei oder vier oder fünf unabhängig gewählten R³⁰, OR³⁰, OCH₂R³⁰, SR³⁰, S(O)R³⁰, SO₂R³⁰, C(O)R³⁰, CO(O)R³⁰, OC(O)R³⁰, OC(O)OR³⁰, NO₂, NH₂, NHR³⁰, N(R³⁰)₂, C(O)NH₂, C(O)NHR³⁰, C(O)N(R³⁰)₂, NHC(O)R³⁰, NHC(O)NHR³⁰, NHC(O)N(R³⁰)₂, NR³⁰C(O)NHR³⁰, NR³⁰C(O)N(R³⁰)₂, C(O)NHOH, C(O)NHOR³⁰, C(O)NHSO₂R³⁰, C(O)NR³⁰SO₂R³⁰, SO₂NH₂, SO₂NHR³⁰, SO₂N(R³⁰)₂, CF₃, CF₂CF₃, C(O)H, C(O)OH, C(N)NH₃, C(N)NHR³¹, C(N)N(R³⁰)₂, CNOH, CNOCH₃, OH, (O), N₃, CF₃, CF₂CF₃, OCF₃, OCF₂CF₃, F, Cl, Br oder I;
R³⁰ ist R³¹, R³², R³³ oder R³⁴;
R³¹ ist Phenyl, das nicht ankondensiert ist oder an Benzen, Heteroaren oder R^{31A} ankondensiert ist; R^{31A} ist Cycloalkan, Cycloalken, Heterocycloalkan oder Heterocycloalken;
R³² ist Heteroaryl, das nicht ankondensiert ist oder an Benzen, Heteroaren oder R^{32A} ankondensiert ist; R^{32A} ist Cycloalkan, Cycloalken, Heterocycloalkan oder Heterocycloalken;
R³³ ist Cycloalkyl, Cycloalkenyl, Heterocycloalkyl oder Heterocycloalkenyl, wobei jedes davon nicht ankondensiert ist oder an Benzen, Heteroaren oder R^{33A} ankondensiert ist; R^{33A} ist Cycloalkan, Cycloalken, Heterocycloalkan oder Heterocycloalken;
R³⁴ ist Alkyl, Alkenyl oder Alkenyl, wobei jedes davon unsubstituiert ist oder substituiert ist mit einem, zwei, drei, vier oder fünf unabhängig gewählten R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O) R³⁵, NR³⁵C (O) R³⁵, NHSO₂R³⁵, NR³⁵SO₂R³⁵, NHC(O) OR³⁵, NR³⁵C (0) OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br oder I;
R³⁵ ist R³⁶, R³⁷, R³⁸ oder R³⁹;
R³⁶ ist Phenyl, das nicht ankondensiert ist oder an Benzen, Heteroaren oder R^{36A} ankondensiert ist; R^{36A} ist Cycloalkan, Cycloalken, Heterocycloalkan oder Heterocycloalken;
R³⁷ ist Heteroaryl, das nicht ankondensiert ist oder an Benzen, Heteroaren oder R^{37A} ankondensiert ist; R^{37A} ist Cycloalkan, Cycloalken, Heterocycloalkan oder Heterocycloalken;
R³⁸ ist Cycloalkyl, Cycloalkenyl, Heterocycloalkyl oder Heterocycloalkenyl, wobei jedes davon nicht ankondensiert ist oder an Benzen, Heteroaren oder R^{38A} ankondensiert ist; R^{38A} ist Cycloalkan, Cycloalken, Heterocycloalkan oder Heterocycloalken;
R³⁹ ist Alkyl, Alkenyl oder Alkenyl, wobei jedes davon unsubstituiert ist oder substituiert ist mit R⁴⁰;
R⁴⁰ ist Phenyl, Heteroaryl, Cycloalkyl, Cycloalkenyl oder Heterocycloalkyl;
worin die Anteile, die durch R³¹, R³², R³³, R³⁶, R³⁷ und R³⁸ dargestellt sind, unabhängig unsubstituiert oder substituiert sind mit einem oder zwei oder drei unabhängig gewählten F, Cl, Br, I, OH, (O)OH, NO₂, NH₂, CF₃, OH, R⁴⁵, OR⁴⁵, SR⁴⁵, S(O)R⁴⁵, SO₂R⁴⁵, C(O)NHR⁴⁵, C(O)N(R⁴⁵)₂, NHC(O)R⁴⁵, NR⁴⁵C(O)R⁴⁵, NHC(O)NHR⁴⁵, NHC(O)N(R⁴⁵)₂, NR⁴⁵C(O)NHR⁴⁵, NR⁴⁵C(O)N(R⁴⁵)₂, SO₂NHR⁴⁵, SO₂N(R⁴⁵)₂, NHSO₂R⁴⁵, NR¹SO₂R⁴⁵, OC(O)OR⁴⁵, NHC(O)OR⁴⁵ oder NR⁴⁵C(O)OR⁴⁵;
R⁴⁵ ist Alkyl, Alkenyl oder Alkinyl, wobei jedes davon unsubstituiert oder substituiert ist mit einem oder zwei oder drei unabhängig gewählten R⁵⁰, F, Cl, Br, I, OH, C(O)OH, NO₂ oder NH₂; und
R⁵⁰ ist Phenyl, Heteroaryl, Cycloalkyl, Cycloalkenyl oder Heterocycloalkyl.

2. Die Verbindung gemäß Anspruch 1, worin
A¹ C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, ONR¹C(O)OR¹ oder R⁵ ist;
B¹ und C¹ sind unabhängig H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ oder R⁵; worin
R¹ R², R³ oder R⁴ ist;
R² ist Phenyl, das nicht ankondensiert ist oder an Benzen, Heteroaren oder R^{2A} ankondensiert ist; R^{2A} ist Cycloalkan, Cycloalken, Heterocycloalkan oder Heterocycloalken;
R³ ist Heteroaryl, das nicht ankondensiert ist oder an Benzen, Heteroaren oder R^{3A} ankondensiert ist; R^{3A} ist Cycloalkan, Cycloalken, Heterocycloalkan oder Heterocycloalken;
R⁴ ist Cycloalkyl, Cycloalkenyl, Heterocycloalkyl oder Heterocycloalkenyl, wobei jedes davon nicht ankondensiert ist oder an Benzen, Heteroaren oder R^{4A} ankondensiert ist; R^{4A} ist Cycloalkan, Cycloalken, Heterocycloalkan oder Heterocycloalken;
R⁵ ist Alkyl, Alkenyl oder Alkinyl, wobei jedes davon unsubstituiert oder substituiert ist mit einem, zwei, drei, vier oder fünf unabhängig gewählten R⁶, OR⁶, SR⁶, S(O)R⁶, SO₂R⁶, NH₂, NHR⁶, N(R⁶)₂, C(O)R⁶, C(O)NH₂, C(O)NHR⁶, C(O)N(R⁶)2, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R₆)₂, NHC(O)NH₂, NHC(O)R⁶, NHC(O)N(R⁶)₂, NR⁶C(O)N(R⁶)2, OH, (O), C(O)OH, CN, CF₃, OCF₃, CF₂CF₃, F, Cl, Br oder I;
R⁶ ist R⁷, R⁸, R⁹ oder R^{9A};
R⁷ ist Phenyl, das nicht ankondensiert ist oder an Benzen, Heteroaren oder R^{7A} ankondensiert ist; R^{7A} ist Cycloalkan, Cycloalken, Heterocycloalkan oder Heterocycloalken;
R⁸ ist Heteroaryl, das nicht ankondensiert ist oder an Benzen, Heteroaren oder R^{8A} ankondensiert ist; R^{8A} ist Cycloalkan, Cycloalken, Heterocycloalkan oder Heterocycloalken;
R⁹ ist Cycloalkyl, Cycloalkenyl, Heterocycloalkyl oder Heterocycloalkenyl, wobei jedes davon nicht ankondensiert ist oder an Benzen, Heteroaren oder R^{9A} ankondensiert ist; R^{9A} ist Cycloalkan, Cycloalken, Heterocycloalkan oder Heterocycloalken;
R^{9A} ist Alkyl, Alkenyl oder Alkinyl, wobei jedes davon unsubstituiert oder substituiert ist mit einem, zwei, drei, vier oder fünf unabhängig gewählten NH₂, C(O)NH₂, SO₂NH₂, NHC(O)NH₂, OH, (O), C(O)OH, CN, CF₃, OCF₃, CF₂CF₃, F, Cl, Br oder I;
worin jeder vorhergehende zyklische Anteil unabhängig unsubstituiert oder substituiert ist mit einem oder zwei oder drei oder vier oder fünf unabhängig gewählten R³⁰, NHC(O)R³⁰, NHC(O)NHR³⁰, F, Cl, Br oder I;
R³⁰ ist R³¹, R³², R³³ oder R³⁴;
R³¹ ist Phenyl, das nicht ankondensiert ist oder an Benzen, Heteroaren oder R^{31A} ankondensiert ist; R^{31A} ist Cycloalkan, Cycloalken, Heterocycloalkan oder Heterocycloalken;
R³² ist Heteroaryl, das nicht ankondensiert ist oder an Benzen, Heteroaren oder R^{32A} ankondensiert ist; R^{32A} ist Cycloalkan, Cycloalken, Heterocycloalkan oder Heterocycloalken;
R³³ ist Cycloalkyl, Cycloalkenyl, Heterocycloalkyl oder Heterocycloalkenyl, wobei jedes davon nicht ankondensiert ist oder an Benzen, Heteroaren oder R^{33A} ankondensiert ist; R^{33A} ist Cycloalkan, Cycloalken, Heterocycloalkan oder Heterocycloalken;
R³⁴ ist Alkyl, Alkenyl oder Alkenyl, wobei jedes davon unsubstituiert ist oder substituiert ist mit einem, zwei, drei, vier oder fünf unabhängig gewählten R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵(O)OR³⁵, SO₂NH₂, SO₂NHR³⁵, SO₂N (R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br oder I;
R³⁵ ist R³⁶, R³⁷, R³⁸ oder R³⁹;
R³⁶ ist Phenyl, das nicht ankondensiert ist oder an Benzen, Heteroaren oder R^{36A} ankondensiert ist; R^{36A} ist Cycloalkan, Cycloalken, Heterocycloalkan oder Heterocycloalken;
R³⁷ ist Heteroaryl, das nicht ankondensiert ist oder an Benzen, Heteroaren oder R^{37A} ankondensiert ist; R^{37A} ist Cycloalkan, Cycloalken, Heterocycloalkan oder Heterocycloalken;
R³⁸ ist Cycloalkyl, Cycloalkenyl, Heterocycloalkyl oder Heterocycloalkenyl, wobei jedes davon nicht ankondensiert ist oder an Benzen, Heteroaren oder R^{38A} ankondensiert ist; R^{38A} ist Cycloalkan, Cycloalken, Heterocycloalkan oder Heterocycloalken;
R³⁹ ist Alkyl, Alkenyl oder Alkenyl, wobei jedes davon unsubstituiert ist oder substituiert ist mit R¹⁰;
R⁴⁰ ist Phenyl, Heteroaryl, Cycloalkyl, Cycloalkenyl oder Heterocycloalkyl;
worin die Anteile, die durch R³¹, R³², R³³, R³⁶, R³⁷ und R³⁸ dargestellt sind, unabhängig unsubstituiert sind oder substituiert sind mit einem oder zwei oder drei unabhängig gewählten F, Cl, Br, I, CF₃, R⁴⁵; und
R⁴⁵ ist Alkyl, Alkenyl oder Alkinyl, wobei jedes davon unsubstituiert ist oder substituiert ist mit einem oder zwei oder drei unabhängig gewählten F, Cl, Br, I, OH, C(O)OH, NO₂ oder NH₂.

3. Die Verbindung gemäß Anspruch 2, worin
A¹ C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO²N(R¹)₂, NHSO₂R¹, NR¹SO₂R¹, OC(O)OR¹, NHC(O)OR¹, NR¹C(O)OR¹ oder R⁵ ist;
B¹ und C¹ sind unabhängig H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₃N(R¹)₂, NHSO₂R¹, NR¹SO₂R¹, OC(O)OR¹, NHC(O)OR¹, NR¹C(O)OR¹ oder R⁵; worin
R¹ R², R³ oder R⁴ ist;
R² ist Phenyl;
R³ ist Heteroaryl;
R⁴ ist Cycloalkyl;
R⁵ ist Alkyl, Alkenyl oder Alkinyl, wobei jedes davon unsubstituiert ist oder substituiert ist mit einem, zwei, drei, vier oder fünf unabhängig gewählten R⁶, OR⁶, SR⁶, S(O)R⁶, SO₂R⁶, NH₂, NHR⁶, N(R⁶)₂, C(O)R⁶, C(O)NH₂, C(0)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (O), C(O)OH, CN, CF₃, OCF₃, CF₂CF₃, F, Cl, Br oder I;
R⁶ ist R⁷, R⁶, R⁹ oder R^{9A};
R⁷ ist Phenyl;
R⁸ ist Heteroaryl;
R⁹ ist Cycloalkyl, Cycloalkenyl, Heterocycloalkyl oder Heterocycloalkenyl;
R^{9A} ist Alkyl, Alkenyl oder Alkinyl, wobei jedes davon unsubstituiert ist oder substituiert ist mit einem, zwei, drei, vier oder fünf unabhängig gewählten NH₂, C(O)NH₂, SO₂NH₂, NHC(O)NH₂, OH, (O), C(O)OH, CN, CF₃, OCF₃, CF₂CF, F, Cl, Br oder I;
worin jeder vorhergehende zyklische Anteil unabhängig unsubstituiert ist oder substituiert ist mit einem oder zwei oder drei oder vier oder fünf unabhängig gewähltem R³⁰, NHC(O)R³⁰, NHC(O)NHR³⁰, F, Cl, Br oder I;
R³⁰ ist R³¹, R³², R³³ oder R³⁴;
R³¹ ist Phenyl;
R³² ist Heteroaryl;
R³³ ist Cycloalkyl, Cycloalkenyl, Heterocycloalkyl oder Heterocycloalkenyl;
R³⁴ ist Alkyl, Alkenyl oder Alkenyl, wobei jedes davon unsubstituiert ist oder substituiert ist mit einem, zwei, drei, vier oder fünf unabhängig gewählten R³⁵, OR³⁵, SR³⁵ S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br oder I;
R³⁵ ist R³⁶, R³⁷, R³⁸ oder R³⁹;
R³⁶ ist Phenyl;
R³⁷ ist Heteroaryl;
R³⁸ ist Cycloalkyl, Cycloalkenyl, Heterocycloalkyl oder Heterocycloalkenyl;
R³⁹ ist Alkyl, Alkenyl oder Alkenyl, wobei jedes davon unsubstituiert ist oder substituiert ist mit R⁴⁰;
R⁴⁰ ist Phenyl, Heteroaryl, Cycloalkyl, Cycloalkenyl oder Heterocycloalkyl;
worin die Anteile, die durch R³¹, R³², R³³, R³⁶, R³⁷ und R³⁸ dargestellt sind, unabhängig unsubstituiert sind oder substituiert sind mit einem oder zwei oder drei unabhängig gewählten F, Cl, Br, I, CF₃, R⁴⁵; und
R⁴⁵ ist Alkyl, Alkenyl oder Alkinyl, wobei jedes davon unsubstituiert ist oder substituiert ist mit einem oder zwei oder drei unabhängig gewählten F, Cl, Br, I, OH, C(O)OH, NO₂ oder NH₂.

4. Eine Verbindung gemäß Anspruch 1, welche folgendes ist:
8-Amino-N-(4-((((3-fluorphenyl)amino)carbonyl)amino)phenyl)-3-methylimidazol[1,5-a]pyrazin-1-carboxamid;
8-Amino-N-(4-((anilinocarbonyl)amino)phenyl)-3-methylimidazol[1,5-a]pyrazin-1-carboxamid;
8-Amino-N-(4-(benzoylamino)phenyl)-3-methylimidazo[1,5-a]pyrazin-1-carboxamid;
8-Amino-3-methyl-N-(4-((((3-(trifluormethyl)phenyl)amino)carbonyl)amino)phenyl)imidazo[1,5-a]pyrazin-1-carboxamid;
8-Amino-N-(3-(((((3-fluorphenyl)amino)carbonyl)amino)methyl)phenyl)-3-methylimidazo[1,5-a]pyrazin-1-carboxamid;
8-Amino-N-(3-(((anilinocarbonyl)amino)methyl)phenyl)-3-methylimidazo[1,5-a]pyrazin-1-carboxamid;
8-Amino-N-(3-(((((2-fluorphenyl)amino)carbonyl)amino)methyl)phenyl)-3-methylimidazo[1,5-a]pyrazin-1-carboxamid;
8-Amino-N-(3-(((((4-fluorphenyl)amino)carbonyl)amino)methyl)phenyl)-3-methylimidazo[1,5-a]pyrazin-1-carboxamid;
8-Amino-3-methyl-N-(3-(((((4-(trifluormethyl)phenyl)amino)carbonyl)amino)methyl)phenyl)imidaz o[1,5-a]pyrazin-1-carboxamid;
8-Amino-N-(4-((((3-(2-hydroxyethyl)phenyl)amino)carbonyl)amino)phenyl)-3-methylimidazo[1,5-a]pyrazin-1-carboxamid;
8-Amino-N-(4-((((4-(2-hydroxyethyl)phenyl)amino)carbonyl)amino)phenyl)-3-methylimidazo[1,5-a]pyrazin-1-carboxamid;
8-Amino-3-(3-(ethyl(2-hydroxyethyl)amino)-3-oxopropyl)-N-(3-(((((4-(trifluormethyl)phenyl)amino)carbonyl)amino)methyl)phenyl)imidaz o[1,5-a]pyrazin-1-carboxamid;
8-Amino-N-(3-(((((4-chlor-2-fluorphenyl)amino)carbonyl)amino)methyl)phenyl)-3-(3-(ethyl(2-hydroxyethyl)amino)-3-oxopropyl)imidazo[1,5-a]pyrazin-1-carboxamid;
8-Amino-3-(3-(ethyl(2-hydroxyethyl)amino)-3-oxopropyl)-N-(3-(((((3-fluorphenyl)amino)carbonyl)amino)methyl)phenyl)imidazo[1,5-a]pyrazin-1-carboxamid; und
8-Amino-3-(3-(ethyl(2-hydroxyethyl)amino)-3-oxopropyl)-N-(4-((((3-fluorphenyl)amino)carbonyl)amino)phenyl)imidazo[1,5-a]pyrazin-1-carboxamid;
oder ein therapeutisch verträgliches Salz davon.

5. Eine Zusammensetzung, die ein Bindemittel und eine therapeutisch wirksame Menge einer Verbindung gemäß Anspruch 1 mit der Formel I umfasst.

6. Eine Verbindung gemäß Anspruch 1, mit der Formel I zur Verwendung in der Behandlung von Blasenkrebs, Brustkrebs, Gebärmutterhalskrebs, Darmkrebs, Endometriumkarzinom, Speiseröhrenkrebs, Lungenkrebs, Eierstockkrebs, Bauchspeicheldrüsenkrebs, Prostatakrebs, Rektumkarzinom, Hautkrebs, Magenkrebs oder Schilddrüsenkrebs, in einem Säugetier durch Verabreichen einer therapeutisch wirksamen Menge dieser Verbindung.

7. Eine Verbindung mit der Formel I 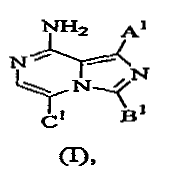 oder ein therapeutisch verträgliches Salz davon, worin
A¹ C(O)NH-Phenyl ist;
B¹ und C¹ sind unabhängig H oder R⁴;
R⁴ ist Cycloalkyl;
worin jeder vorhergehende zyklische Anteil unabhängig unsubstituiert ist oder substituiert ist mit einem oder zwei oder drei oder vier oder fünf unabhängig gewählten R³⁰, NHC(O)R³⁰, NHC(O)NHR³⁰, F, Cl, Br oder I;
R³⁰ ist R³¹, R³² oder R³⁴;
R³¹ ist Phenyl;
R³² ist Heteroaryl;
R³⁴ ist Alkyl, wobei jedes davon unsubstituiert ist oder substituiert ist mit NHSO₂R³⁵, NHC(O)NHR³⁵, F, Cl, Br oder I;
R³⁵ ist R³⁶ oder R³⁷;
R³⁶ ist Phenyl;
R³⁷ ist Heteroaryl;
worin die Anteile, die durch R³¹ und R³⁶ dargestellt sind, unabhängig unsubstituiert sind oder substituiert sind mit einem oder zwei oder drei unabhängig gewählten F, Cl, Br, I, CF₃, R⁴⁵;
und
R⁴⁵ ist Alkyl, das unsubstituiert ist oder substituiert ist mit einem oder zwei oder drei unabhängig gewählten F, Cl, Br, I oder OH.

8. Eine Verbindung gemäß Anspruch 7, welche folgendes ist:
8-Amino-3-cyclopropyl-N-(4-((((3-fluorphenyl)amino)carbonyl)amino)phenyl)imidazo[1,5-a]pyrazin-1-carboxamid;
8-Amino-N-(9-((anilinocarbonyl)amino)phenyl)-3-cyclopropylimidazo[1,5-a]pyrazin-1-carboxamid;
8-Amino-3-cyclopropyl-N-(4-((((3-methylphenyl)amino)carbonyl)amino)phenyl)imidazo[1,5-a]pyrazin-1-carboxamid;
8-Amino-3-cyclopropyl-N-(4-((((2-fluor-5-(trifluormethyl)phenyl)amino)carbonyl)amino)phenyl)imidazo[1,5-a]pyrazin-1-carboxamid;
8-Amino-3-cyclopropyl-N-(4-((((4-(trifluormethyl)phenyl)amino)carbonyl)amino)phenyl)imidazo[1,5-a]pyrazin-1-carboxamid;
8-Amino-3-cyclopropyl-N-(3-((((pyridin-3-ylamino)carbonyl)amino)methyl)phenyl)imidazo[1,5-a]pyrazin-1-carboxamid;
8-Amino-3-cyclopropyl-N-(4-((((3-fluorphenyl)amino)carbonyl)amino)-3-methylphenyl)imidazo[1,5-a]pyrazin-1-carboxamid;
8-Amino-3-cyclopropyl-N-(4-((((2-fluorphenyl)amino)carbonyl)amino)-3-methylphenyl)imidazo[1,5-a]pyrazin-1-carboxamid;
8-Amino-3-cyclopropyl-N-(3-methyl-4-((((4-(trifluormethyl)phenyl)amino)carbonyl)amino)phenyl)imidazo[1,5-a]pyrazin-1-carboxamid;
8-Amino-3-cyclopropyl-N-(3-((((3-fluorphenyl)amino)carbonyl)amino)phenyl)imidazo[1,5-a]pyrazin-1-carboxamid;
8-Amino-3-cyclopropyl-N-(3-(((((3-fluorphenyl)amino)carbonyl)amino)methyl)phenyl)imidazo[1,5-a]pyrazin-1-carboxamid;
8-Amino-N-(3-(((((4-chlor-2-fluorphenyl)amino)carbonyl)amino)methyl)phenyl)-3-cyclopropylimidazo[1,5-a]pyrazin-1-carboxamid;
8-Amino-N-(3-(((anilinocarbonyl)amino)methyl)phenyl)-3-cyclopropylimidazo[1,5-a]pyrazin-1-carboxamid;
8-Amino-3-cyclopropyl-N-(3-(((((3,4-difluorphenyl)amino)carbonyl)amino)methyl)phenyl)imidazo[1,5-a]pyrazin-1-carboxamid;
8-Amino-3-cyclopropyl-N-(3-(((((2-fluorphenyl)amino)carbonyl)amino)methyl)phenyl)imidazo[1,5-a]pyrazin-1-carboxamid;
8-Amino-3-cyclopropyl-N-(3-(((((4-fluorphenyl)amino)carbonyl)amino)methyl)phenyl)imidazo[1,5-a]pyrazin-1-carboxamid;
8-Amino-N-(3-(((((3-chlorphenyl)amino)carbonyl)amino)methyl)phenyl)-3-cyclopropylimidazo[1,5-a]pyrazin-1-carboxamid;
8-Amino-N-(3-(((((4-chlorphenyl)amino)carbonyl)amino)methyl)phenyl)-3-cyclopropylimidazo[1,5-a]pyrazin-1-carboxamid;
8-Amino-3-cyclopropyl-N-(4-(((pyridin-3-ylamino)carbonyl)amino)phenyl)imidazo[1,5-a]pyrazin-1-carboxamid;
8-Amino-3-cyclopropyl-N-(3-(((phenylsulfonyl)amino)methyl)phenyl)imidazo[1,5-a]pyrazin-1-carboxamid;
8-Amino-3-cyclopropyl-N-(3-(((((4-(trifluormethyl)phenyl)amino)carbonyl)amino)methyl)phenyl)imidaz o[1,5-a]pyrazin-1-carboxamid;
oder ein pharmazeutisch verträgliches Salz davon.

9. Eine pharmazeutische Zusammensetzung, umfassend ein Bindemittel und eine therapeutisch wirksame Menge einer Verbindung gemäß Anspruch 7 mit der Formel I.

10. Eine Verbindung gemäß Anspruch 7 mit der Formel I zur Verwendung in der Behandlung von Blasenkrebs, Brustkrebs, Gebärmutterhalskrebs, Darmkrebs, Endometriumkarzinom, Speiseröhrenkrebs, Lungenkrebs, Eierstockkrebs, Bauchspeicheldrüsenkrebs, Prostatakrebs, Rektumkarzinom, Hautkrebs, Magenkrebs oder Schilddrüsenkrebs, in einem Säugetier durch Verabreichen einer therapeutisch wirksamen Menge dieser Verbindung.

## Revendications

1. Composé de formule 1 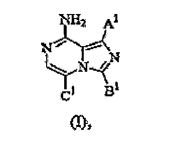 ou sel thérapeutiquement acceptable de celui-ci, dans lequel
A¹ est C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ ou R⁵;
B¹ et C¹ sont indépendamment H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ ou R⁵; où
R¹ est R², R³ ou R⁴;
R² est un groupe phényle qui est non condensé ou condensé avec du benzène, un hétéroarène ou R^{2A}; R^{2A} est un groupe cycloalcane, cycloalcène, hétérocycloalcane ou hétérocycloalcène ;
R³ est un groupe hétéroaryle qui est non condensé ou condensé avec du benzène, un hétéroarène ou R^{3A}; R^{3A} est un groupe cycloalcane, cycloalcène, hétérocycloalcane ou hétérocycloalcène ;
R⁴ est un groupe cycloalkyle, cycloalcényle, hétérocycloalkyle ou hétérocycloalcényle, dont chacun est non condensé ou condensé avec du benzène, un hétéroarène ou R^{4A}; R^{4A} est un groupe cycloalcane, cycloalcène, hétérocycloalcane ou hétérocycloalcène ;
R⁵ est un groupe alkyle, alcényle, ou alcynyle, dont chacun est non substitué ou substitué par un, deux, trois, quatre ou cinq éléments indépendamment choisis parmi R⁶, OR⁶, SR⁶, S(O)R⁶, SO₂R⁶, NH₂, NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (0), C(O)OH, CN, CF₃, OCF₃, CF₂CF₃, F, CI, Br ou I ;
R⁶ est R⁷, R⁸, R⁹, ou R^{9A};
R⁷ est un groupe phényle qui est non condensé ou condensé avec du benzène, un hétéroarène ou R^{7A}; R^{7A} est un groupe cycloalcane, cycloalcène, hétérocycloalcane ou hétérocycloalcène ;
R⁸ est un groupe hétéroaryle qui est non condensé ou condensé avec du benzène, un hétéroarène ou R^{8A} ; R^{8A} est un groupe cycloalcane, cycloalcène, hétérocycloalcane ou hétérocycloalcène ;
R⁹ est un groupe cycloalkyle, cycloalcényle, hétérocycloalkyle ou hétérocycloalcényle, dont chacun est non condensé ou condensé avec du benzène, un hétéroarène ou R^{9A} ; R^{9A} est un groupe cycloalcane, cycloalcène, hétérocycloalcane ou hétérocycloalcène;
R^{9A} est un groupe alkyle, alcényle, ou alcynyle, dont chacun est non substitué ou substitué par un, deux, trois, quatre ou cinq éléments indépendamment choisis parmi NH₂, C(O)NH₂, SO₂NH₂, NHC(O)NH₂, OH, (0), C(O)OH, CN, CF₃, OCF₃, CF₂CF₃, F, Cl, Br ou I ;
dans lequel chaque fragment cyclique susmentionné est indépendamment non substitué ou substitué par un ou deux ou trois ou quatre ou cinq éléments indépendamment choisis parmi R³⁰, OR³⁰, OCH₂R³⁰, SR³⁰, S(O)R³⁰, SO₂R³⁰, C(O)R³⁰, CO(O)R³⁰, OC(O)R³⁰, OC(O)OR³⁰, NO₂, NH₂, NHR³⁰, N(R³⁰)₂, C(O)NH₂, C(O)NHR³⁰, C(O)N(R³⁰)₂, NHC(O)R³⁰, NHC(O)NHR³⁰, NHC(O)N(R³⁰)₂, NR³⁰C(O)NHR³⁰, NR³⁰C(O)N(R³⁰)₂, C(O)NHOH, C(O)NHOR³⁰, C(O)NHSO₂R³⁰, C(O)NR³⁰SO₂R³⁰, SO₂NH₂, SO₂NHR³⁰, SO₂N(R³⁰)₂, CF₃, CF₂CF₃, C(O)H, C(O)OH, C(N)NH₂, C(N)NHR³⁰, C(N)N(R³⁰)₂, CNOH, CNOCH₃, OH, (O), N₃, CF₃, CF₂CF₃, OCF₃, OCF₂CF₃, F, Cl, Br ou I ;
R³⁰ est R³¹, R³², R³³ ou R³⁴ ;
R³¹ est un groupe phényle qui est non condensé ou condensé avec du benzène, un hétéroarène ou R^{31A} ; R^{31A} est un groupe cycloalcane, cycloalcène, hétérocycloalcane ou hétérocycloalcène ;
R³² est un groupe hétéroaryle qui est non condensé ou condensé avec du benzène, un hétéroarène ou R^{32A}; R^{32A} est un groupe cycloalcane, cycloalcène, hétérocycloalcane ou hétérocycloalcène ;
R³³ est un groupe cycloalkyle, cycloalcényle, hétérocycloalkyle ou hétérocycloalcényle, dont chacun est non condensé ou condensé avec du benzène, un hétéroarène ou R^{33A} ; R^{33A} est un groupe cycloalcane, cycloalcène, hétérocycloalcane ou hétérocycloalcène ;
R³⁴ est un groupe alkyle, alcényle, ou alcényle, dont chacun est non substitué ou substitué par un, deux, trois, quatre ou cinq éléments indépendamment choisis parmi R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵, NHSO₂R³⁵, NR³⁵SO₂R³⁵, NHC(O)OR³⁵, NR³⁵C(O)OR³⁵, SO₂NH₂, SO₂NHR³⁵, SO₂N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br ou I ;
R³⁵ est R³⁶, R³⁷, R³⁸ ou R³⁹ ;
R³⁶ est un groupe phényle qui est non condensé ou condensé avec du benzène, un hétéroarène ou R^{36A}; R^{36A} est un groupe cycloalcane, cycloalcène, hétérocycloalcane ou hétérocycloalcène ;
R³⁷ est un groupe hétéroaryle qui est non condensé ou condensé avec du benzène, un hétéroarène ou R^{37A} ; R^{37A} est un groupe cycloalcane, cycloalcène, hétérocycloalcane ou hétérocycloalcène ;
R³⁸ est un groupe cycloalkyle, cycloalcényle, hétérocycloalkyle ou hétérocycloalcényle, dont chacun est non condensé ou condensé avec du benzène, un hétéroarène ou R^{38A} ; R^{38A} est un groupe cycloalcane, cycloalcène, hétérocycloalcane ou hétérocycloalcène ;
R³⁹ est un groupe alkyle, alcényle ou alcényle, dont chacun est non substitué ou substitué par R⁴⁰ ;
R⁴⁰ est un groupe phényle, hétéroaryle, cycloalkyle, cycloalcényle ou hétérocycloalkyle ;
où les fragments représentés par R³¹, R³², R³³ R³⁶, R³⁷ et R³⁸ sont indépendamment non substitués ou substitués par un ou deux ou trois éléments indépendamment choisis parmi F, Cl, Br, I, OH, (O)OH, NO₂, NH₂, CF₃, OH, R⁴⁵, OR⁴⁵, SR⁴⁵, S(O)R⁴⁵, SO₂R⁴⁵, C(O)NHR⁴⁵, C(O)N(R⁴⁵)₂, NHC(O)R⁴⁵, NR⁴⁵C(O)R⁴⁵, NHC(O)NHR⁴⁵, NHC(O)N(R⁴⁵)₂, NR⁴⁵C(O)NHR⁴⁵, NR⁴⁵C(O)N(R⁴⁵)₂, SO₂NHR⁴⁵, SO₂N(R⁴⁵)₂, NHSO₂R⁴⁵, NR¹SO₂R⁴⁵, OC(O)OR⁴⁵, NHC(O)OR⁴⁵ ou NR⁴⁵C(O)OR⁴⁵ ;
R⁴⁵ est un groupe alkyle, alcényle ou alcynyle, dont chacun est non substitué ou substitué par un ou deux ou trois éléments indépendamment choisis parmi R⁵⁰, F, Cl, Br, I, OH, C(O)OH, NO₂ ou NH₂ ; et
R⁵⁰ est un groupe phényle, hétéroaryle, cycloalkyle, cycloalcényle ou hétérocycloalkyle.

2. Composé selon la revendication 1, dans lequel
A¹ est C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ ou R⁵ ;
B¹ et C¹ sont indépendamment H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(O)OR¹, NR¹C(O)OR¹ ou R⁵ ; où
R¹ est R², R³ ou R⁴ ;
R² est un groupe phényle qui est non condensé ou condensé avec du benzène, un hétéroarène ou R^{2A} ; R^{2A} est un groupe cycloalcane, cycloalcène, hétérocycloalcane ou hétérocycloalcène ;
R³ est un groupe hétéroaryle qui est non condensé ou condensé avec du benzène, un hétéroarène ou R^{3A} ; R^{3A} est un groupe cycloalcane, cycloalcène, hétérocycloalcane or hétérocycloalcène ;
R⁴ est un groupe cycloalkyle, cycloalcényle, hétérocycloalkyle ou hétérocycloalcényle, dont chacun est non condensé ou condensé avec du benzène, un hétéroarène ou R^{4A} ; R^{4A} est un groupe cycloalcane, cycloalcène, hétérocycloalcane ou hétérocycloalcène ;
R⁵ est un groupe alkyle, alcényle, ou alcynyle, dont chacun est non substitué ou substitué par un, deux, trois, quatre ou cinq éléments indépendamment choisis parmi R⁶, OR⁶, SR⁶, S(O)R⁶, SO₂R⁶, NH², NHR⁶, N(R⁶)₂, C(O)R⁶, C(O)NH₂, C(O)NHR⁶, C(O)N(R⁶)₂, NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂, NHC(O)NH₂, NHC(O)R⁶, NHC(O)N(R⁶)₂, NR⁶C(O)N(R⁶)₂, OH, (0), C(O)OH, CN, CF₃, OCF₃, CF₂CF₃, F, Cl, Br ou I ;
R⁶ est R⁷, R⁸, R⁹, ou R^{9A} _{;}
R⁷ est un groupe phényle qui est non condensé ou condensé avec du benzène, un hétéroarène ou R^{7A}; R^{7A} est un groupe cycloalcane, cycloalcène, hétérocycloalcane ou hétérocycloalcène ;
R⁸ est un groupe hétéroaryle qui est non condensé ou condensé avec du benzène, un hétéroarène ou R^{8A} ; R^{8A} est un groupe cycloalcane, cycloalcène, hétérocycloalcane ou hétérocycloalcène ;
R⁹ est un groupe cycloalkyle, cycloalcényle, hétérocycloalkyle ou hétérocycloalcényle, dont chacun est non condensé ou condensé avec du benzène, un hétéroarène ou R^{9A}; R^{9A} est un groupe cycloalcane, cycloalcène, hétérocycloalcane ou hétérocycloalcène ;
R^{9A} est un groupe alkyle, alcényle, ou alcynyle, dont chacun est non substitué ou substitué par un, deux, trois, quatre ou cinq éléments indépendamment choisis parmi NH₂, C(O)NH₂, SO₂NH₂, NHC(O)NH₂, OH, (O), C(O)OH, CN, CF₃, OCF₃, CF₂CF₃, F, Cl, Br ou I ;
où chaque fragment cyclique précédent est indépendamment non substitué ou substitué par un ou deux ou trois ou quatre ou cinq éléments indépendamment choisis parmi R³⁰, NHC(O)R³⁰, NHC(O)NHR³⁰, F, Cl, Br ou I ;
R³⁰ est R³¹, R³², R³³ ou R³⁴ ;
R³¹ est un groupe phényle qui est non condensé ou condensé avec du benzène, un hétéroarène ou R^{31A} ; R^{31A} est un groupe cycloalcane, cycloalcène, hétérocycloalcane ou hétérocycloalcène ;
R³² est un groupe hétéroaryle qui est non condensé ou condensé avec du benzène, un hétéroarène ou R^{32A} ; R^{32A} est un groupe cycloalcane, cycloalcène, hétérocycloalcane ou hétérocycloalcène ;
R³³ est un groupe cycloalkyle, cycloalcényle, hétérocycloalkyle ou hétérocycloalcényle, dont chacun est non condensé ou condensé avec du benzène, un hétéroarène ou R^{33A} ; R^{33A} est un groupe cycloalcane, cycloalcène, hétérocycloalcane ou hétérocycloalcène ;
R³⁴ est un groupe alkyle, alcényle, ou alcényle, dont chacun est non substitué ou substitué par un, deux, trois, quatre ou cinq éléments indépendamment choisis parmi R³⁵, OR³⁵, SR³⁵, S(O)R³⁵, SO₂R³⁵, NH₂, NHR³⁵, N(R³⁵)₂, C(O)R³⁵, C(O)NH₂, C(O)NHR³⁵, C(O)N(R³⁵)₂, NHC(O)R³⁵, NR³⁵C(O)R³⁵ NHSO₂R³⁵, NR³⁵SO₂R³⁵ , NHC(O)OR³⁵, NR³⁵C(O)OR³⁵, SO₂NH₂, SO₂NHR³⁵, SO₂N(R³⁵)_{2.} NHC(O)NH₂, NHC(O)NHR³⁵, NHC(O)R³⁵, NHC(O)N(R³⁵)_{2,} NR35C(O)N(R³⁵)₂, OH, (O), C(O)OH, CN, CF₃, OCF₃, CF₂CF₃, F, Cl, Br ou I ;
R³⁵ est R³⁶, R³⁷ R³⁸ ou R³⁹ ;
R³⁶ est un groupe phényle qui est non condensé ou condensé avec du benzène, un hétéroarène ou R^{36A}; R^{36A} est un groupe cycloalcane, cycloalcène, hétérocycloalcane ou hétérocycloalcène ;
R³⁷ est un groupe hétéroaryle qui est non condensé ou condensé avec du benzène, un hétéroarène ou R^{37A}; R^{37A} est un groupe cycloalcane, cycloalcène, hétérocycloalcane ou hétérocycloalcène ;
R³⁸ est un groupe cycloalkyle, cycloalcényle, hétérocycloalkyle ou hétérocycloalcényle, dont chacun est non condensé ou condensé avec du benzène, un hétéroarène ou R^{38A}; R^{38A} est un groupe cycloalcane, cycloalcène, hétérocycloalcane ou hétérocycloalcène ;
R³⁹ est un groupe alkyle, alcényle ou alcényle, dont chacun est non substitué ou substitué par R⁴⁰ ;
R⁴⁰ est un groupe phényle, hétéroaryle, cycloalkyle, cycloalcényle ou hétérocycloalkyle ;
où les fragments représentés par R³¹, R³², R³³ R³⁶ R³⁷ et R³⁸ sont indépendamment non substitués ou substitués par un ou deux ou trois éléments indépendamment choisis parmi F, Cl, Br, I, CF₃, R⁴⁵ ; et
R⁴⁵ est un groupe alkyle, alcényle ou alcynyle, dont chacun est non substitué ou substitué par un ou deux ou trois éléments indépendamment choisis parmi F, Cl, Br, I, OH, C(O)OH, NO₂ ou NH₂.

3. Composé selon la revendication 2, dans lequel
A¹ est C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO²R¹, OC(O)OR¹, NHC(O)OR¹, NR¹C(O)OR1 ou R⁵ ;
B¹ et C¹ sont indépendamment H, C(O)NHR¹, C(O)N(R¹)₂, NHC(O)R¹, NR¹C(O)R¹, NHC(O)NHR¹, NHC(O)N(R¹)₂, NR¹C(O)NHR¹, NR¹C(O)N(R¹)₂, SO₂NHR¹, SO₂N(R¹)₂, NHSO₂R¹, NR¹SO₂R¹, OC(O)OR¹, NHC(C)OR¹, NR¹C(O)OR¹ ou R⁵ ; où
R¹ est R², R³ ou R⁴ ;
R² est un groupe phényle ;
R³ est un groupe hétéroaryle ;
R⁴ est un groupe cycloalkyle ;
R⁵ est un groupe alkyle, alcényle, ou alcynyle, dont chacun est non substitué ou substitué par un, deux, trois, quatre ou cinq éléments indépendamment choisis parmi R⁶, OR⁶, SR⁶, S(O)R⁶, SO₂R⁶, NH², NHR⁶, N(R⁶)2, C(O)R⁶, C(O)NH₂, C(O)NHR⁶, C(O)N(R⁶)₂,NHC(O)R⁶, NR⁶C(O)R⁶, NHSO₂R⁶, NR⁶SO₂R⁶, NHC(O)OR⁶, NR⁶C(O)OR⁶, SO₂NH₂, SO₂NHR⁶, SO₂N(R⁶)₂ NHC(O)NH₂, NHC(O)R⁶, NHC(O)N(R⁶)₂, NR⁶C(O)N(R⁶)₂, OH, (0), C(O)OH, CN, CF₃, OCF₃, CF₂CF₃, F, CI, Br ou I ;
R⁶ est R⁷, R⁸, R⁹, ou R^{9A};
R⁷ est un groupe phényle ;
R⁸ est un groupe hétéroaryle ;
R⁹ est un groupe cycloalkyle, cycloalcényle, hétérocycloalkyle ou hétérocycloalcényle ;
R^{9A} est un groupe alkyle, alcényle, ou alcynyle, dont chacun est non substitué ou substitué par un, deux, trois, quatre ou cinq éléments indépendamment choisis parmi NH₂, C(O)NH₂, SO₂NH₂, NHC(O)NH₂, OH, (0), C(O)OH, CN, CF₃, OCF₃, CF₂CF₃, F,Cl,Br ou I;
où chaque fragment cyclique précédent est indépendamment non substitué ou substitué par un ou deux ou trois ou quatre ou cinq éléments indépendamment choisis parmi R³⁰, NHC(O)R³⁰, NHC(O)NHR³⁰, F, Cl, Br ou I ;
R³⁰ est R³¹, R³², R³³ 0U R³⁴ ;
R³¹ est un groupe phényle ;
R³² est un groupe hétéroaryle ;
R³³ est un groupe cycloalkyle, cycloalcényle, hétérocycloalkyle ou hétérocycloalcényle ;
R³⁴ est un groupe alkyle, alcényle, ou alcynyle, dont chacun est non substitué ou substitué par un, deux, trois, quatre ou cinq éléments indépendamment choisis parmi R³⁵, OR³⁵, SR³⁵, S(O)R³⁵, SO₂R³⁵, NH₂, NHR³⁵, N(R35)₂, C(O)R³⁵, C(O)NH₂, C(O)NHR³⁵, C(O)N(R³⁵)₂, NHC(O)R³⁵, NR³⁵C(O)R³⁵, NHSO2R³⁵, NR³⁵SO₂R³⁵, NHC(O)OR³⁵, NR³⁵C(O)OR³⁵, SO₂NH₂, SO₂NHR³⁵, SO2N(R³⁵)₂, NHC(O)NH₂, NHC(O)NHR³⁵, NHC(O)R³⁵, NHC(O)N(R³⁵)₂, NR³⁵C(O)N(R³⁵)₂, OH, (O), C(O)OH, CN, CF₃, OCF₃, CF₂CF₃, F, Cl, Br ou I ;
R³⁵ est R³⁶, R³⁷, R³⁸ ou R³⁹ ;
R³⁶ est un groupe phényle ;
R³⁷ est un groupe hétéroaryle ;
R³⁸ est un groupe cycloalkyle, cycloalcényle, hétérocycloalkyle ou hétérocycloalcényle ;
R³⁹' est un groupe alkyle, alcényle ou alcényle, dont chacun est non substitué ou substitué par R⁴⁰ ;
R⁴⁰ est un groupe phényle, hétéroaryle, cycloalkyle, cycloalcényle ou hétérocycloalkyle ;
où les fragments représentés par R³¹, R³², R³³ R³⁶, R³⁷ et R³⁸ sont indépendamment non substitués ou substitués par un ou deux ou trois éléments indépendamment choisis parmi F, Cl, Br, I, CF₃, R⁴⁵ ; et
R⁴⁵ est un groupe alkyle, alcényle ou alcynyle, dont chacun est non substitué ou substitué par un ou deux ou trois éléments indépendamment choisis parmi F, CI, Br, I, OH, C(O)OH, NO₂ ou NH₂.

4. Composé selon la revendication 1, qui est
le 8-amino-N-(4-((((3-fluorophényl)amino)carbonyl)amino)phényl)-3-méthylimidazo[1,5-a]pyrazine-1-carboxamide,
le 8-amino-N-(4-((anilinocarbonyl)amino)phényl)-3-méthylimidazo[1,5-a]pyrazine-1-carboxamide,
le 8-amino-N-(4-(benzoyiamino)phényl)-3-méthyilmidazo[1,5-a]pyrazine-1-carboxamide,
le 8-amino-3-méthyl-N-(4-((((3-(trifluorométhyl)phényl)amino)carbonyl)amino)phényl)imidazo[1,5-a]pyrazine-1-carboxamide,
le 8-amino-N-(3-(((((3-fluorophényl)amino)carbonyl)amino)méthyl)phényl)-3-méthylimidazo[1,5-a]pyrazine-1-carboxamide,
le 8-amino-N-(3-(((anilinocarbonyl)amino)méthyl)phényl)-3-méthylimidazo[1,5-a]pyrazine-1-carboxamide,
le 8-amino-N-(3-(((((2-fluorophényl)amino)carbonyl)amino)méthyl)phényl)-3-méthylimïdazo[1,5-a]pyrazine-1-carboxamide,
le 8-amino-N-(3-(((((4-fluorophényl)amino)carbonyl)amino)méthyl)phényl)-3-méthylimidazo[1,5-a]pyrazine-1-carboxamide,
le 8-amino- 3-méthyl-N-(3-(((((4-(trifluorométhyl)phényl)amino)carbonyl)amino)méthyl)phényl)imidazo[1,5-a]pyrazine-1-carboxamide,
le 8-amino-N-(4-((((3-(2-hydroxyéthyl)phényl)amino)carbonyl)amino)phényl)-3-méthylimidazo[1,5-a]pyrazine-1-carboxamide,
le 8-amino-N-(4-((((4-(2-hydroxyéthyl)phényl)amino)carbonyl)amino)phényl)-3-méthylimidazo[1,5-a]pyrazine-1-carboxamide ;
le 8-amino-3-(3-(éthyl(2-hydroxyéthyl)amino)-3-oxopropyl)-N-(3-(((((4-(trifluorométhyl)phényl)amino)carbonyl)amino)méthyl)phényl)imidazo[1,5-alpyrazine-1-carboxamide ;
le 8-amino-N-(3-(((((4-chloro-2-fluorophényl)amino)carbonyl)amino)méthyl)phényl)-3-(3-(éthyl(2-hydroxyéthyl)amino)-3-oxopropyl)imidazo[1,5-a]pyrazine-1-carboxamide ;
le 8-amino-3-(3-(éthyl(2-hydroxyéthyl)amino)-3-oxopropyl)-N-(3-(((((3-fluorophényl)amino)carbonyl)amino)méthyl)phényl)imidazo[1,5-a]pyrazine-1-carboxamide ; et
le 8-amino-3-(3-(éthyl(2-hydroxyéthyl)amino)-3-oxopropyl)-N-(4-((((3-fluorophényl)amino)carbonyl)amino)phényl)imidazo [1,5-a]pyrazine-1-carboxamide ;
ou sel thérapeutiquement acceptable de celui-ci.

5. Composition comprenant un excipient et une quantité thérapeutiquement efficace d'un composé de la revendication 1 de formule I.

6. Composé selon la revendication 1 de formule I à utiliser dans le traitement du cancer de la vessie, du cancer du sein, du cancer du col de l'utérus, du cancer du colon, du cancer de l'endomètre, du cancer de l'oesophage, du cancer du poumon, du cancer de l'ovaire, du cancer du pancréas, du cancer de la prostate, du cancer rectal, du cancer de la peau, du cancer de l'estomac ou du cancer de la thyroïde chez un mammifère par administration à celui-ci d'une quantité thérapeutiquement efficace dudit composé.

7. Composé de formule I 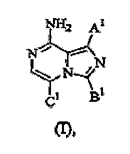 ou sel thérapeutiquement acceptable de celui-ci, dans lequel
A¹ est un groupe C(O)NH-phényle ;
B¹ et C¹ sont indépendamment H ou R⁴ ;
R⁴ est un groupe cycloalkyle ;
où chaque fragment cyclique précédent est indépendamment non substitué ou substitué par un ou deux ou trois ou quatre ou cinq éléments indépendamment choisis parmi R³⁰, NHC(O)R³⁰, NHC(O)NHR³⁰, F, Cl, Br ou I ;
R^{3o} est R³¹, R³² ou R³⁴ ;
R³¹ est un groupe phényle ;
R³² est un groupe hétéroaryle ;
R³⁴ est un groupe alkyle, dont chacun est non substitué ou substitué par NHSO₂R³⁵, NHC(O)NHR³⁵, F, Cl, Br, ou I ;
R³⁵ est R³⁶, ou R³⁷ ;
R³⁶ est un groupe phényle
R³⁷ est un groupe hétéroaryle ;
où les fragments représentés par R³¹ et R³⁶ sont indépendamment non substitués ou substitués par un ou deux ou trois éléments indépendamment choisis parmi F, Cl, Br, I, CF₃, R⁴⁵; et
R⁴⁵ est un groupe alkyle, qui est non substitué ou substitué par un ou deux ou trois éléments indépendamment choisis parmi F, CI, Br, I, ou OH.

8. Composé selon la revendication 7, qui est
le 8-amino-3-cyclopropyl-N-(4-((((3-fluorophényl)amino)carbonyl)amino)phényl)imidazo[1,5-a]pyrazine-1-carboxamide ;
le 8-amino-N-(4-((anilinocarbonyl)amino)phényl)-3-cyclopropylimidazo[1,5-a]pyrazine-1-carboxamide ;
le 8-amino-3-cyclopropyl-N-(4-((((3-méthylphényl)amino)carbonyl)amino)phényl)imidazo[1,5-a]pyrazine-1-carboxamide ;
le 8-amino-3-cydopropyl-N-(4-((((2-f)uoro-5-(trifluorométhyl)phényl)amino)carbonyl)amino)phényl)imidazo[1,5-a]pyrazine-1-carboxamide ;
le 8-amino-3-cyclopropyl-N-(4-((((4-(trifluorométhyl)phényl)amino)carbonyl)amino)phényl)imidazo[1,5-a]pyrazine-1-carboxamide ;
le 8-amino-3-cyclopropyl-N-(3-((((pyridin-3-ylamino)carbonyl)amino)méthyl)phényl)imidazo[1,5-a]pyrazine-1-carboxamide ;
le 8-amino-3-cyclopropyl-N-(4-((((3-fluorophényl)amino)carbonyl)amino)-3-méthylphényl)imidazo[1,5-a]pyrazine-1-carboxamide ;
le 8-amino-3-cyclopropyl-N-(4-((((2-fluorophényl)amino)carbonyl)amino)-3-méthylphényl)imidazo[1,5-a]pyrazine-1-carboxamide ;
le 8-amino-3-cyclopropyl-N-(3-méthyl-4-((((4-(trifluorométhyl)phényl)amino)carbonyl)amino)phényl)imidazo[1,5-a]pyrazine-1-carboxamide ;
le 8-amino-3-cyclopropyl-N-(3-((((3-fluorophényl)amino)carbonyl)amino)phényl)imidazo[1,5-a]pyrazine-1-carboxamide ;
le 8-amino-3-cyclopropyl-N-(3-(((((3-fluorophényl)amino)carbony))amino)méthyl)phényl)imidazo[1,5-a]pyrazine-1-carboxamide ;
le 8-amino-N-(3-(((((4-chloro-2-fluorophényl)amino)carbonyl)amino)méthyl)phényl)-3-cyclopropylimidazo[1,5-a]pyrazine-1-carboxamide ;
le 8-amino-N-(3-(((anilinocarbonyl)amino)méthyl)phényl)-3-cyclopropylimidazo[1,5-a]pyrazine-1-carboxamide ;
le 8-amino-3-cyclopropyl-N-(3-(((((3,4-difluorophényl)amino)carbonyl)amino)méthyl)phényl)imidazo[1,5-a]pyrazine-1-carboxamide ;
le 8-amino-3-cyclopropyl-N-(3-(((((2-fluorophényl)amino)carbonyl)amino)méthyl)phényl)imidazo[1,5-a]pyrazine-1-carboxamide ;
le 8-amino-3-cyclopropyl-N-(3-(((((4-fluorophényl)amino)carbonyl)amino)méthyl)phényl)imidazo[1,5-a]pyrazine-1-carboxamide ;
le 8-amino-N-(3-(((((3-chlorophényl)amino)carbonyl)amino)méthyl)phényl)-3-cyclopropylimidazo[1,5-a]pyrazine-1-carboxamide ;
le 8-amino-N-(3-(((((4-chlorophényl)amino)carbonyl)amino)méthyl)phényl)-3-cyclopropylimidazo[1,5-a]pyrazine-1-carboxamide ;
le 8-amino-3-cyclopropyl-N-(4-(((pyridin-3-ylamino)carbonyl)amino)phényl)imidazo[1,5-a]pyrazine-1-carboxamide ;
le 8-amino-3-cyclopropyl-N-(3-(((phénylsulfonyl)amino)méthyl)phényl)imidazo[1,5-a]pyrazine-1-carboxamide ;
le 8-amino-3-cyclopropyl-N-(3-(((((4-(trifluorométhyl)phényl)amino)carbonyl)amino)méthyl)phényl)imidazo[1,5-a]pyrazine-1-carboxamide ;
ou un sel pharmaceutiquement acceptable de celui-ci.

9. Composition pharmaceutique comprenant un excipient et une quantité thérapeutiquement efficace d'un composé de la revendication 7 de formule I.

10. Composé selon la revendication 7 de formule I à utiliser dans le traitement du cancer de la vessie, du cancer du sein, du cancer du col de l'utérus, du cancer du colon, du cancer de l'endomètre, du cancer de l'oesophage, du cancer du poumon, du cancer de l'ovaire, du cancer du pancréas, du cancer de la prostate, du cancer rectal, du cancer de la peau, du cancer de l'estomac ou du cancer de la thyroïde chez un mammifère par administration à celui-ci d'une quantité thérapeutiquement efficace dudit composé.
